(19) Europäisches Patentamt / European Patent Office / Office européen des brevets

(11) **EP 4 016 081 B1**

(12) **EUROPÄISCHE PATENTSCHRIFT**

(45) Veröffentlichungstag und Bekanntmachung des Hinweises auf die Patenterteilung:
**22.02.2023 Patentblatt 2023/08**

(21) Anmeldenummer: **20215995.0**

(22) Anmeldetag: **21.12.2020**

(51) Internationale Patentklassifikation (IPC):
**G01N 33/58** (2006.01)   **G06T 7/00** (2006.01)
**G06T 7/10** (2017.01)   **G06N 3/02** (2006.01)
**G06K 9/62** (2006.01)

(52) Gemeinsame Patentklassifikation (CPC):
**G01N 33/582; G01N 33/564; G06F 18/2414;
G06N 3/084; G06T 7/0012; G06T 7/11;
G06V 10/44; G06V 20/695; G06V 20/698;**
G06T 2207/10024; G06T 2207/10056;
G06T 2207/10064; G06T 2207/20081;
G06T 2207/30024

(54) **VERFAHREN UND VORRICHTUNG ZUM DETEKTIEREN EINER PRÄSENZ EINES FLUORESZENZMUSTERTYPS AUF EINEM ORGANSCHNITT MITTELS IMMUNFLUORESZENZMIKROSKOPIE**

METHOD AND DEVICE FOR DETECTING A PRESENCE OF A FLUORESCENCE PATTERN TYPE ON AN ORGAN SEGMENT BY MEANS OF IMMUNOFLUORESCENCE MICROSCOPY

PROCÉDÉ ET DISPOSITIF DE DÉTECTION D'UNE PRÉSENCE D'UN TYPE DE MOTIF FLUORESCENT SUR UNE SECTION D'ORGANES AU MOYEN D'UNE MICROSCOPIE IMMUNOFLUORESCENTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**22.06.2022 Patentblatt 2022/25**

(73) Patentinhaber: **Euroimmun Medizinische Labordiagnostika AG**
**23560 Lübeck (DE)**

(72) Erfinder:
• **KRAUTH, Jens**
**23552 Lübeck (DE)**
• **GERLACH, Stefan**
**23627 Gross Grönau (DE)**

• **MARZAHL, Christian**
**91052 Erlangen (DE)**
• **KRAUSE, Christopher**
**21514 Büchen (DE)**
• **HOCKE, Jens**
**23552 Lübeck (DE)**
• **HAHN, Melanie**
**23617 Stockelsdorf (DE)**
• **VOIGT, Jörn**
**23558 Lübeck (DE)**

(56) Entgegenhaltungen:
**EP-A1- 3 258 247    EP-A1- 3 712 618
EP-A1- 3 767 587**

**Beschreibung**

Die Erfindung betrifft ein Verfahren sowie eine Vorrichtung zum Detektieren wenigstens einer potentiellen Präsenz wenigstens eines Fluoreszenzmustertyps auf einem Organschnitt mittels Immunfluoreszenzmikroskopie mittels digitaler Bildverarbeitung.

EP3258247 A1 enthüllt ein Verfahren zum Detektieren wenigstens einer potentiellen Präsenz wenigstens eines Fluoreszenzmustertyps auf einem Organschnitt mittels Immunfluoreszenzmikroskopie aber kein Verfahren, bei dem neuronale Netzwerke zum Einsatz kommen.

EP3712618 A1 enthüllt ein Verfahren, bei dem neuronale Netze Teilflächen eines Fluoreszenzbildes bestimmen, welche für eine Ausbildung des Fluoreszenzbildes relevant sind aber kein Verfahren, bei dem Organschnitte untersucht werden.

EP3767587 A1 enthüllt ein Verfahren, in dem ein "Zellsubstrat" verwendet wird, welches humane Epithel- oder Epitheliomzellen aufweist. Ein Organschnitt wird nicht verwendet.

Eine Immunfluoreszenzmikroskopie bzw. indirekte Immunfluoreszenzmikroskopie ist ein invitro-Test für eine Bestimmung einer Präsenz humaner Antikörper gegen bestimmte Antigene, um eine diagnostische Fragestellung beantworten bzw. einschätzen zu können. Derartige Antigene sind beispielsweise in bestimmten Bereichen von Organschnitten wie einer Rattenniere oder eines Oesophagus eines Affen vorhanden. Als Substrat dient also ein Organschnitt, welcher mit einer Patientenprobe in Form von Blut oder verdünntem Blut oder aber Blutserum oder verdünntem Blutserum inkubiert wird. Die Patientenprobe weist also potentiell bestimmte primäre Antikörper auf, welche Ausdruck eines Vorhandenseins einer Erkrankung des Patienten sein können. Solche primären bzw. spezifischen Antikörper können dann an Antigene des Substrats bzw. des Organschnittes binden. Derartig gebundene primäre Antikörper können dann dadurch markiert werden, dass in einem weiteren Inkubationsschritt sogenannte sekundäre Antikörper, vorzugsweise Anti-Human-Antikörper, an die gebundenen primären Antikörper anbinden und später dadurch sichtbar gemacht werden können, dass die sekundären Antikörper mit einem Fluoreszenzfarbstoff markiert sind. Ein solcher Fluoreszenzfarbstoff ist vorzugsweise ein grüner Fluoreszenzfarbstoff, insbesondere der Fluoreszenzfarbstoff FITC. Eine solche Bindung eines primären Antikörpers gemeinsam mit einem fluoreszenzmarkierten sekundären Antikörper kann dann später dadurch sichtbar gemacht werden, dass der Organschnitt mit Anregungslicht einer bestimmten Wellenlänge bestrahlt wird und somit die gebundenen Fluoreszenzfarbstoffe zur Emission von Fluoreszenzstrahlung angeregt werden.

Abhängig von der diagnostischen Fragestellung kann auf eine Präsenz eines oder ganz bestimmter Fluoreszenzmustertypen auf bestimmten Organschnitten bzw. ganz bestimmten Teilbereichen bzw. Teilflächen der Organschnitte abgestellt werden. Es ergibt sich also die Aufgabe, mittels digitaler Bildverarbeitung im Zuge einer Immunfluoreszenzmikroskopie bei einem in der vorgeschriebenen Weise inkubierten Organschnitt mittels digitaler Bildverarbeitung einen oder mehrere Fluoreszenzmustertypen in einem Fluoreszenzbild einer Immunfluoreszenzmikroskopie zu Detektieren.

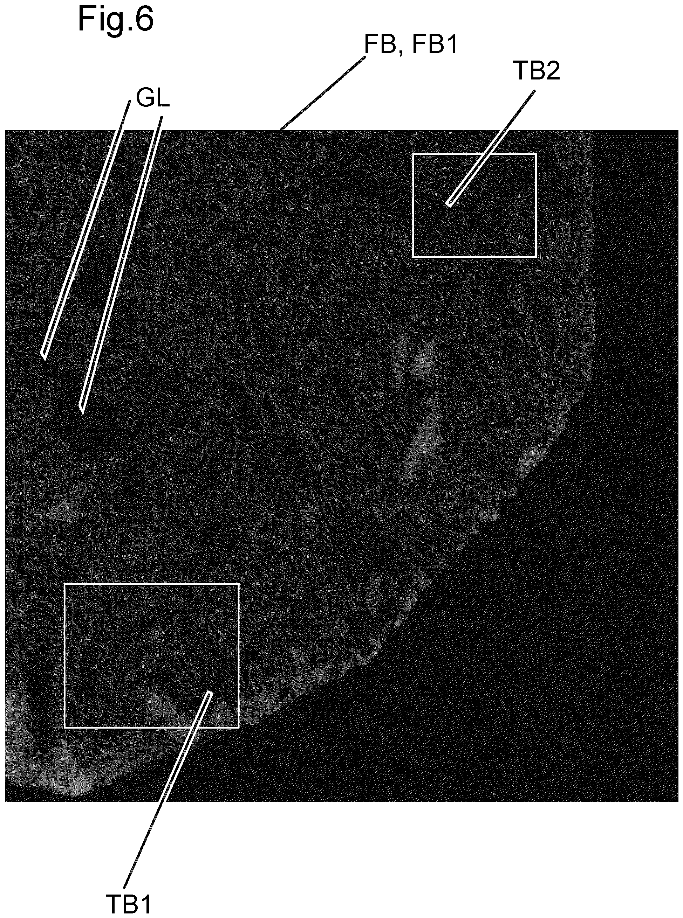

Die Figur 6 zeigt als ein Fluoreszenzbild FB ein beispielhaftes Fluoreszenzbild FB1 eines Organschnittes einer Rattenniere. Es ergeben sich in den Teilbereichen TB1, TB2, jeweils indiziert durch helle Rechtecke, Regionen, in welchen ein für einen diagnostischen PositivFall zu erwartendes LKM-Muster präsent sein kann, zumindest teilweise. Ein solches LKM-Muster wird auch Liver-Kidney-Microsomal-Muster genannt, da sogenannte LKM-Antikörper (Liver-Kidney Microsomal Antibodies) in einer positiven Patientenprobe präsent sind. Das LKM-Muster ist ein erster Fluoreszenzmustertyp.

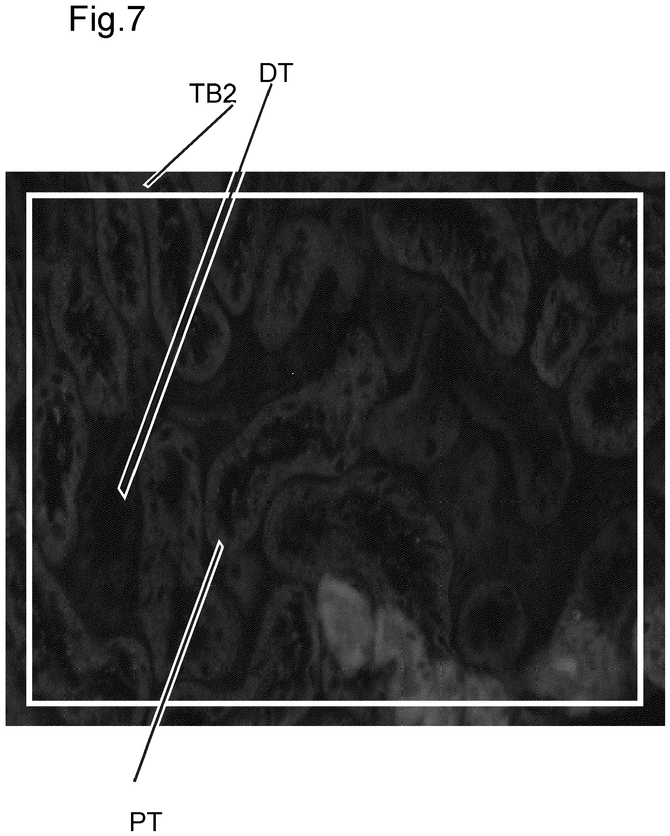

Ein solches LKM-Muster kann in der Figur 7 in einer vergrößerten Darstellung des Teilbereiches TB1 zumindest teilweise genauer betrachtet werden. Bei diesem LKM-Muster ist im Bereich der Nebennierenrinde eine fein granuläre cytoplasmatische Fluoreszenz der proximalen Tubuli PT sichtbar. Die distalen Tubuli DT sind hierbei negativ. Auch die in der Figur 6 gekennzeichneten Glomeruli GL als ein weiteres Teilmuster des LKM-Musters sind hierbei negativ. Eine solche Mehrstufigkeit der Fluoreszenz der proximalen Tubuli PT mit einer fein granulären cytoplasmatischen Fluoreszenz gegenüber negativen distalen Tubuli DT und negativen Glomeruli GL macht ein solches LKM-Muster aus.

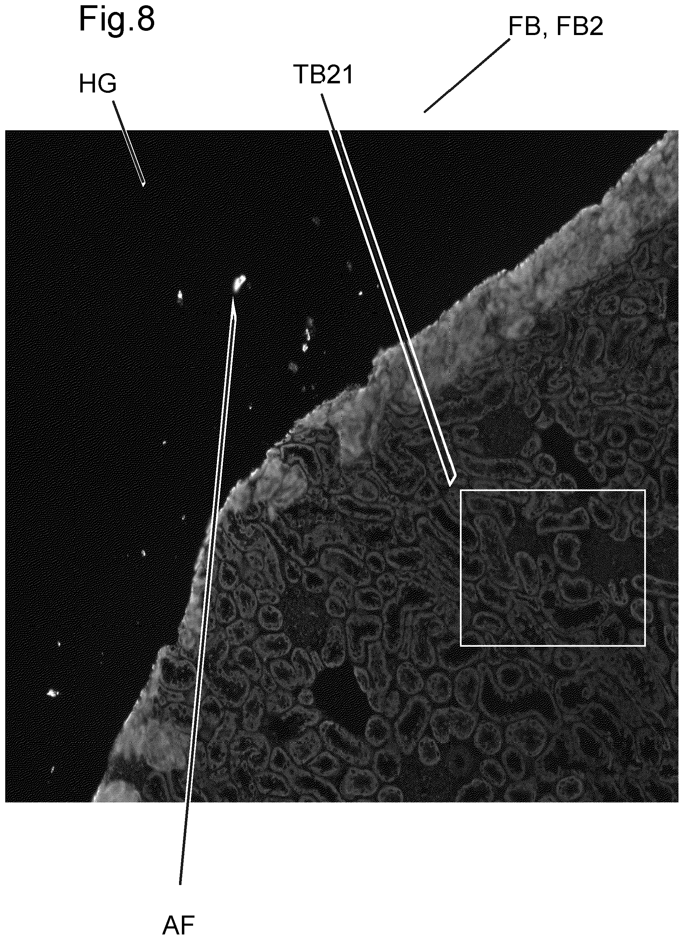

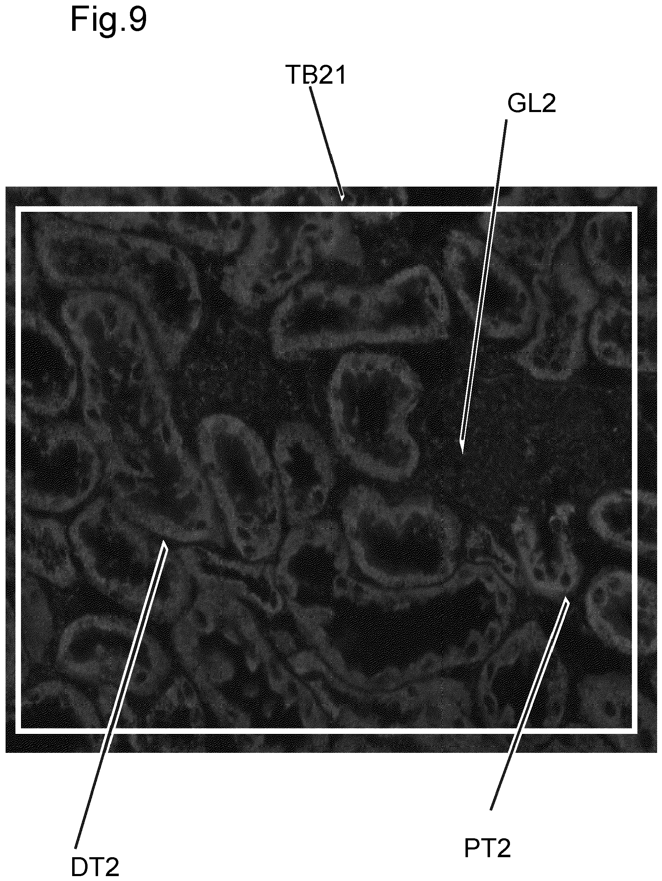

Die Figur 8 zeigt ein weiteres Fluoreszenzbild FB2 eines anderen Organschnittes einer Rattenniere, wobei in einem Teilbereich TB21, vergrößert dargestellt in der Figur 9, ein sogenanntes AMA-Muster so genannter Antimitochondrialer Antikörper vorhanden ist. Für ein solches AMA-Muster ist das Cytoplasma der proximalen Tubuli PT2 und der distalen Tubuli DT2 mit einer granulären Fluoreszenz gefärbt, während die Glomeruli GL2 nur schwach leuchten bzw. eine schwache Fluoreszenzfärbung aufweisen. Das AMA-Muster ist also ein zweiter Fluoreszenzmustertyp, dessen Präsenz detektiert werden kann.

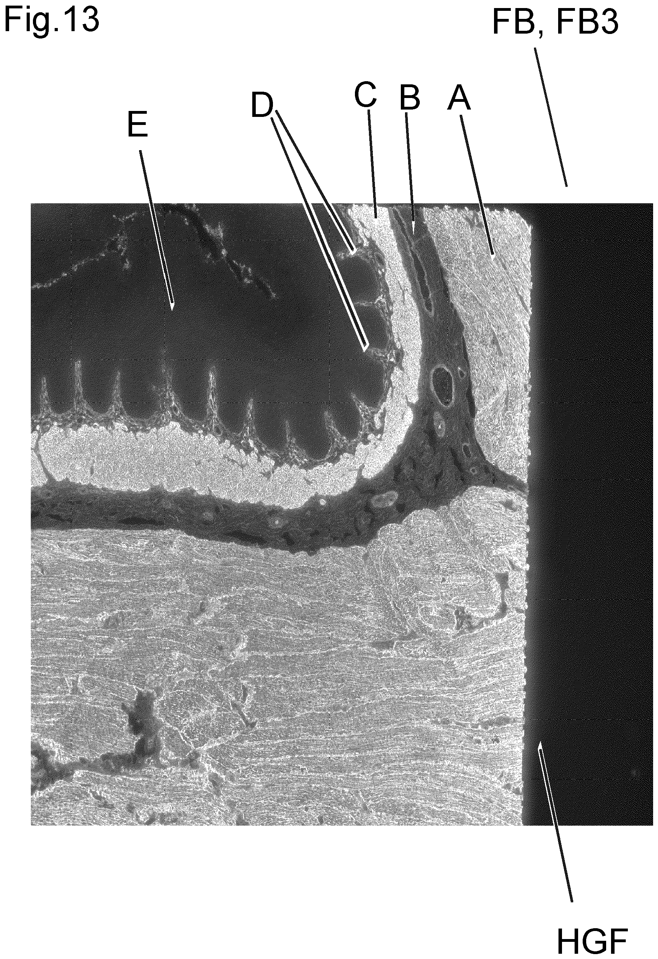

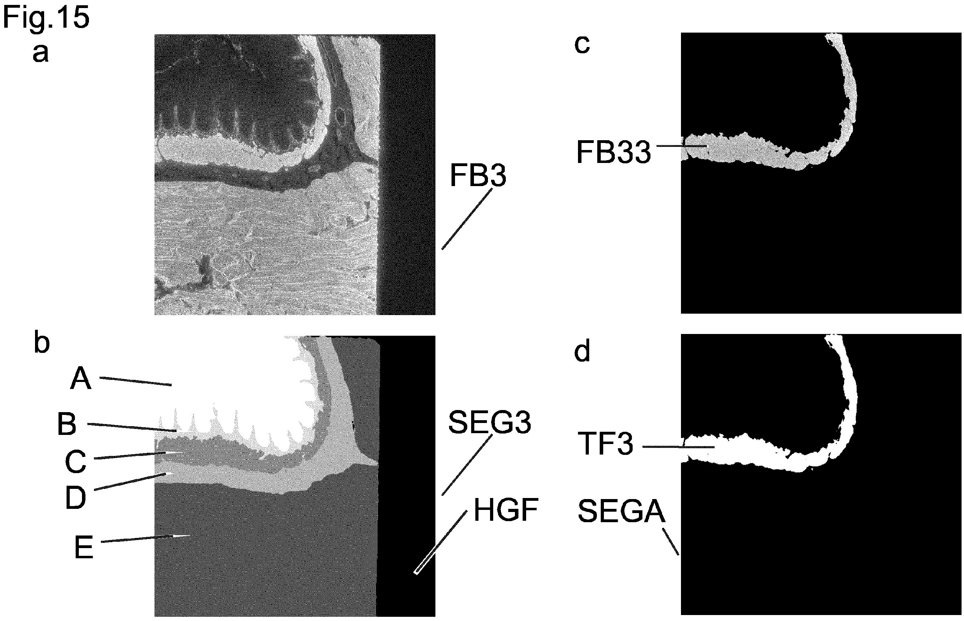

Die Figur 13 zeigt ein Fluoreszenzbild einer anderen Art von Organschnitt, hier ein Bild FB3 eines Schnittes eines Oesophagus eines Affen. Eine bestimmte Teilfläche des Organschnittes, nämlich der Bereich *muscularis mucosae,* indiziert durch das Bezugszeichen C, ist für eine Ausbildung eines sogenannten Endomysium-Musters relevant. Figur 15c zeigt hierzu den entsprechenden ausgespielten relevanten Bereich des *muscularis mucosae,* in welchem in einem positiven Fall eine netzartigeFärbung dieses *muscularis mucosae* präsent ist. Der Bereich HGF stellt den sogenannten Hintergrund des Fluoreszenzbildes bzw. des Objektträgers dar, auf welchem kein Organschnitt vorhanden ist.

Es ergibt sich also für verschiedene Organschnitte die Aufgabe, einen oder mehrere Fluoreszenzmustertypen hinsichtlich seiner Präsenz bzw. ihrer Präsenzen zu Detektieren, welches durch digitale Bildverarbeitung durchgeführt werden kann.

Die erfindungsgemäße Aufgabe wird gelöst durch ein Verfahren zum Detektieren wenigstens einer potentiellen Präsenz wenigstens eines Fluoreszenzmustertyps auf einem Organschnitt mittels Immunfluoreszenzmikroskopie mittels digitaler Bildverarbeitung. Das Verfahren weist verschiedene Schritte auf. Zunächst wird ein Organschnitt auf einem Objektträger bereitgestellt. Es erfolgt dann ein Inkubieren des Organschnittes mit einer flüssigen Patientenprobe, welche potenziell primäre Antikörper aufweist. Es erfolgt dann ein Inkubieren des Organschnittes mit sekundären Antikörpern, welche mit einem Fluoreszenzfarbstoff markiert sind. Es erfolgt ferner ein Erfassen eines Fluoreszenzbildes des Organschnittes in einem Farbkanal, welcher zu dem Fluoreszenzfarbstoff korrespondiert. Ferner erfolgt ein Bestimmen einer Teilfläche des Fluoreszenzbildes, welche für eine Ausbildung des Fluoreszenzmustertyps relevant ist, durch Segmentieren des Fluoreszenzbildes mittels eines ersten neuronalen Netzes. Ferner erfolgt ein Bestimmen des Konfidenzmaßes, welches eine tatsächliche Präsenz des Fluoreszenzmustertyps indiziert, auf Basis des Fluoreszenzbildes mittels eines zweiten neuronalen Netzes. Ferner erfolgt ein Bestimmen einer Validitätsinformation, welche einen Grad einer Validität des Konfidenzmaßes indiziert, auf Basis der zuvor bestimmten Teilfläche. Schließlich erfolgt ein Ausgeben des Konfidenzmaßes der tatsächlichen Präsenz des Fluoreszenzmustertyps und der Validitätsinformation.

Zur Erläuterung eines oder mehrerer möglicher Vorteile des vorgeschlagenen Verfahrens folgen nun genauere Ausführungen.

Prinzipiell ist es für eine digitale Bildverarbeitung bekannt, ein Gesamtbild wie zum Beispiel ein gesamtes Fluoreszenzbild mittels eines einzigen neuronalen Netzes zu analysieren und hierdurch eine Präsenz eines zu erwartenden Musters zu detektieren. Hierbei kann also das gesamte Bild auf einmal dem Klassifikator bzw. dem neuronalen Netz zugeführt werden, welches dann ein Konfidenzmaß bezüglich einer Präsenz eines bestimmten Musters ermitteln kann.

Die Erfinder haben erkannt, dass sich bei der Immunfluoreszenzmikroskopie basierend auf Organschnitten in der Produktion bestimmte negative Auswirkungen ergeben können, welchen das erfindungsgemäße Verfahren entgegenwirken kann. Ein Organschnitt wie aus der Figur 6 bzw. der Figur 8 ist nicht über die gesamte Fläche des Bildes FB1 bzw. FB2 präsent, da derartiges biologisches Material im Zuge einer Produktionsverarbeitung zur Erstellung von mit Organschnitten bedeckten Objektträgern mitunter nicht vollflächig auf den Objektträger aufgebracht wird. Notwendiges Organmaterial steht nicht unbegrenzt zur Verfügung. Ein größerer Organschnitt wird zunächst auf eine Trägerfläche aufgebracht und dann die Trägerfläche in Teilträgerflächen bzw. Objektträger zerteilt, vorzugsweise mittels Schneiden, so dass es gerade in Randbereichen des Organschnittes zu einer nur teilweisen Abdeckung eines Objektträgers kommen kann, wie in der Figur 6 bzw. der Figur 8 zu sehen ist. Wird dann ein solches Fluoreszenzbild FB1 bzw. FB2 mittels eines neuronalen Netzes als Ganzes ausgewertet, so kann zwar dann das neuronale Netz ein Konfidenzmaß hinsichtlich einer Präsenz eines Fluoreszenzmustertyps auf Basis des gesamten Bildes FB1 bzw. FB2 ermitteln. Aufgrund einer möglicherweise aber mangelnden flächigen Gesamtabdeckung des Objektträgers bzw. des Fluoreszenzbildes durch den Organschnitt kann aber jener Teilbereich des Bildes, auf welchem kein Organschnitt vorhanden ist, als Bildinformation die Qualität der Bestimmung des Konfidenzmaßes durch das neuronale Netz beeinträchtigen. Hierdurch kann es also möglich sein, dass ein Diagnostiker, wie beispielsweise ein Arzt, nur zu einem gewissen Grade auf das durch das neuronale Netz bestimmte Konfidenzmaß vertrauen kann, um dann seinerseits eine Entscheidung für eine finale Diagnostik zu treffen. Insbesondere in dem Fall, dass der Organschnitt ein Ösophagus eines Affen ist, kann eine Musterdetektion nur in einem bestimmten Teilbereich des Organschnittes sicher erfolgen, hier in dem Bereich C, Bereich des *muscularis mucosae,* aus dem Beispielbild der Figur 13. Dieser Bereich C bzw. die entsprechende Teilfläche stellt nur einen bestimmten flächigen Anteil des Fluoreszenzbildes. Alternativ oder zusätzlich kann ein weiterer negativer technischer Effekt auftreten: Zur hinreichenden Darstellung bzw. Erkennbarkeit von Mustern in einem Fluoreszenzbild werden mitunter die Fluoreszenzbilder unter Verwendung von Mikroskopoptiken bestimmter optischer Vergrößerungen erfasst. Dies kann zu einem Fluoreszenzbild führen, welches nicht den gesamten Objektträger und auch nicht den gesamten Organschnitt erfasst bzw. darstellt. Auch hierdurch kann es notwendig sein festzustellen, wieviel Teilfläche des Fluoreszenzbildes durch den Organschnitt bzw. einen für eine Musterausbildung relevanten Bereich des Organschnittes abgedeckt wird.

Dadurch, dass das erfindungsgemäße Verfahren nicht nur ein einzelnes neuronales Netz verwendet, um eine Gesamtbild zu analysieren und dann eine Präsenz eines Fluoreszenzmustertyps zu detektieren, sondern dass zwei verschiedene neuronale Netze unterschiedliche Informationen generieren, ist das erfindungsgemäße Verfahren robuster als das Verfahren aus dem Stand der Technik. Besonders vorteilhaft wird eben als eine Information auch noch eine Validitätsinformation bestimmt und diese gemeinsam mit dem Konfidenzmaß ausgegeben, so dass der Nutzer bzw. Arzt eine Zusatzinformation bezüglich einer Validität des Konfidenzmaßes erhält.

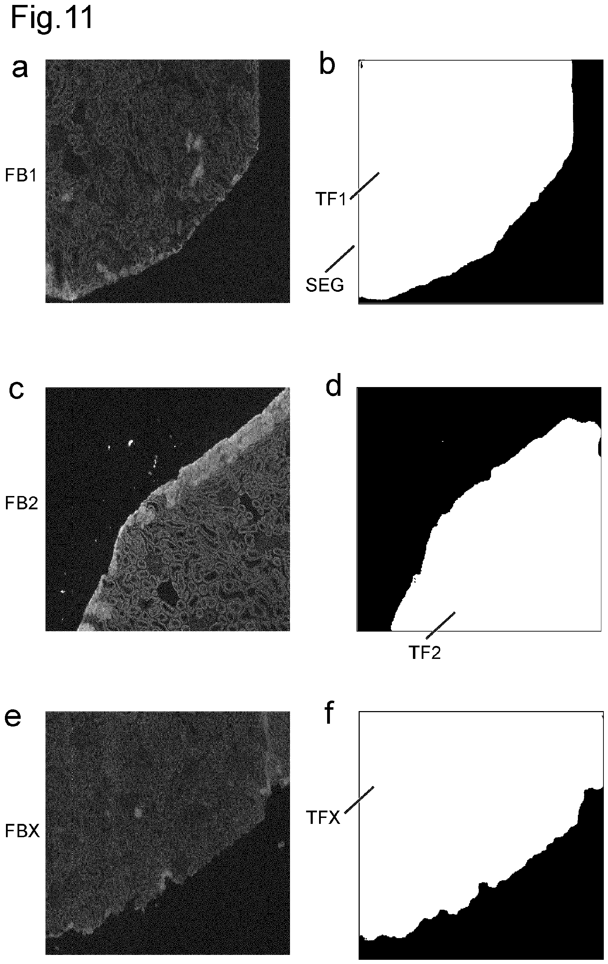

Erfindungsgemäß wird die Validitätsinformation in besonderer Weise bestimmt. Zunächst erfolgt durch ein neuronales Netz ein Bestimmen einer Teilfläche des Fluoreszenzbildes, welche für eine Ausbildung des Fluoreszenzmustertyps relevant ist. Im Beispiel des Organschnittes einer Rattenniere kann dies für das Beispiel des Bildes FB1, siehe Figur 11a, die Teilfläche TF1 aus der Figur 11b sein. Die Teilfläche TF1 korrespondiert zu eben der Teilfläche, welche der Organschnitt in dem Bild FB1 einnimmt. Für das andere Beispiel des Fluoreszenzbildes FB2, siehe Figur 11c, ist eine entsprechende Teilfläche TF2 in der Figur 11d gezeigt. In dem Beispiel eines Fluoreszenzbildes FB3 eines Ösophagus eines Affen aus der Figur 13 kann eine solche Teilfläche, welche für eine Ausbildung des Fluoreszenzmustertyps relevant ist, eine Teilfläche des Organschnittes selber sein, also ein bestimmter Organbereich. In diesem Beispiel des Bildes FB3 aus der Figur 13, welches abermals in der Figur 15a dargestellt ist, ist die entsprechende Teilfläche TF3 als heller Bereich in die Figur 15d dargestellt. Es wird erfindungsgemäß eine solche Teilfläche TF1, TF2, TF3 herangezogen, um basierend darauf die Validitätsinformation zu bestimmen.

Das erfindungsgemäße Verfahren ist insbesondere deshalb vorteilhaft, da ein Nutzer nicht nur auf ein durch ein einzelnes neuronales Netz bestimmtes Konfidenzmaß hinsichtlich einer Präsenz eines Fluoreszenzmustertyps vertrauen muss, sondern dass eben auch noch ihm eine Validitätsinformation ausgegeben wird, welche berücksichtigt, zu welchem Grade das durch das erste neuronale Netz analysierte Fluoreszenzbild durch einen relevanten Bereich bzw. eine relevante Teilfläche des Organs abgedeckt ist. Hierdurch kann also in dem Fall, dass ein aus einer Massenproduktion bereitgestellter Objektträger mit einem Organschnitt unbeabsichtigter Weise eine nur geringe Teilfläche auf dem Objektträger und dann eben auch auf dem Fluoreszenzbild aufweist, welche für die Ausbildung eines zu erwartenden Fluoreszenzmustertyps relevant ist, mittels der Validitätsinformation hierdurch eine Art Warnung erhält, dass er möglicherweise nicht nur alleine auf Basis des ausgegebenen Konfidenzmaßes seine Entscheidung fällen sollte, sondern eher berücksichtigen sollte, inwiefern tatsächlich innerhalb des Fluoreszenzbildes Teilflächen vorhanden sind, welche für die Ausbildung des Fluoreszenzmustertyps relevant sind.

Das erfindungsgemäße Verfahren ist also deshalb vorteilhaft, da die Teilaufgaben der Ermittlung des Konfidenzmaßes einer Präsenz des Fluoreszenzmustertyp als auch der Validitätsinformation nicht durch ein einzelnes neuronales Netz kombiniert geleistet werden muss, sondern weil dies in zwei Teilaufgaben aufgeteilt wird für jeweilige neuronale Netze. Hierbei kann dann das erste neuronale Netz speziell auf die Teilaufgabe des Segmentierens des Fluoreszenzbildes trainiert werden, ohne eine Präsenz von bestimmten Fluoreszenzmustertypen detektieren zu müssen. Es muss also das erste neuronale Netz zur Segmentierung des Fluoreszenzbildes lediglich für eine Segmentierung des Fluoreszenzbildes hinsichtlich bestimmter Teilflächen trainiert werden, so dass Trainingsdaten verwendet werden können, bei denen bestimmte Fluoreszenzmustertypen nicht präsent sein müssen und solche Präsenz auch nicht in Form von Meta-Daten einer sogenannten "ground truth" für das Training bereitgestellt werden müssen. Es reicht nämlich aus, die Aufgabe der Segmentierung des Fluoreszenzbild auf Basis von Trainingsdaten bzw. Trainingsbildern durchzuführen, welche als Konnotationsform-Information die Unterteilung des Fluoreszenzbildes in verschiedene Segmentierungsbereiche aufweist, wie beispielsweise die Segmentierungsinformation aus der Figur 11b oder aber eine Segmentierungsinformation aus der Figur 15b.

Das zweite neuronale Netz als ein Klassifikationsnetzwerk muss dann eben nicht auf die Erkennung solcher Teilflächen bzw. Segment trainiert werden, sondern muss nur so trainiert werden, dass es Präsenzen von Fluoreszenzmustertypen detektiert. Insbesondere kann das zweite neuronale Netz vorzugsweise das Konfidenzmaß auf Basis des Fluoreszenzbildes selber und auch auf Basis des segmentierten Fluoreszenzbildes bzw. der daraus gewonnenen Segmentierungsinformation ermitteln. Hierbei kann also beispielsweise in das zweite neuronale Netz zur Ermittlung des Konfidenzmaßes vorzugsweise nicht nur das Fluoreszenzbild FB3 aus der Figur 13 sondern auch die Segmentierungsinformation SEG bzw. die Teilfläche TF3 aus der Figur 15d eingehen. Es kann dann mittels besonders bevorzugt das zweite neuronale Netz eine Teil-Fluoreszenzbild-Information, wie als Information FB33 in der Figur 15c dargestellt, verstärkt berücksichtigen, und somit auf Basis dieser Teil-Fluoreszenzbild-Information FB33 die Konfidenzmaße ermitteln.

Vorteilhafte Ausführungsformen der Erfindung sind Gegenstand der abhängigen Ansprüche und werden in der folgenden Beschreibung unter teilweiser Bezugnahme auf die Figuren näher erläutert.

Vorzugsweise ist das Verfahren ausgebildet zum Detektieren jeweiliger potentieller Präsenzen jeweiliger Fluoreszenzmustertypen auf einem Organschnitt mittels Immunfluoreszenzmikroskopie und mittels digitaler Bildverarbeitung, wobei das Verfahren vorzugsweise aufweist: Bestimmen einer Teilfläche des Fluoreszenzbildes, welche für eine Ausbildung der Fluoreszenzmustertypen potentiell relevant ist, durch Segmentieren des Fluoreszenzbildes mittels eines ersten Neuronalen Netzes, Bestimmen jeweiliger Konfidenzmaße, welche jeweilige tatsächliche Präsenzen der jeweiligen Fluoreszenzmustertypen indizieren, auf Basis des Fluoreszenzbildes mittels eines zweiten neuronalen Netzes, Bestimmen einer Validitätsinformation, welche einen Grad einer Validität der Konfidenzmaße indiziert, auf Basis der zuvor bestimmten Teilfläche, Ausgeben zumindest einer Teilmenge der jeweiligen Konfidenzmaße der jeweiligen tatsächlichen Präsenzen der jeweiligen Fluoreszenzmustertypen und der Validitätsinformation.

Vorzugsweise weist das Verfahren ferner auf: Bestimmen des Konfidenzmaßes auf Basis des Fluoreszenzbildes und auf Basis des segmentierten Fluoreszenzbildes mittels des zweiten neuronalen Netzes.

Vorzugsweise weist das Verfahren ferner auf: Bestimmen des Konfidenzmaßes auf Basis des Fluoreszenzbildes und auf Basis einer Information, welche die Teilfläche indiziert, mittels des zweiten neuronalen Netzes.

Vorzugsweise weist das Verfahren ferner auf: Bestimmen der Validitätsinformation mittels Bestimmen eines Anteiles einer flächigen Abdeckung des Fluoreszenzbildes durch die Teilfläche, welche für eine Ausbildung von Fluoreszenzmustern potentiell relevant ist.

Vorzugsweise weist das Verfahren ferner auf: in dem Fall, dass ein Fluoreszenzmustertyp als tatsächlich präsent bestimmt wird, Bestimmen eines Helligkeitsgrades der Teilfläche in dem Fluoreszenzbild, welche für eine Ausbildung des Fluoreszenzmustertyps potentiell relevant ist.

Vorzugsweise weist das Verfahren ferner auf: Abschätzen eines maximalen Verdünnungsgrades der Patientenprobe, bei welchem eine Inkubation des Organschnittes mit der Patientenprobe noch zu einer Präsenz eines des Fluoreszenzmustertyps führt.

Vorgeschlagen wird ferner eine Vorrichtung zum Detektieren wenigstens einer potentiellen Präsenz wenigstens eines Fluoreszenzmustertyps auf einem Organschnitt mittels Immunfluoreszenzmikroskopie und mittels digitaler Bildverarbeitung, aufweisend eine Haltevorrichtung für einen Objektträger mit einem Organschnitt, welcher mit einer Patientenprobe, aufweisend die Autoantikörper, sowie ferner mit sekundären Antikörpern, welche jeweils mit einem Fluoreszenzfarbstoff markiert sind, inkubiert wurde, wenigstens eine Bilderfassungseinheit zum Erfassen eines Fluoreszenzbildes des Organschnittes in einem Farbkanal, welcher zu dem Fluoreszenzfarbstoff korrespondiert, sowie ferner aufweisend wenigstens eine Recheneinheit, welche ausgebildet ist, eine Teilfläche in dem Fluoreszenzbild zu bestimmen, welche für eine Ausbildung des Fluoreszenzmustertyps relevant ist, durch Segmentieren des Fluoreszenzbildes mittels eines ersten Neuronalen Netzes, ein Konfidenzmaß zu bestimmen, welches eine tatsächliche Präsenz des Fluoreszenzmustertyps indiziert, auf Basis des Fluoreszenzbildes mittels eines zweiten neuronalen Netzes, eine Validitätsinformation zu bestimmen, welche einen Grad einer Validität des Konfidenzmaßes indiziert, auf Basis der Teilfläche, sowie ferner das Konfidenzmaß der tatsächlichen Präsenzen des Fluoreszenzmustertyps und die Validitätsinformation auszugeben.

Vorgeschlagen wird ferner eine Recheneinheit, welche ausgebildet ist im Zuge einer digitalen Bildverarbeitung ein Fluoreszenzbild entgegenzunehmen, welches eine Färbung eines Organschnittes durch einen Fluoreszenzfarbstoff repräsentiert, eine Teilfläche in dem Fluoreszenzbild zu bestimmen, welche für eine Ausbildung des Fluoreszenzmustertyps relevant ist, durch Segmentieren des Fluoreszenzbildes mittels eines ersten Neuronalen Netzes, ein Konfidenzmaß zu bestimmen, welches eine tatsächliche Präsenz des Fluoreszenzmustertyps indiziert, auf Basis des Fluoreszenzbildes mittels eines zweiten neuronalen Netzes, eine Validitätsinformation zu bestimmen, welche einen Grad einer Validität des Konfidenzmaßes indiziert, auf Basis der zuvor bestimmten Teilfläche, sowie ferner das Konfidenzmaß der tatsächlichen Präsenz des Fluoreszenzmustertyps und die Validitätsinformation auszugeben.

Vorgeschlagen wird ferner eine Datennetzwerkvorrichtung, aufweisend wenigstens eine Datenschnittstelle zum Entgegennehmen eines Fluoreszenzbildes, welches eine Färbung eines Organschnittes durch einen Fluoreszenzfarbstoff repräsentiert, sowie ferner aufweisend wenigstens eine Recheneinheit, welche ausgebildet ist im Zuge einer digitalen Bildverarbeitung eine Teilfläche in dem Fluoreszenzbild zu bestimmen, welche für eine Ausbildung des Fluoreszenzmustertyps relevant ist, durch Segmentieren des Fluoreszenzbildes mittels eines ersten Neuronalen Netzes, ein Konfidenzmaß zu bestimmen, welches eine tatsächliche Präsenz des Fluoreszenzmustertyps indiziert, auf Basis des Fluoreszenzbildes mittels eines zweiten neuronalen Netzes, eine Validitätsinformation zu bestimmen, welche einen Grad einer Validität des Konfidenzmaßes indiziert, auf Basis der zuvor bestimmten Teilfläche, sowie ferner das Konfidenzmaß der tatsächlichen Präsenz des Fluoreszenzmustertyps und die Validitätsinformation auszugeben.

Vorgeschlagen wird ferner ein Verfahren zur digitalen Bildverarbeitung aufweisend: Entgegennehmen eines Fluoreszenzbildes, welches eine Färbung eines Organschnittes (S), durch einen Fluoreszenzfarbstoff repräsentiert, Bestimmen einer Teilfläche in dem Fluoreszenzbild, welche für eine Ausbildung des Fluoreszenzmustertyps relevant ist, durch Segmentieren des Fluoreszenzbildes mittels eines ersten Neuronalen Netzes, Bestimmen eines Konfidenzmaßes, welches eine tatsächliche Präsenz des Fluoreszenzmustertyps indiziert, auf Basis des Fluoreszenzbildes mittels eines zweiten neuronalen Netzes, Bestimmen einer Validitätsinformation, welche einen Grad einer Validität des Konfidenzmaßes indiziert, auf Basis der zuvor bestimmten Teilfläche, Ausgeben des Konfidenzmaßes der tatsächlichen Präsenz des Fluoreszenzmustertypes und der Validitätsinformation.

Vorgeschlagen wird ferner ein Computerprogrammprodukt, umfassend Befehle, die bei der Ausführung des Programms durch einen Computer diesen veranlassen, das Verfahren zur digitalen Bildverarbeitung durchzuführen.

Vorgeschlagen wird ferner ein Datenträgersignal, welches das Computerprogrammprodukt überträgt.

Im Folgenden wird die Erfindung anhand spezieller Ausführungsform ohne Beschränkung des allgemeinen Erfindungsgedankens anhand der Figuren näher erläutert. Dabei zeigen:

| | |
|---|---|
| Figur 1 | Schritte des erfindungsgemäßen Verfahrens gemäß einer ersten Ausführungsform, |
| Figur 2 | Schritte des erfindungsgemäßen Verfahrens gemäß einer zweiten Ausführungsform, |
| Figur 3 | einen Schritt zur Ermittlung einer Validitätsinformation, |
| Figur 4 | bevorzugte Teilschritte zur Ermittlung eines oder mehrerer Konfidenzmaße, |
| Figur 5 | bevorzugte Schritte zur Ermittlung eines maximalen Verdünnungsgrades einer Patientenprobe, bei welchem eine Inkubation eines Organschnittes mit der Patientenprobe noch zu einer Präsenz |

des Fluoreszenzmustertyps führt,

Figur 6          ein erstes Fluoreszenzbild,
Figur 7          einen Teilbereich des ersten Fluoreszenzbildes,
Figur 8          ein zweites Fluoreszenzbild,
Figur 9          einen Teilbereich des zweiten Fluoreszenzbildes,
Figur 10         eine erfindungsgemäße Vorrichtung gemäß einer bevorzugten Ausführungsform,
Figur 11         jeweilige Fluoreszenzbilder mit jeweils dazu korrespondierenden Teilflächen, welche für eine jeweilge Ausbildung von Fluoreszenzmustertypen relevant sind,
Figur 12         eine Schichtaufteilung unterschiedlicher Schichten eines Organschnittes eines Oesophagus,
Figur 13         ein drittes Fluoreszenzbild,
Figur 14a        sowie
Figur 14b        weitere Fluoreszenzbilder,
Figur 15a        das dritte Fluoreszenzbild,
Figur 15b        ein Segmentierungsergebnis des dritten Fluoreszenzbildes,
Figur 15c        eine relevante Bildinformation des dritten Fluoreszenzbildes,
Figur 15d        eine relevante Teilfläche des dritten Fluoreszenzbildes,
Figur 16a-d      korrespondierende Bilder bzw. korrespondierende Flächen analog zu Figur 15 bezogen auf ein viertes Fluoreszenzbild,
Figur 17         eine erfindungsgemäße Recheneinheit gemäß einer bevorzugten Ausführungsform,
Figur 18         eine erfindungsgemäße Datennetzwerkvorrichtung gemäß einer bevorzugten Ausführungsform,
Figur 19         ein erfindungsgemäßes Computerprogrammprodukt sowie ein erfindungsgemäßes Datenträgersignal,
Figuren 20 bis 23  Experimentalergebnisse für unterschiedliche Organschnitte
Figuren 24 bis 25  bevorzugte Ausgestaltungen des ersten und des zweiten neuronalen Netzes

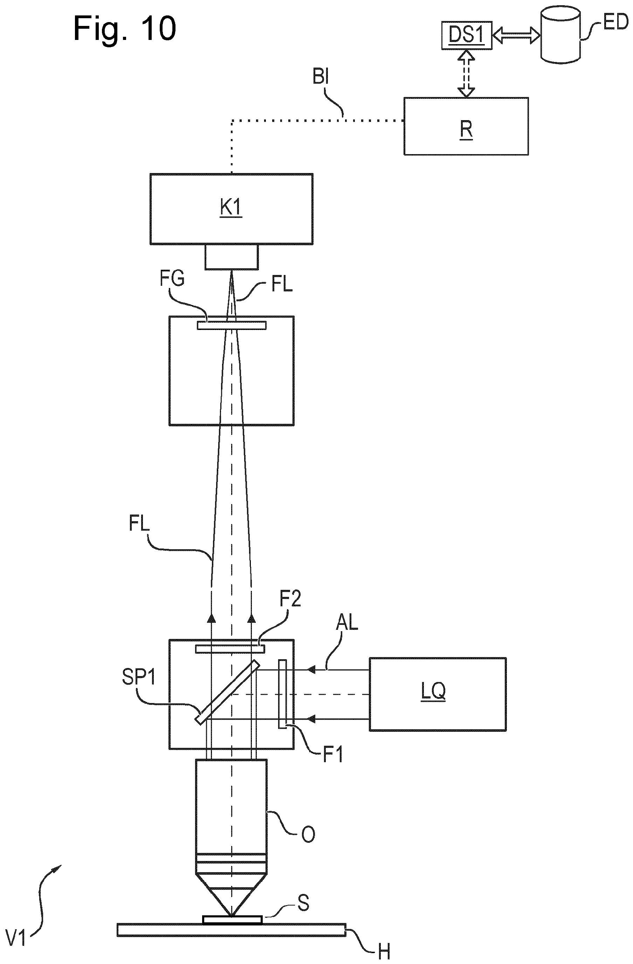

Figur 10 zeigt eine Vorrichtung V1, mittels welcher das erfindungsgemäße Verfahren vorzugsweise durchgeführt werden kann. Die Vorrichtung V1 kann als ein Fluoreszenzmikroskop bezeichnet werden. Die Vorrichtung V1 weist eine Halterung H für ein Substrat S bzw. einen Objektträger auf, welches bzw. welcher in der zuvor beschriebenen Weise inkubiert wurde. Über eine Optik O wird Anregungslicht AL einer Anregungslichtquelle LQ hin zu dem Substrat S geleitet. Sich ergebende Fluoreszenzstrahlung FL wird dann durch die Optik O zurücktransmittiert und passiert den dichroitischen Spiegel SP1 und einen optionalen optischen Filter F2. Vorzugsweise passiert die Fluoreszenzstrahlung FL einen optischen Filter FG, welcher einen Grünkanal herausfiltert. Eine Kamera K1 ist vorzugsweise eine Monochromkamera, welche dann bei Vorhandensein eines optischen Filters FG die Fluoreszenzstrahlung FL in einem Grünkanal erfasst. Gemäß einer alternativen Ausführungsform ist die Kamera K1 eine Farbbildkamera, welche ohne Verwendung des optischen Filters FG auskommt und mittels einer Bayer-Matrix das Fluoreszenzbild in dem entsprechenden Farbkanal als einen Grünkanal erfasst. Die Kamera K1 stellt die Bildinformation BI bzw. das Fluoreszenzbild an eine Recheneinheit R bereit, welche diese Bildinformation BI verarbeitet. Vorzugsweise kann die Recheneinheit R über eine Datenschnittstelle DS1 Daten wie beispielsweise ein Fluoreszenzbild, Konfidenzmaße als auch eine Validitätsinformation ausgeben bzw. bereitstellen.

Die Figur 6 zeigt ein erstes Fluoreszenzbild FB1, FB, welches einen Organschnitt einer Niere einer Ratte auf einem Objektträger repräsentiert, wobei ein sogenanntes LKM-Muster. In Teilbereichen TB1, TB2 ist ein LKM-Muster zumindest teilweise präsent, wie für den Teilbereich TB1 in der Figur 7 vergrößert dargestellt.

Die Figur 8 zeigt ein zweites Teilbild FB2, FB, auf welchem ein sogenanntes AMA-Muster als ein Mustertyp präsent ist, wie in der Figur 9 für ein Teilbildbereich TB21 vergrößert dargestellt.

Das erste Fluoreszenzbild FB1 ist noch einmal in der Figur 11a dargestellt sowie das zweite Fluoreszenzbild FB2 in der Figur 11c. Die Figur 11e zeigt ein weiteres Fluoreszenzbild FBX, in welchen keiner der Fluoreszenzmustertypen LKM, AMA präsent ist. Prinzipiell ist es möglich, dass mehrere, unterschiedliche Fluoreszenzmustertypen gleichzeitig präsent sind. In den Beispielen der Fluoreszenzbilder FB1 bzw. FB2 aus den Figuren 6 bzw. 8 ist jedoch jeweils nur ein einzelnes Muster präsent.

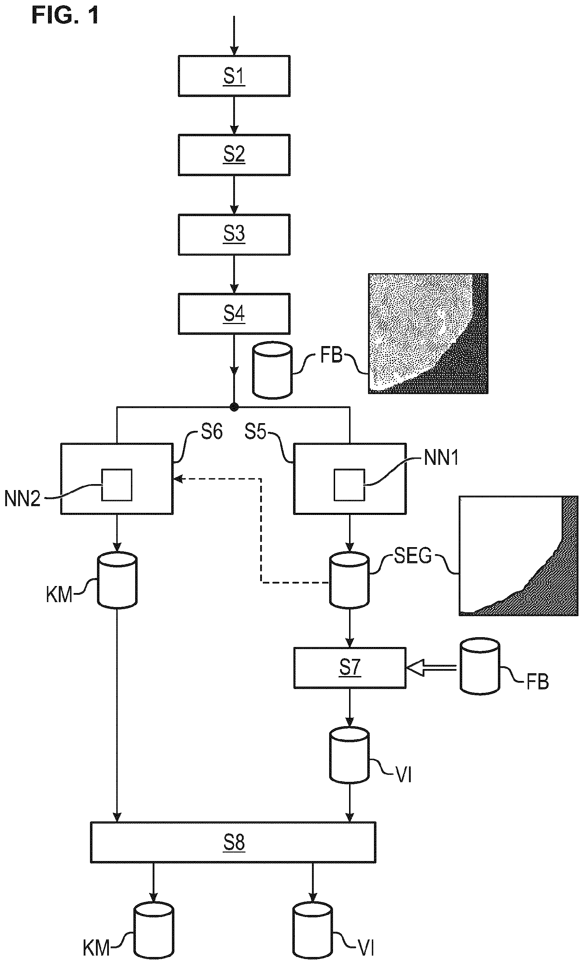

Die Figur 1 zeigt Schritte des erfindungsgemäßen Verfahrens gemäß einer bevorzugten Ausführungsform. In einem ersten Schritt S1 erfolgt ein Bereitstellen eines Organschnittes auf einem Objektträger. In einem zweiten Schritt S2 erfolgt ein Inkubieren des Organschnittes mit einer flüssigen Patientenprobe, welche potenziell primäre Antikörper aufweist. In einem Schritt S3 erfolgt ein Inkubieren des Organschnittes mit sekundären Antikörpern, welche mit einem Fluoreszenzfarbstoff markiert sind. In einem Schritt S4 erfolgt ein Erfassen eines Fluoreszenzbildes FB des Organschnittes in einem Farbkanal, welcher zu dem Fluoreszenzfarbstoff korrespondiert. Dieses beinhaltet insbesondere ein Bestrahlen des Objektträgers mit Anregungsstrahlung. Es erfolgt dann in einem Schritt S5 ein Bestimmen einer Teilfläche des Fluoreszenzbildes FB, welche für eine Ausbildung mindestens eines Fluoreszenzmustertyps relevant ist, durch Segmentieren des Fluoreszenzbildes FB mittels eines ersten neuronalen Netzes NN1. Hierdurch wird eine Segmentierungsinformation SEG gewonnen. Diese Segmentierungsinformation SEG indiziert die bestimmte Teilfläche des Fluoreszenzbildes.

In dem bevorzugten Beispiel, dass der Organschnitt ein Organschnitt einer Niere einer Ratte ist, ist die Teilfläche des Fluoreszenzbildes jene Fläche, welche der Organschnitt auf dem Fluoreszenzbild einnimmt, wie in der Figur 1 beispielhaft dargestellt und auch in der Figur 11b als Beispiel einer Teilfläche TF1 bzw. Segmentierungsinformation SEG für das Beispiel des ersten Fluoreszenzbild FB1 aus der Figur 11a dargestellt.

In dem bevorzugten Beispiel, dass der Organschnitt ein Organschnitt eines Oesophagus eines Affen ist, wie in dem dritten Fluoreszenzbild FB3 aus der Figur 13 dargestellt, stellt die Teilfläche des Fluoreszenzbildes eine Teilfläche des Organschnittes dar, welche als Teilfläche TF3 bzw. Segmentierungsinformation SEG für das dritte Fluoreszenzbild FB3 in der Figur 15d als weißer Bereich dargestellt ist. Hierbei handelt es sich also bei der Teilfläche des Fluoreszenzbildes um eine Unterteilfläche des Organschnittes bzw. um einen bestimmten Teil-Flächenbereich des Organschnittes.

In einem Schritt S6 aus der Figur 1 wird auf Basis des Fluoreszenzbildes FB mittels eines zweiten neuronalen Netzes NN2 ein Konfidenzmaß oder mehrere Konfidenzmaße KM bestimmt, welches bzw. welche eine tatsächliche Präsenz des Fluoreszenzmustertyps bzw. tatsächliche Präsenzen der Fluoreszenzmustertypen indiziert bzw. indizieren.

Für das Beispiel einer Rattenniere können Präsenzen der unterschiedlichen Fluoreszenzmustertypen LKM und AMA detektiert werden und jeweilige entsprechende Konfidenzmaße bestimmt werden. Diese Konfidenzmaße sind dann als Konfidenzmaßinformation KM in der Figur 1 gegeben.

In einem Schritt S7 wird auf Basis der zuvor bestimmten Teilfläche bzw. der zuvor bestimmten Segmentierungsinformation eine Validitätsinformation VI bestimmt, welche einen Grad einer Validität des Konfidenzmaßes bzw. der Konfidenzmaße KM indiziert. In einem Schritt S8 wird das Konfidenzmaß bzw. die Konfidenzmaße KM als auch die Validitätsinformation VI ausgegeben.

Vorzugsweise ist das Verfahren also ausgebildet zum Detektieren jeweiliger potentieller Präsenzen jeweiliger Fluoreszenzmustertypen auf einem Organschnitt mittels Immunfluoreszenzmikroskopie und digitaler Bildverarbeitung. Diese Fluoreszenzmustertypen sind vorzugsweise ein LKM-Muster und ein AMA-Muster für einen Organschnitt in Form einer Rattenniere. Hierbei erfolgt also vorzugsweise ein Bestimmen einer Teilfläche TF1 des Fluoreszenzbildes FB1, welche für eine Ausbildung der Fluoreszenzmustertypen potentiell relevant, ist durch Segmentieren des Fluoreszenzbildes FB1 mittels eines ersten neuronalen Netzes NN1. Ferner erfolgt vorzugsweise ein Bestimmen jeweiliger Konfidenzmaße KM, welche jeweilige tatsächliche Präsenzen der jeweiligen Fluoreszenzmustertypen indizieren, auf Basis des Fluoreszenzbildes FB1 mittels eines zweiten neuronalen Netzes NN2. Vorzugsweise erfolgt ein Bestimmen einer Validitätsinformation VI, welche einen Grad einer Validität der Konfidenzmaße KM indiziert auf Basis der zuvor bestimmten Teilfläche TF1. Vorzugsweise erfolgt ein Ausgeben zu mindestens einer Teilmenge der jeweiligen Konfidenzmaße KM der jeweiligen tatsächlichen Präsenzen der jeweiligen Fluoreszenzmustertypen und der Validitätsinformation VI.

In dem Ausführungsbeispiel aus der Figur 1 erfolgt vorzugsweise das Bestimmen des Konfidenzmaßes bzw. der Konfidenzmaße KM lediglich auf Basis des Fluoreszenzbildes FB. Für das Beispiel des Fluoreszenzbildes FB1 aus der Figur 6 wurde bei im Weiteren dargelegten Detektionsergebnissen das gesamte Fluoreszenzbild FB1 durch das neuronale Netz NN2 verarbeitet, um die Konfidenzmaße zu bestimmen. Vorzugsweise kann, wie in Figur 1 durch einen gestrichelten Pfeil dargestellt, zusätzlich zum Fluoreszenzbild FB das segmentierte Fluoreszenzbild bzw. die Segmentierungsinformation SEG in dem Schritt S6 ebenfalls in das neuronale Netz NN2 eingehen und somit das neuronale Netz NN2 das Konfidenzmaß bzw. die Konfidenzmaße auf Basis des Fluoreszenzbildes FB und der zuvor bestimmten Teilfläche TF1 bzw. der Segmentierungsinformation SEG bestimmen. Es kann dann also das neuronale Netz NN2 verstärkt die Bildinformationen des Teil-Fluoreszenzbildes berücksichtigen, welche zu der detektierten Teilfläche TF1 korrespondiert. Hierdurch kann dann bevorzugt eine Nicht-Berücksichtigung von Bildinformationen und Artefakten in dem sogenannten Hintergrundbildbereich außerhalb des relevanten Organschnittes durch das zweite neuronale Netz NN2 erreicht werden. Figur 8 zeigt hierzu beispielhafte Artefakte AF in dem Hintergrundbereich HG des zweiten Fluoreszenzbildes FB2.

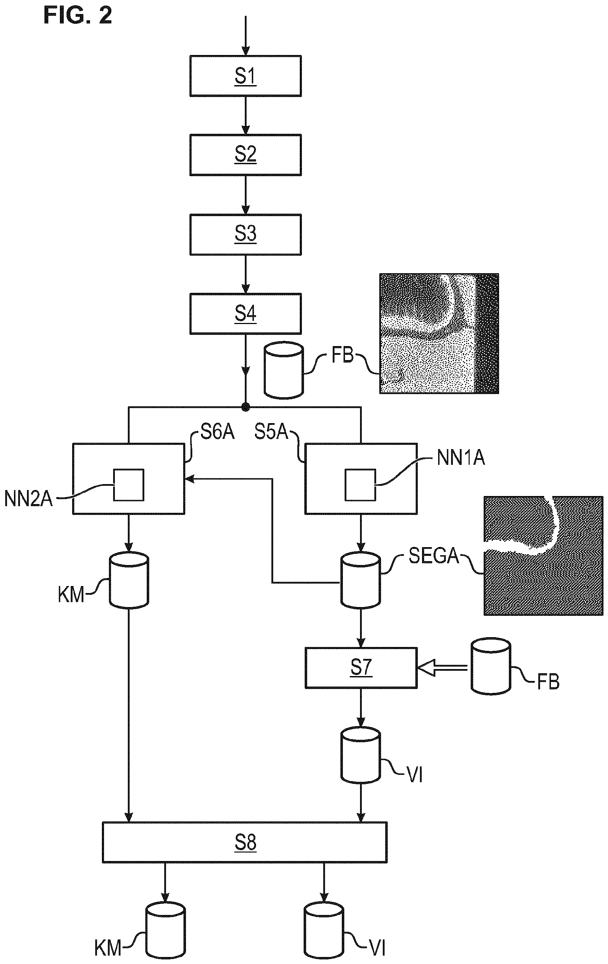

Die Figur 2 zeigt eine bevorzugte Ausführungsform des erfindungsgemäßen Verfahrens. Die Ausführungsform des erfindungsgemäßen Verfahrens in Figur 2 kann bevorzugt für Fluoreszenzbilder von Organschnitten eines Oesophagus von Affen angewendet werden. Die Schritte S1 bis S4 korrespondieren zu den Schritten S1 bis S4 aus der Figur 1.

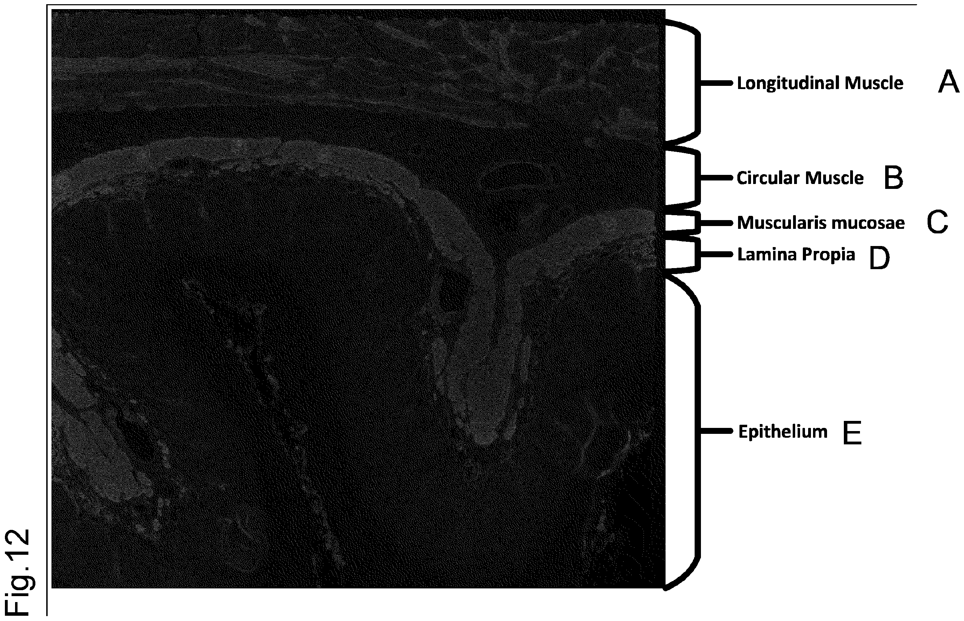

Die Figur 12 verdeutlicht die Besonderheit bei der Aufgabe eines Bestimmens einer Präsenz eines Fluoreszenzmusters im Fall eines Organschnittes in Form eines Oesophagus-Organschnittes eines Affen. Ein solcher Organschnitt eines Oesophagus weist unterschiedliche Teilflächen des Organschnittes A, B, C, D, E auf. Ein Teilbereich bzw. eine Teilfläche des Oesophagus ist der *longitudinal musculae A.* Ein weiterer Teilbereich bzw. eine weitere Teilfläche ist der *circula musculae 8.* Ein weiterer Teilbereich bzw. eine weitere Teilfläche ist der *muscularis mucosae C.* Ein weiterer Teilbereich bzw. eine weitere Teilfläche ist *lamina propria D.* Ein weiterer Teilbereich bzw. eine weitere Teilfläche ist *epithelium E.*

Die Figur 13 zeigt für das Fluoreszenzbild FB3 eine solche Indizierung entsprechender Teilbereiche bzw. Teilflächen A, B, C, D, E des Organschnittes sowie einen Hintergrundbereich HGF des Fluoreszenzbildes FB3, in welchem kein Organschnitt vorhanden ist.

Bei dem Oesophagus ist für eine Ausbildung des Fluoreszenzmustertyps *Endomysium* die Teilfläche bzw. der Teilbereich C, *muscularis mucosae,* relevant, während die anderen Teilflächen bzw. anderen Teilbereiche A, B, D, E trotz möglicher Färbung bzw. Fluoreszenzen nicht für die Detektion des Fluoreszenzmustertyps *Endomysium* relevant sind.

Wie in der Figur 2 gezeigt, bestimmt in einem Schritt S5A ein erstes neuronales Netz NN1A für das Fluoreszenzbild FB3, siehe Figur 15a, zunächst eine Teilfläche bzw. eine Segmentierungsinformation SEG3, wie in Figur 15b dargestellt, wobei unterschiedliche Teilflächen bzw. Segmentierungsebenen A, B, C, D, E die entsprechenden Teilflächen korrespondierender Organschichten repräsentieren.

In einem Schritt S6A wird dann mittels eines zweiten neuronalen Netzes NN2A auf Basis der zuvor bestimmten Teilfläche TF3 bzw. der Segmentierungsinformation SEGA, siehe Figur 15d, und auf Basis des Fluoreszenzbildes FB ein Konfidenzmaß KM bestimmt, welches die Präsenz des Fluoreszenzmustertyps *Endomysium* indiziert.

Das zweite neuronale Netz NN2A kann dann mittels der Teilfläche TF3 bzw. der Segmentierungsinformation SEGA einen relevanten Fluoreszenzbildbereich FB33, dargestellt in der Figur 15c, verstärkt berücksichtigen, um die Präsenz des Fluoreszenzmustertyps *Endomysium* zu detektieren und ein entsprechendes Konfidenzmaß zu bestimmen. Es kann dann also das neuronale Netz NN2A verstärkt die Bildinformationen des Teil-Fluoreszenzbildes verarbeiten, welche zu der detektierten Teilfläche TF3 korrespondiert. Hierdurch kann dann bevorzugt eine Nicht-Berücksichtigung von Bildinformationen in nicht relevanten Bildbereichen außerhalb des relevanten Organabschnittes, muscularis mucosae, durch das zweite neuronale Netz NN2A erreicht werden.

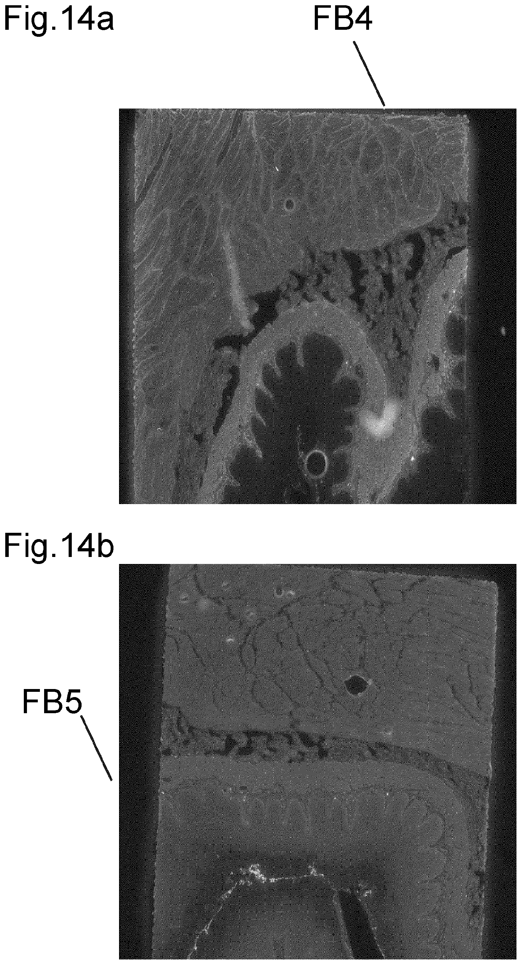

Die Figur 14a zeigt ein weiteres Fluoreszenzbild FB4 als ein viertes Fluoreszenzbild, welches im Bereich *muscularis mucosae* nur eine schwache Färbung bzw. ein schwach ausgeprägtes Muster *Endomysium* aufweist.

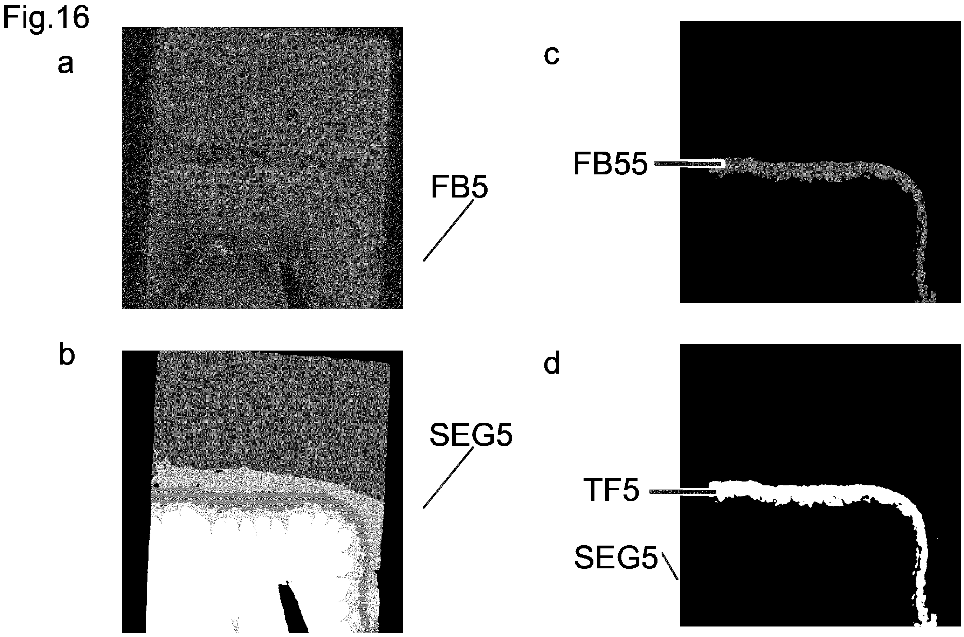

Figur 14b zeigt ein fünftes Fluoreszenzbild FB5 eines Organschnittes eines Oesophagus, wobei bei diesem Fluoreszenzbild FB5 keine Färbung des Bereiches *muscularis mucosae* durch das Muster Endomysium vorliegt. Dieses fünfte Fluoreszenzbild FB5 ist noch einmal in der Figur 16a dargestellt. Die Figur 16b zeigt hierzu ein Segmentierungsergebnis SEG5 durch Segmentierung des Fluoreszenzbildes FB5 mittels des zweiten neuronalen Netzes. Die Figur 16d zeigt hierzu die dann ermittelte Segmentierungsinformation SEGA, welche als weißen Bereich die Teilfläche TF5 indiziert. Die Figur 16c zeigt hierzu den durch das erste neuronale Netz zu verwendenden Bildbereich FB55 in grauer Schattierung.

Durch gleichzeitige Betrachtung des Fluoreszenzbildbereiches FB55 im Falle eines sogenannten Negativfalls gegenüber dem Fluoreszenzbildbereich FB33 aus der Figur 15c in einem Positivfall wird die Leistungsfähigkeit des vorgeschlagenen Verfahrens deutlich, da der Fluoreszenzbildbereich FB33 eindeutig heller eingefärbt ist als der Fluoreszenzbildbereich FB55.

Die Figur 11e zeigt in einem Fluoreszenzbild FBX einen Schnitt einer Rattenniere für einen Negativfall. Auch hier kann eine für eine Ausbildung von Fluoreszenzmustertypen relevante Teilfläche TFX, dargestellt in der Figur 11f, sicher detektiert werden.

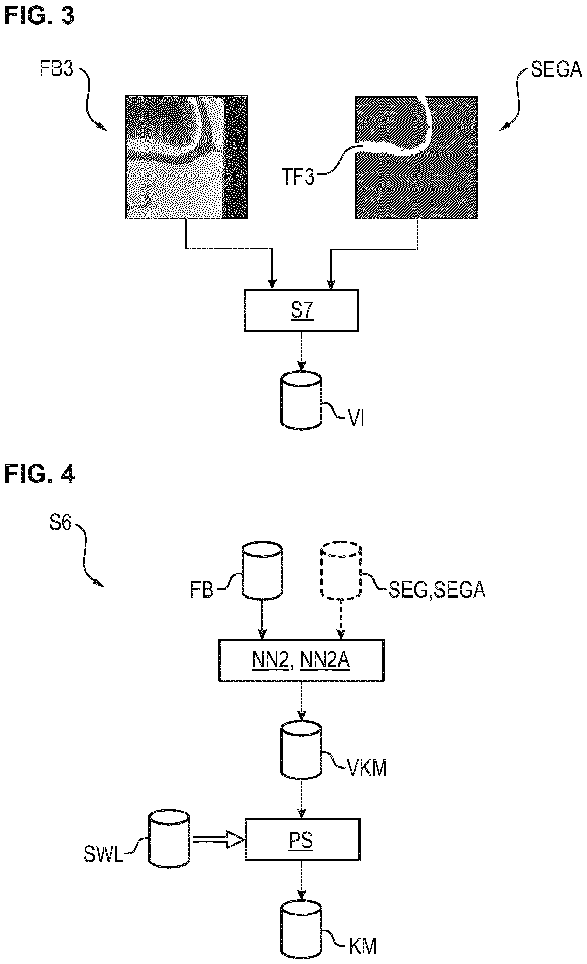

Die Figur 3 zeigt eine bevorzugte Ausführungsform des Schrittes S7, in welchem die Validitätsinformation VI bestimmt wird. Dieses ist hier dargestellt für das Beispiel eines Fluoreszenzbildes FB3 eines Oesophagus als Organschnitt. In dem Schritt S7 geht sowohl das Fluoreszenzbild FB3 als Information ein als auch die Segmentierungsinformation SEGA, welche die Teilfläche TF3 indiziert, welche für eine Ausbildung eines Fluoreszenzmusters potentiell relevant ist.

In dem Schritt S7 wird dann ein Anteil einer Flächenabdeckung des Fluoreszenzbildes FB3 durch die Teilfläche TF3 bestimmt. Dieses ist insbesondere eine prozentuale Abdeckung des Fluoreszenzbildes FB3 durch die Teilfläche TF3. Durch einfache Bestimmung der Größe der Gesamtfläche des Fluoreszenzbildes FB3 und der Größe der Teilfläche TF3 kann dieser Anteil der Flächenabdeckung des Fluoreszenzbildes FB3 durch die Teilfläche TF3 bestimmt werden, vorzugsweise als ein prozentualer Anteil. Liegt dieser Anteil der Flächenabdeckung des Fluoreszenzbildes durch die Teilfläche oberhalb eines bereitgestellten, vorbestimmten Schwellenwertes SWVI, so wird darauf entschieden, dass die flächige Abbildung des Fluoreszenzbildes durch die Teilfläche hinreichend ist. In diesem Fall wird dann vorzugsweise als Validitätsinformation VI der symbolische Wert 1 ausgegeben. Der symbolische Wert 1 repräsentiert vorzugsweise die Aussage "valide". Liegt der Anteil der Flächenabdeckung des Fluoreszenzbildes durch die Teilfläche unterhalb des vorgegebenen, vorbestimmten Schwellenwertes SWVI, so wird dann vorzugsweise als Validitätsinformation VI ein symbolischer Wert 0 ausgegeben. Der symbolische Wert 0 repräsentiert vorzugsweise die Information "nicht valide". Es kann also als Validitätsinformation VI ein Wert "valide" oder einen Wert "nicht valide" ausgegeben werden. Der Schwellenwert SWVI kann vorzugsweise für einen prozentualen Anteil einer Flächenabdeckung für das Beispiel einer Rattenniere der Wert 20-25 % sein. Der Schwellenwert SWVI kann vorzugsweise für einen prozentualen Anteil einer Flächenabdeckung für das Beispiel eines Ösophagus eines Affen der Wert 10% sein.

Figur 4 zeigt Details zur Bestimmung eines oder mehrerer Konfidenzmaße auf Basis des Fluoreszenzbildes. Ein zweites neuronales Netz NN2, NN2A nimmt das Fluoreszenzbild FB entgegen sowie vorzugsweise zusätzlich das segmentierte Fluoreszenzbild bzw. die entsprechende korrespondierende Segmentierungsinformation SEG, SEGA.

Das zweite neuronale Netz NN2, NN2A ermittelt dann eines oder mehrere vorläufige Konfidenzmaße VKM.

Eine vorläufige Konfidenzmaßinformation VKM kann dann für beispielsweise drei Klassen mit Index i = 1 ... 3

ermittelt werden zu

$$\overrightarrow{VKM} = [VKM_1, VKM_2, VKM_3] \quad .$$

Hierbei repräsentiert ein einzelner Eintrag

$$VKM_i \quad , \qquad i = 1 \dots 3$$

ein Konfidenzmaß für die entsprechende Klasse mit dem Index i. Eine erste Klasse ist beispielsweise eine Klasse, welche eine Präsenz eines LKM-Musters repräsentiert und eine zweite Klasse ist beispielsweise eine Klasse, welche eine Präsenz eines AMA-Musters repräsentiert. Eine dritte Klasse ist beispielsweise eine Klasse, welche eine Abwesenheit bzw. Absenz eines LKM-Musters und eine gleichzeitige Abwesenheit eines AMA-Musters repräsentiert und sogenannte negative Klasse darstellt.

Die vorläufigen Konfidenzmaße

$$VKM_i \quad , \qquad i = 1 \dots 3$$

können sogenannte sigmoidale Werte sein, welche durch das zweite neuronale Netz NN2, NN2A ermittelt werden.

In einem Überprüfungsschritt PS wird dann das Konfidenzmaß oder die Konfidenzmaße KM ermittelt, vorzugsweise als

$$\overrightarrow{KM} = [KM_1, KM_2, KM_3] \quad .$$

Hierbei kann ein Schwellenwert SWL als vorbestimmter Schwellenwert vorgegeben werden. Dieser Schwellenwert SWL kann beispielsweise vom Wert 0,5 sein.

Es wird dann ein Konfidenzmaß

$$KM_i \quad , \qquad i = 1 \dots 3$$

ermittelt anhand der bevorzugten Vorschrift

$$KM_i = \begin{cases} VKM_i \ falls \ VKM_i > SWL \\ 0 \ sonst \end{cases}$$

Die so ermittelten Konfidenzmaße

$$\overrightarrow{KM} = [KM_1, KM_2, KM_3] \quad .$$

werden dann vorzugsweise ausgegeben. Der Überprüfungsschritt PS ist vorzugsweise Teil des neuronalen Netzes NN2, NN2A.

Vorzugsweise wird vor der Ausgabe dann auf eine prinzipielle Musterpräsenz entschieden, wenn eines der Konfidenzmaße der Muster, insbesondere der Muster LKM und AMA, größer als Null ist,

$$KM_i > 0 \quad , \qquad i = 1 \dots 2 \quad ,$$

wobei dann vorzugsweise in einem solchen Fall einer prinzipiellen positiven Musterpräsenz automatisch das Konfidenzmaß für den Fall "negativ" auf Null gesetzt wird

$$KM_3 := 0 \quad .$$

Ist das Konfidenzmaß des Falles "negativ" größer als Null

$$KM_3 > 0$$

und sind beide Konfidenzmaße der Muster für i=1,2 gleich Null

$$KM_i = 0 \ , \qquad i = 1\,...\,2 \quad ,$$

so wird auf "negativ" entschieden und es werden die Konfidenzmaße ausgegeben, vorzugsweise wird nur das Konfidenzmaß für "negativ"

$$KM_3$$

ausgegeben.

Sind alle Konfidenzmaße gleich Null

$$KM_i = 0 \ , \qquad i = 1\,...\,3 \quad ,$$

so kann vorzugsweise eine Warnung ausgegeben werden.

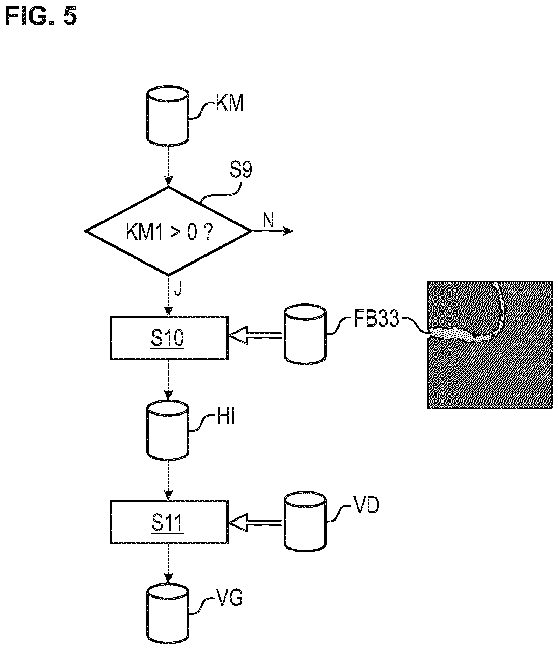

Die Figur 5 zeigt vorzugsweise Schritte zur Bestimmung eines Helligkeitsgrades der zuvor bestimmten und relevanten Teilfläche in dem Fluoreszenzbild.

In dem Fall, dass ein Fluoreszenzmustertyp als tatsächlich präsent bestimmt wird bzw. dass also durch ein Konfidenzmaß KM eine Präsenz eines Musters indiziert wird, wird dann ein Helligkeitsgrad des zu der zuvor bestimmten Teilfläche korrespondierenden Teilbildes des Fluoreszenzbildes ermittelt.

In einem Schritt S9 wird vorzugsweise zunächst überprüft, ob für ein Konfidenzmaß eines zu detektierenden Fluoreszenzmusters, beispielsweise das Konfidenzmaß KM1 für das Muster mit Index i=1, der Wert größer als 0.5 ist. Ist dies der Fall, so wird hin verzweigt zu einem Schritt S10. In dem Schritt S10 wird jenes Teilbild FB33 herangezogen, welches zu dem entsprechenden Fluoreszenzbild FB3 gehört und zu der zuvor bestimmten Teilfläche TF3, welche für eine Ausbildung des Fluoreszenzmustertyps potentiell relevant ist, korrespondiert. Es wird dann in dem Schritt S10 vorzugsweise eine Pixelstatistik auf diesem relevanten Fluoreszenzbildbereich FB33 durchgeführt. Es wird das 85%-Quartil der Helligkeitswerte aus dem Teilbild FB33 bestimmt. Die Helligkeitswerte können beispielsweise im Bereich von 0 bis 255 quantisiert sein. Dieser gesamte Quantisierungsbereich der Helligkeitswerte von 0 bis 255 kann dann in fünf Teilwertebereiche äquidistant unterteilt werden. Der erste Bereich reicht dann von 0 bis 51. Die weiteren Bereiche schließen sich in entsprechenden äquidistanten Schritten an, wobei der oberste fünfte Bereich bei 255 endet.

Auf Basis des Helligkeitsgrades in Form des 85%-Quartils kann dann eine Abschätzung eines maximalen Verdünnungsgrades der Patientenprobe durchgeführt werden, bei welchem eine Inkubation des Organschnittes mit der Patientenprobe noch zu einer Präsenz eines bzw. des Fluoreszenzmustertyps führt. Die zu bestimmende Information HI als das 85 % Quartil wird dann in einem Schritt S11 entsprechend einem der Teilbereiche bzw. der fünf Teilbereiche zugeordnet. Der ermittelte Teilbereich bzw. der Index des ermittelten Teilbereiches bestimmt eine Schrittgröße, ausgehend von der vorliegenden Verdünnung der Patientenprobe für die Erzeugung des Fluoreszenzbildes, um einen Verdünnungsgrad festzulegen, bei welchem die Patientenprobegrade noch zu einem positiven Muster führen würde bzw. zu einer Präsenz des Fluoreszenzmustertyps. Es wird also als Nebeninformation der Verdünnungsgrad VD der Probe aus der Inkubation bereitgestellt. Bei einer Verdünnung bzw. einem Verdünnungsgrad VD von 1 : 10 kann dann bei einer 10er-Verdünnungsreihe der Abstufung

10, 32, 100, 320, 1.000, 3.200, 10.000, 32.000

ausgehend von dem Wert 10 aufgrund einer ermittelten Schrittgröße, beispielsweise 2, und zwei Schritte weiter gegangen werden und dann eine Verdünnung von 100 ermittelt werden als ein Verdünnungsgrad, bei welchem eine Inkubation des Organschnittes mit der Patientenprobe gerade noch zu einer Präsenz des Fluoreszenzmustertyps führen würde. Dies ist dann der ermittelte Verdünnungsgrad VG. Dieser kann gemeinsam mit den weiteren Informationen beispielsweise in einem Schritt S8, siehe Figur 1 oder Figur 2, ausgegeben werden.

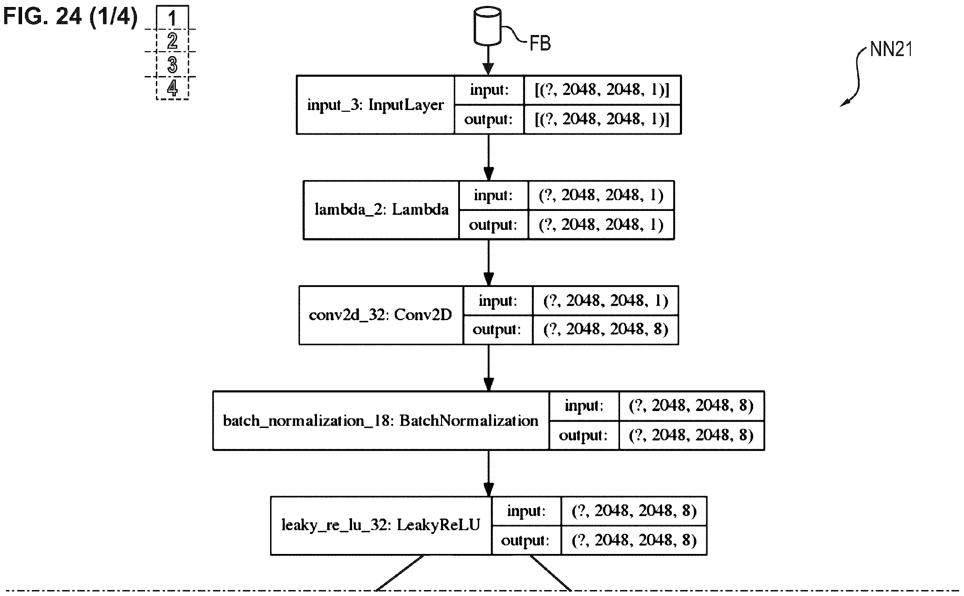

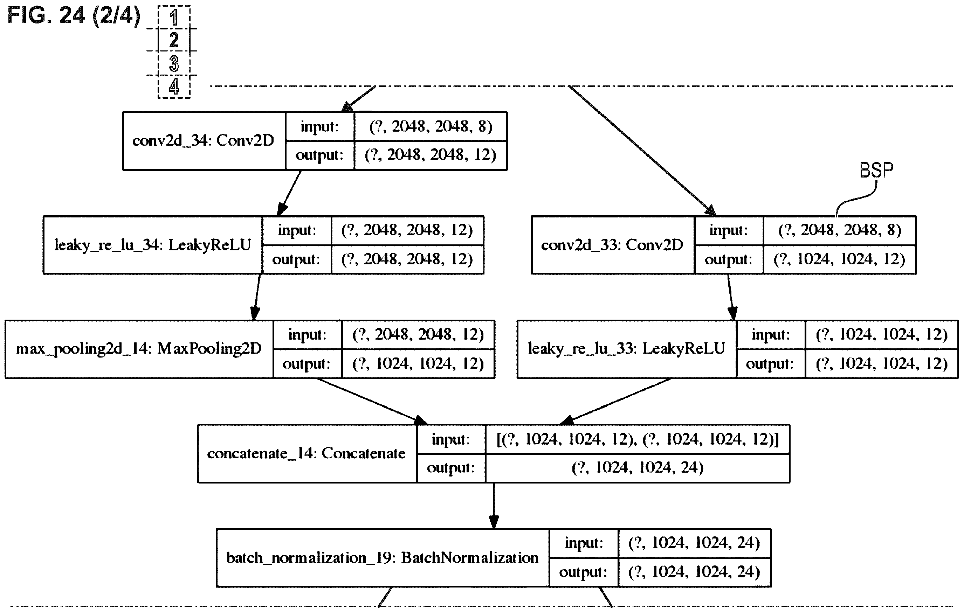

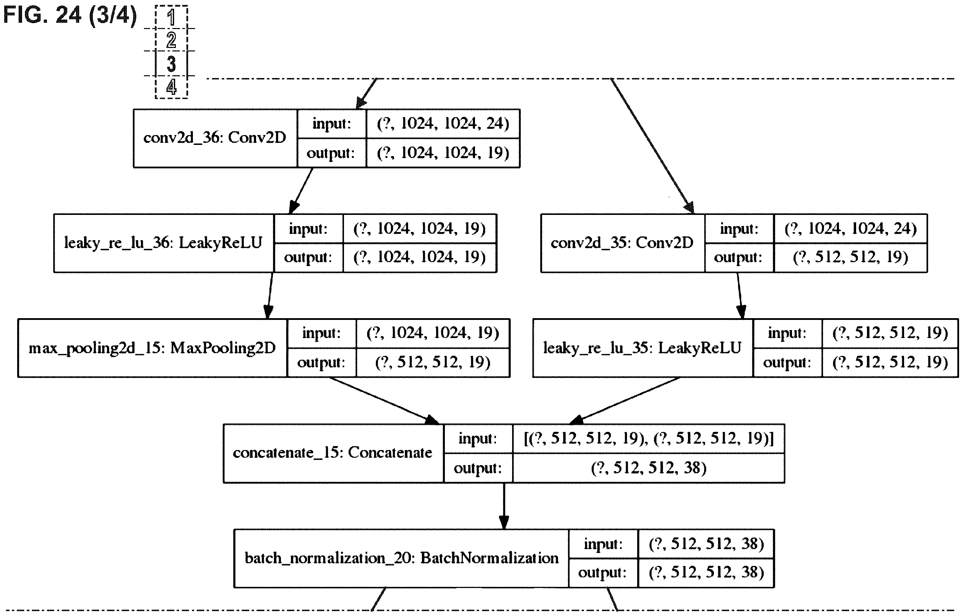

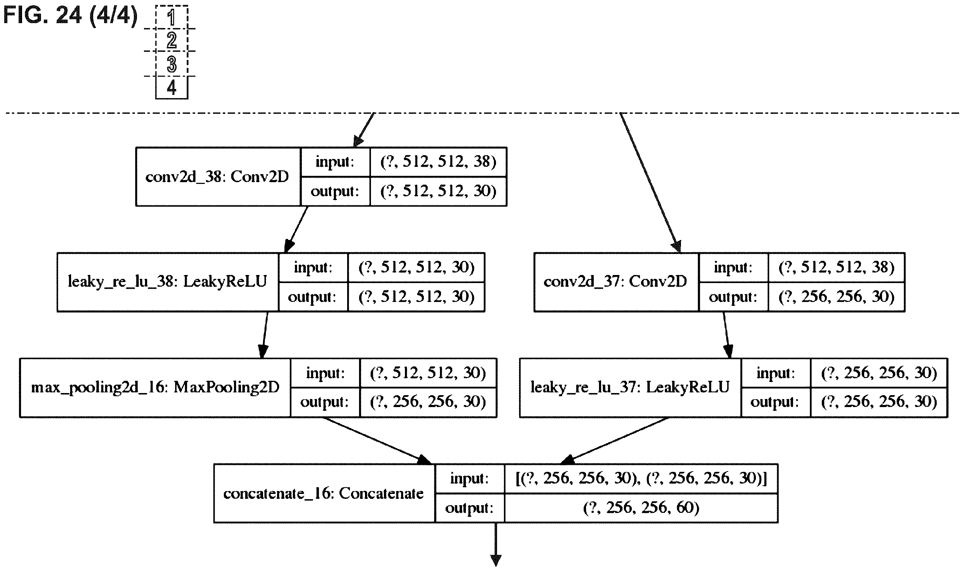

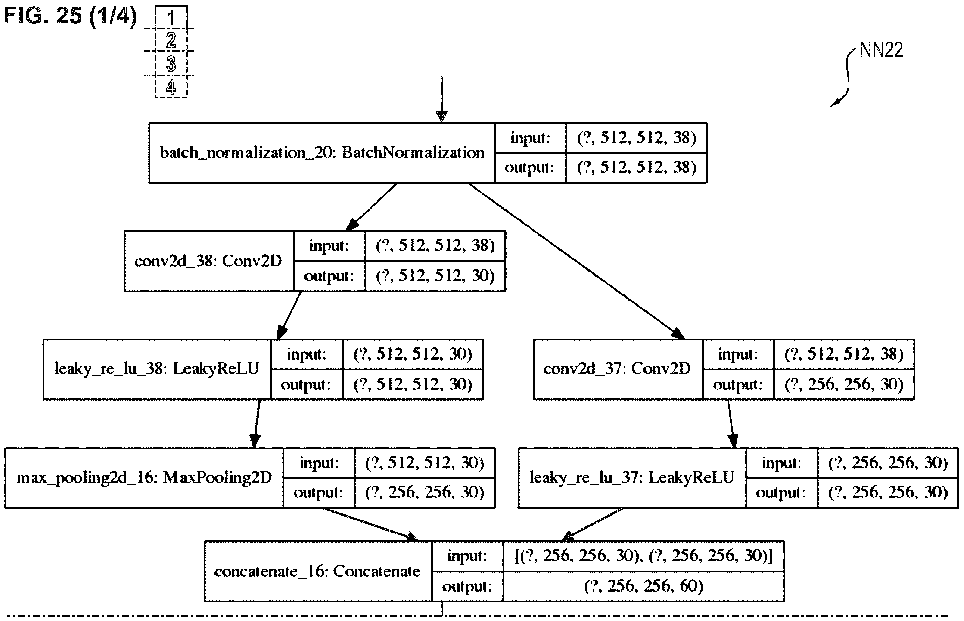

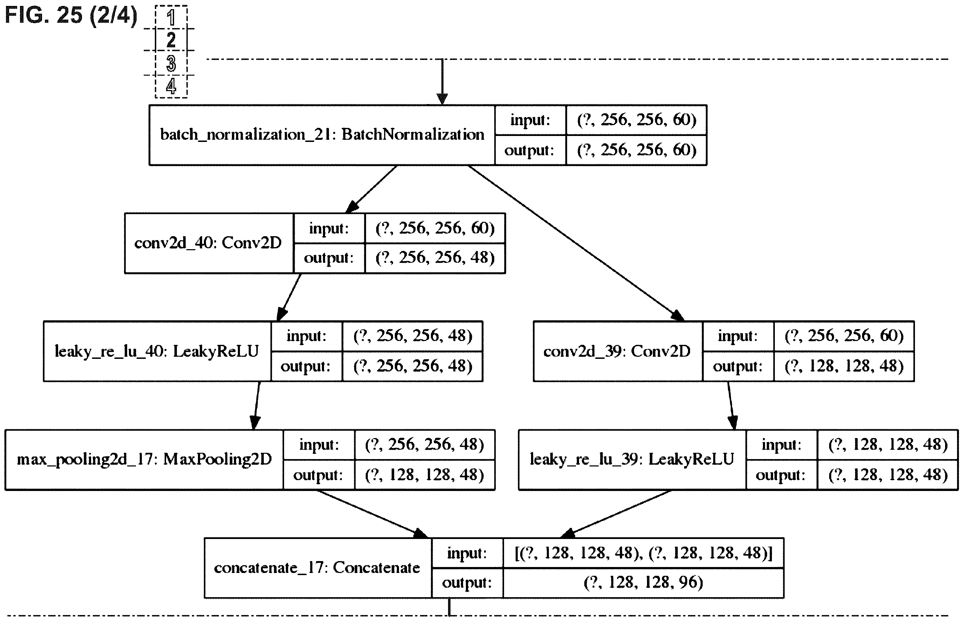

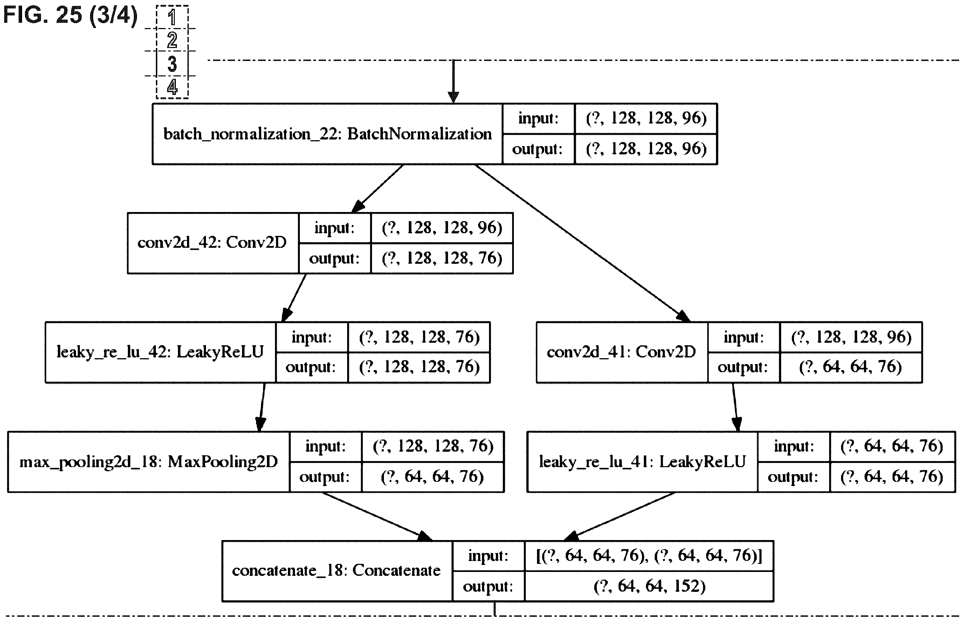

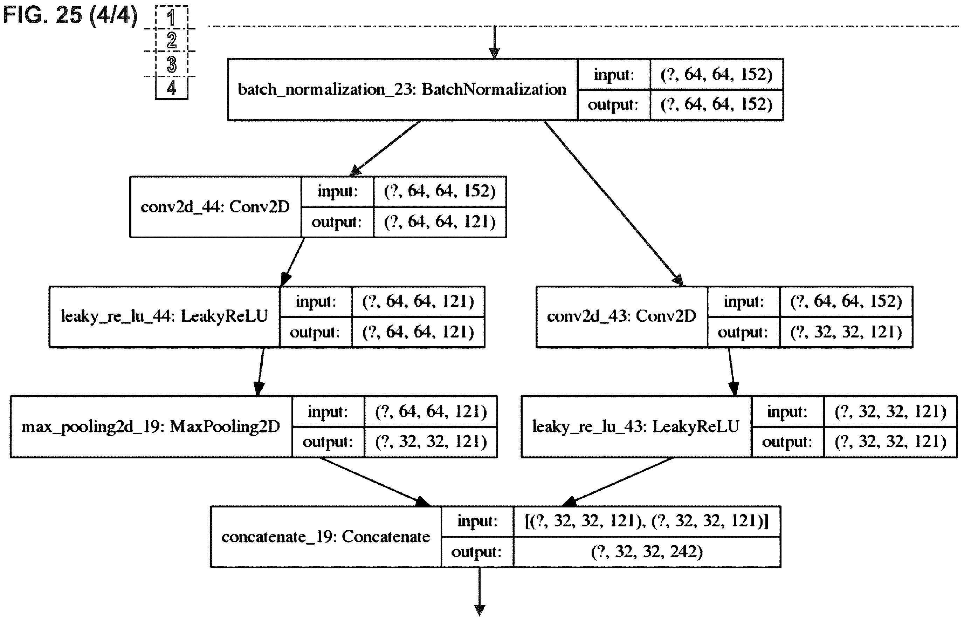

Die Figuren 24, 25 und 26 zeigen Teilelemente NN21, NN22, NN23, welche gemeinsam betrachtet in entsprechender Abfolge das zweite neuronale Netz NN2 aus Figur 1 bilden für das Beispiel des Organschnittes als eine Rattenniere. Das Fluoreszenzbild FB wird durch den ersten Teil NN21 aus Figur 24 entgegengenommen, wobei das Fluoreszenzbild FB vorzugsweise vorher auf eine Dimensionalität von 2048×2048 skaliert wurde.

Das gesamte Netzwerk NN2 wird durch eine Abfolge einer Mehrzahl von Schritten beziehungsweise Prozessierungsoperationen der Teile NN21, NN22, NN23 gebildet, wobei unterschiedliche Arten von Schritten vorkommen. Für einen jeden Prozessierungsschritt ist hierbei in dem linken Bereich des entsprechenden Rechteckes im Detail angegeben, von welcher Art der Schritt ist, sodass ein Fachmann die Prozessierung nacharbeiten kann. Ferner ist für jeden Prozessierungsschritt angegeben, von welcher Dimensionalität die jeweilige Eingangsgröße (Input) und die jeweilige Ausgangsgröße (Output) jeweils ist. Es ist also für jeden einzelnen Schritt im Detail angeben, wie die Prozessierung entsprechend durchzuführen ist. Hierbei kann für jeden einzelnen Schritt in der oberen Zeile "Input" in der darauffolgenden Klammer durch den zweiten und den dritten Eintrag die Dimensionalität der Eingangsgröße entnommen werden. Ferner kann durch den vierten Eintrag entnommen werden, wie viele Eingangsgrößen in dem betreffenden Schritt entgegengenommen werden. Beispielsweise werden in dem Schritt BSP 8 Größen der Dimensionalität 2048×2048 entgegengenommen. Hierbei wird in diesem Schritt BSP eine zweidimensionale Faltung der Art durchgeführt, dass es 12 Ausgangsgrößen gibt, welche jeweils eine Dimensionalität von 1024×1024 aufweisen. Dieses weist also darauf hin, dass im Zuge der zweidimensionalen Faltung 12 Faltungskernel verwendet werden und dass ferner mittels eines entsprechenden Stridings die Eingangsgrößen herunter skaliert werden um einen Faktor 2.

Es kann also ein Fachmann für jeden Prozessierungsschritt und den jeweils dort eingegebenen Parametern klar ableiten, wie dieser Prozessierungsschritt auszugestalten ist.

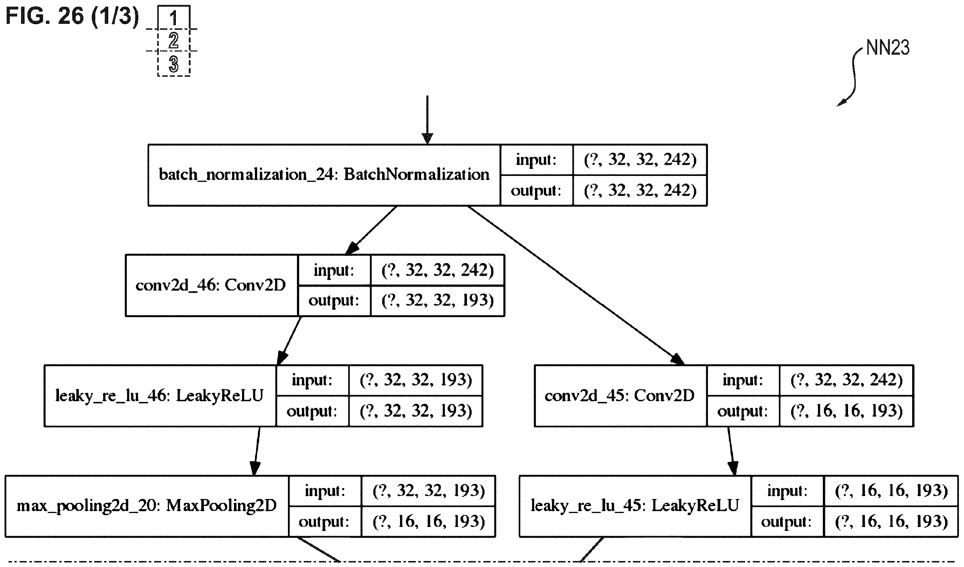

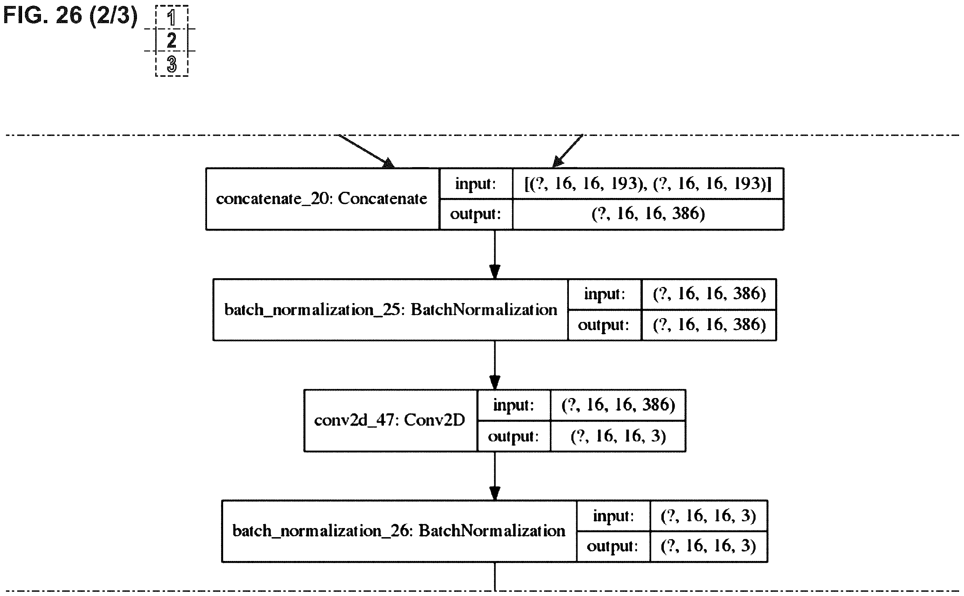

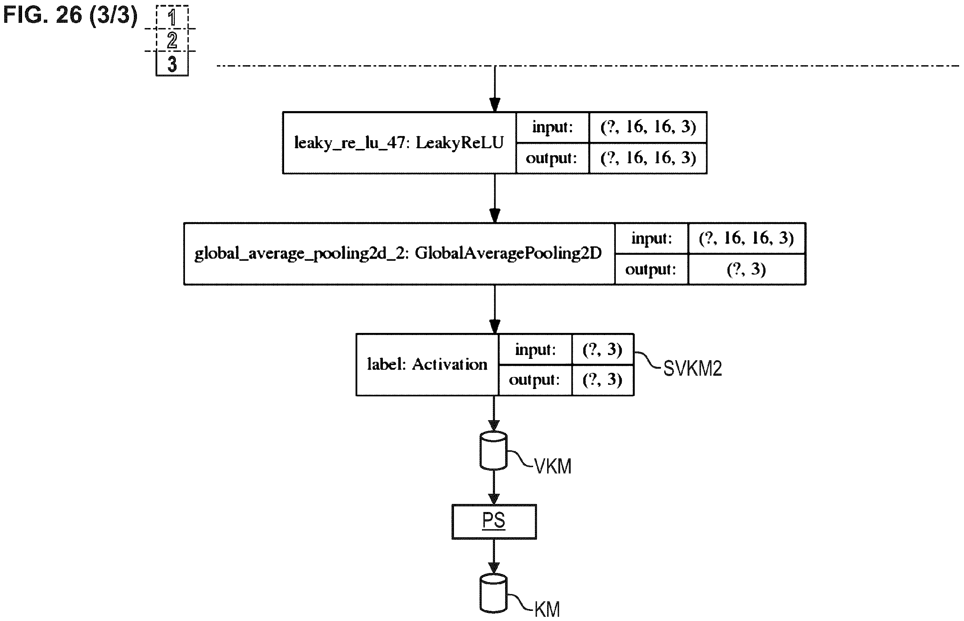

In dem dritten Teil NN23 aus Figur 26 des neuronalen Netzes NN2 wird dann in einem Schritt SVKM2 eine Ausgangsgröße VKM mit 3 Skalarwerten generiert, welche die vorläufigen Konfidenzmaße VKM_i für die 3 Klassen, Klassenindex i, mit i=1 für LKM-Muster, i=2 für AMA-Muster und i=3 Negativ repräsentiert, wie bereits zuvor beschrieben. In einem bereits zuvor beschriebenen Überprüfungsschritt PS werden dann die Konfidenzmaße KM ermittelt.

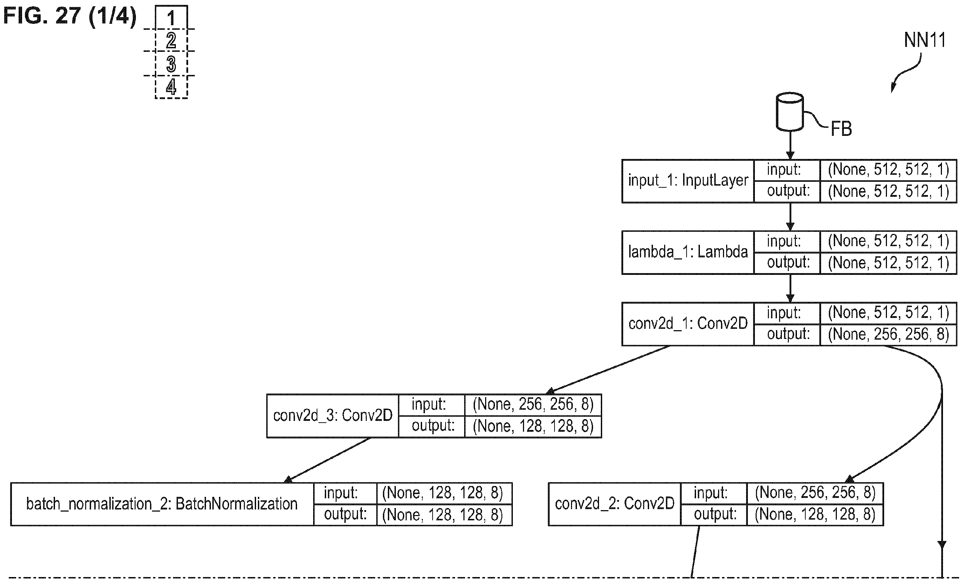

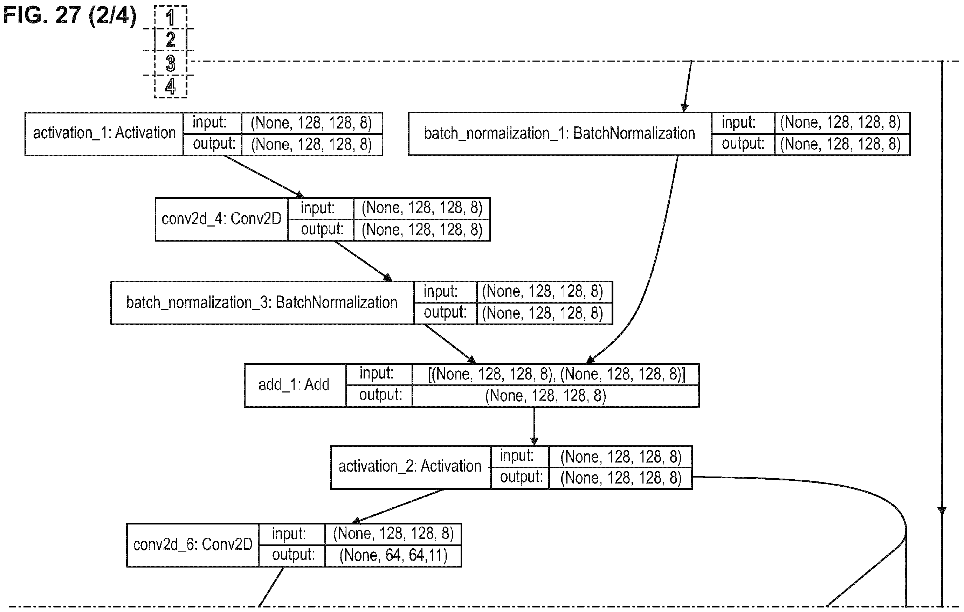

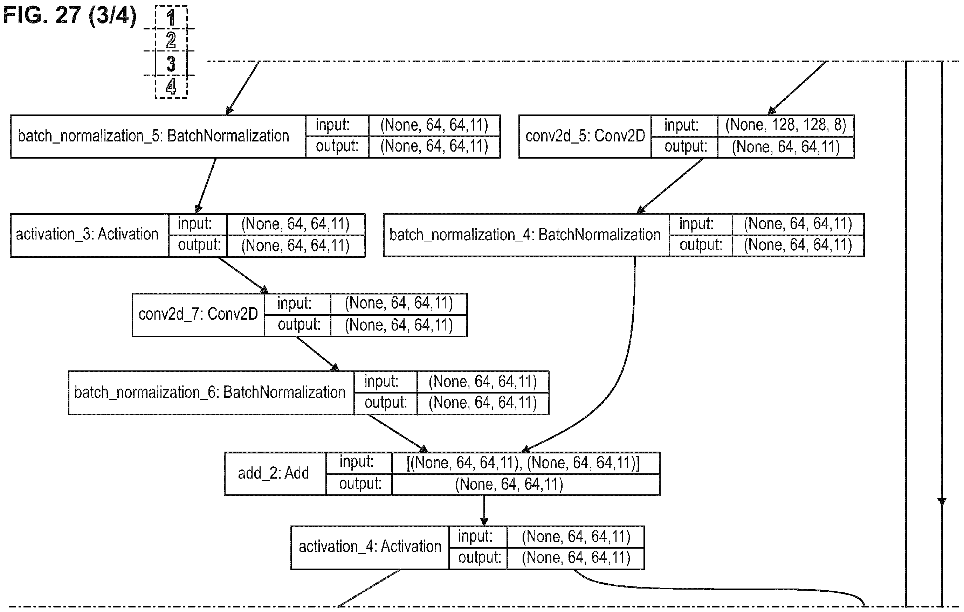

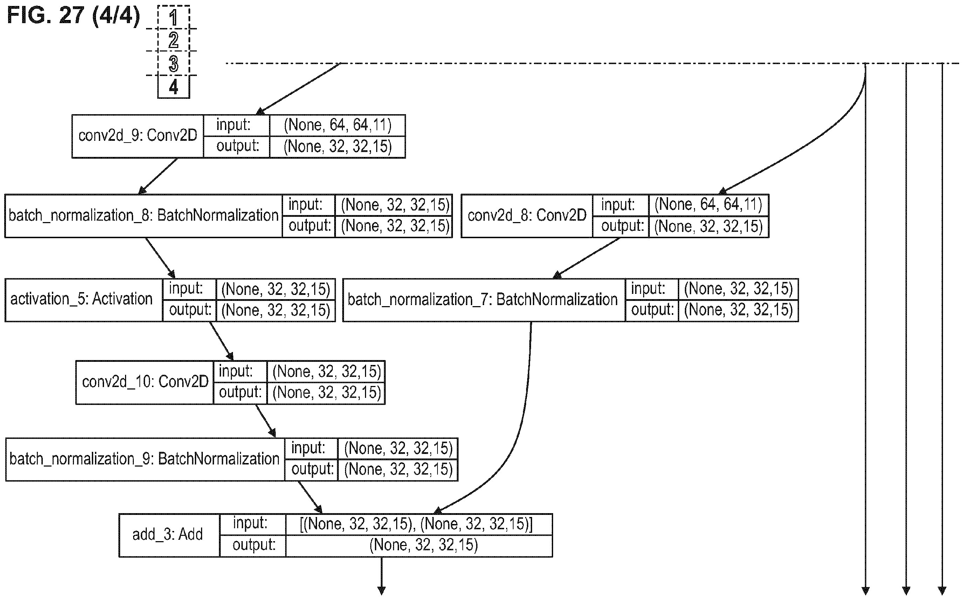

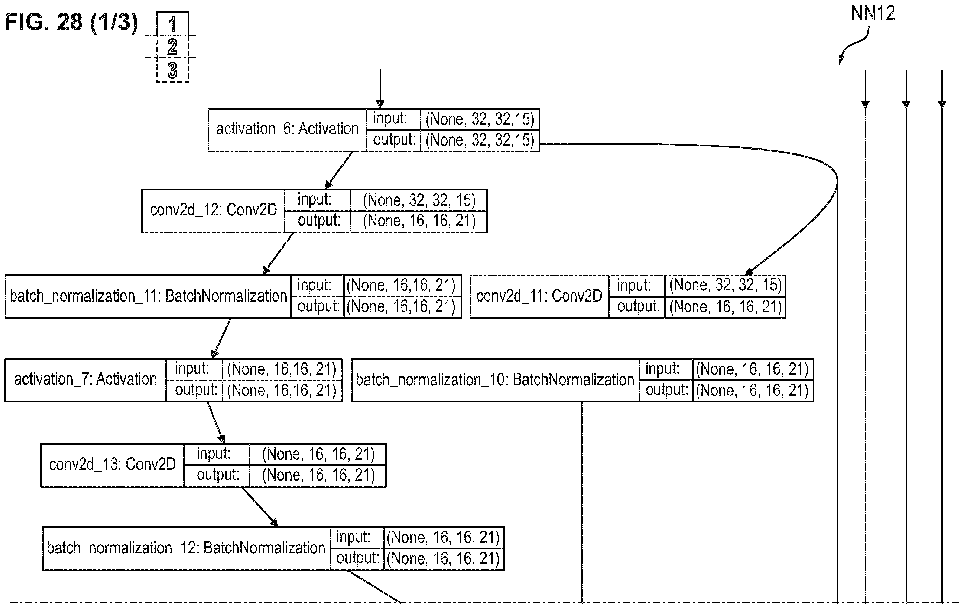

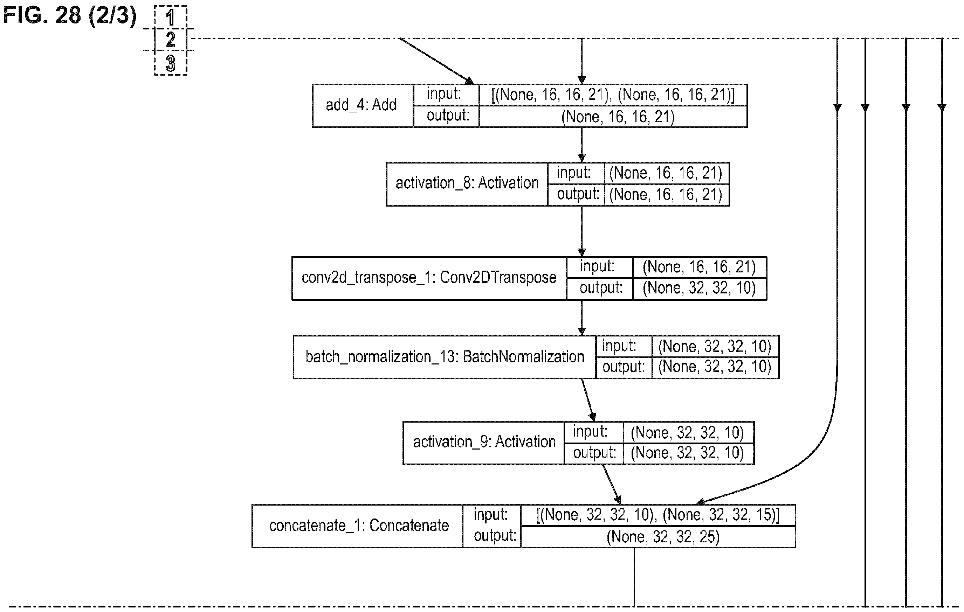

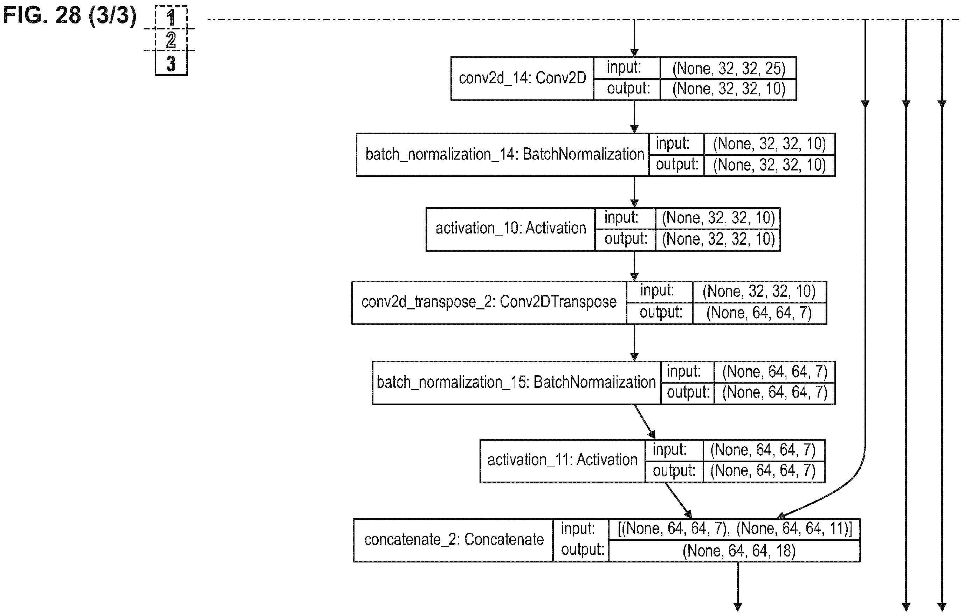

Die Figuren 27,28 und 29 zeigen entsprechende Teilnetze NN11, NN12, NN13, welche gemeinsam betrachtet das erste neuronale Netz NN1 aus der Figur 1 bilden für das Beispiel eines Organschnittes einer Rattenniere. In dem ersten Teil NN11 wird das Fluoreszenzbild FB entgegen genommen, wobei das Fluoreszenzbild FB vorzugsweise auf eine Dimensionalität von 512×512 herunter skaliert wurde. Auch für die hier dargestellten Teilnetze NN11, NN12 und NN13 sind jeweilige Prozessierungsschritte detailliert dargelegt.

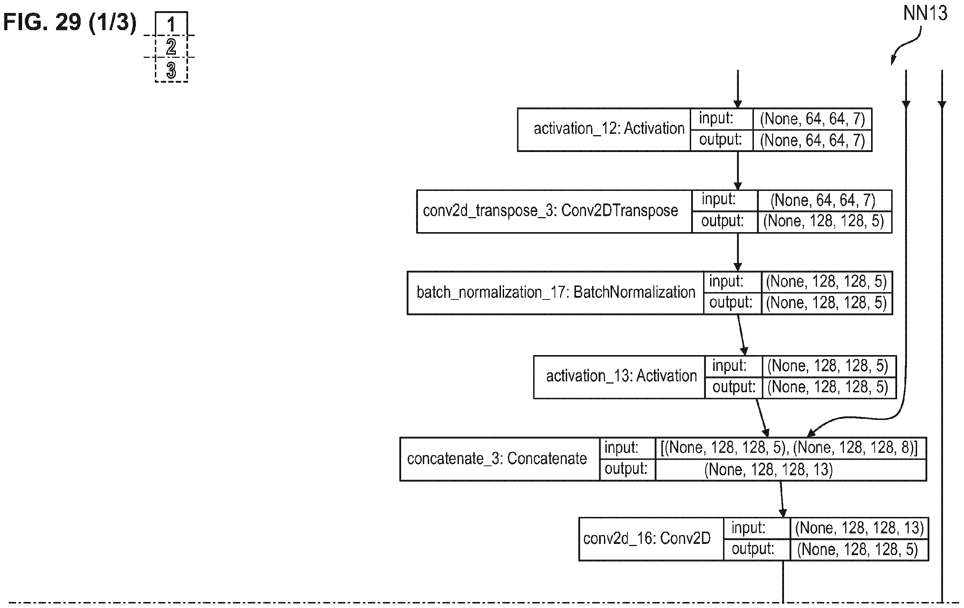

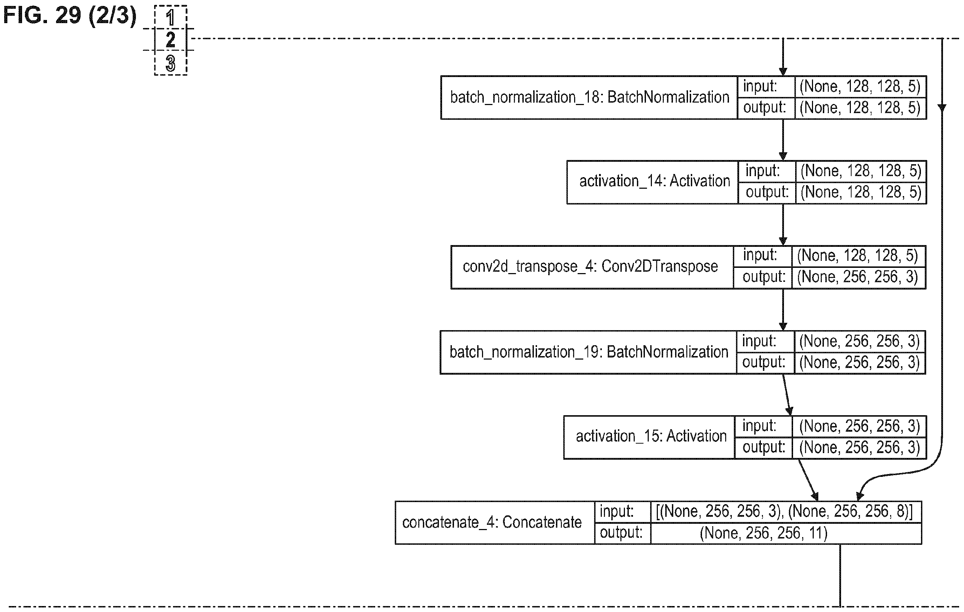

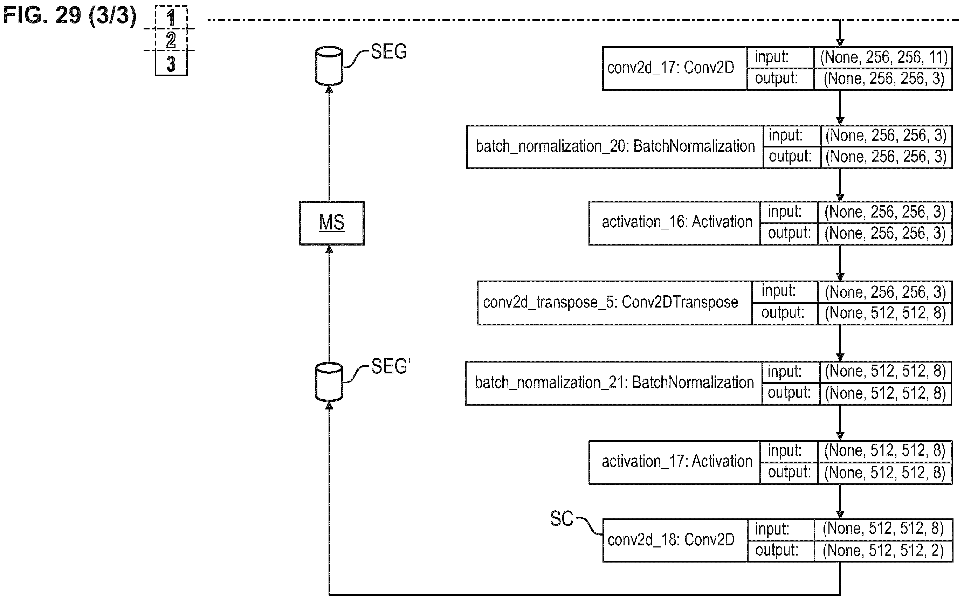

In dem dritten Teil NN13 aus Figur 29 wird dann in einem Schritt SC eine Segmentierungsinformation SEG' generiert, welche 2 Klassen aufweist. Hierbei stellt die Segmentierungsinformation SEG' der Dimensionalität 512×512×2 für jedes entsprechende Pixel des Fluoreszenzbildes FB der Dimensionalität 512×512 jeweils 2 Werte mit Index k=1,2 bereit, welche jeweils ein Konfidenzmaß mit Index k=1,2 angeben, dass das entsprechende Pixel zu der einen Klasse k=1 "Organ" bzw. der anderen Klasse k=2 "Hintergrund" gehört. Es kann dann durch eine Maximumentscheidung MS bezogen auf die zwei Konfidenzmaßwerte mit Index k=1,2 für ein bestimmtes Pixel eine Entscheidung getroffen werden, zu welcher Klasse das entsprechende Pixel gehört, um eine Segmentierungsinformation SEG zu generieren.

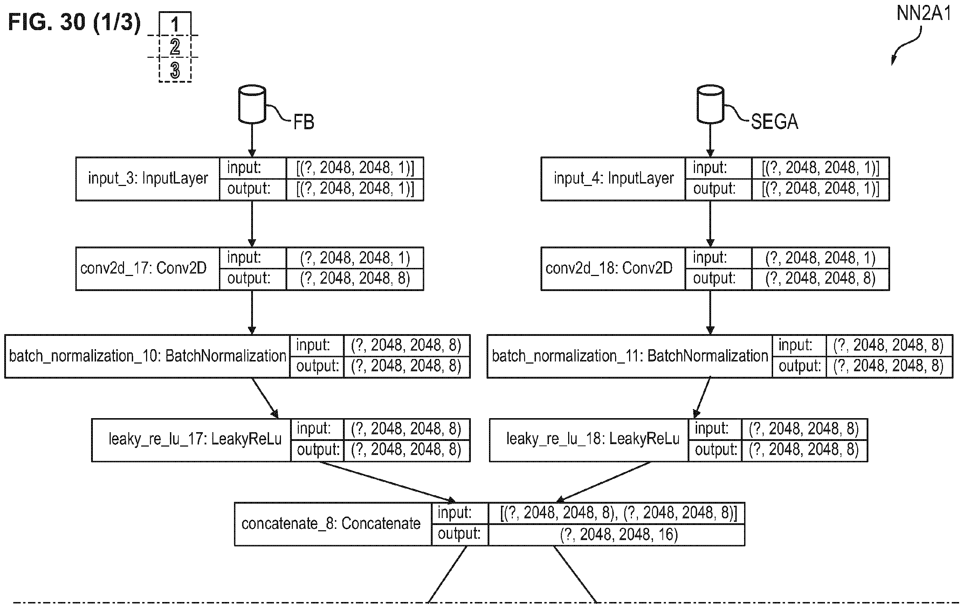

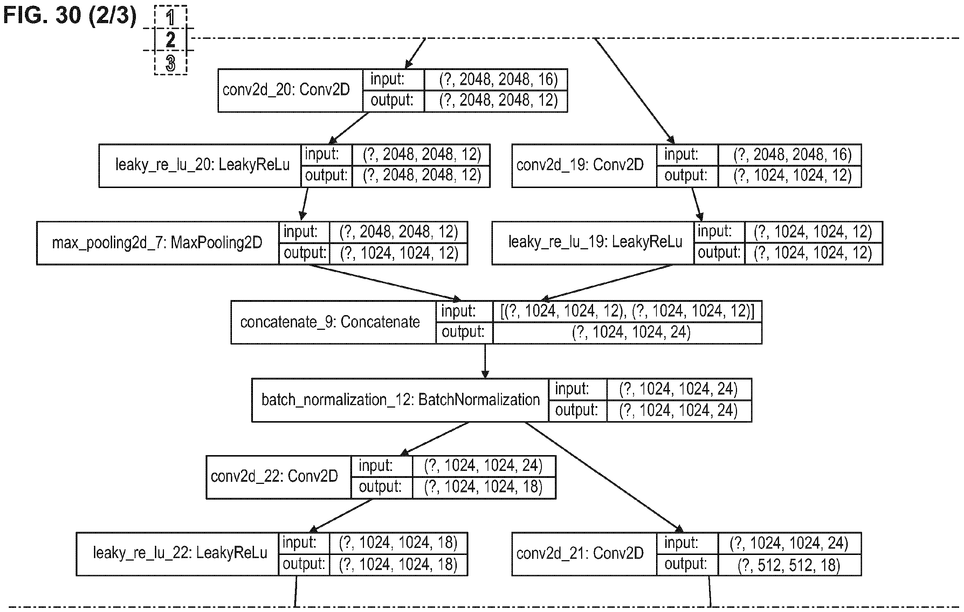

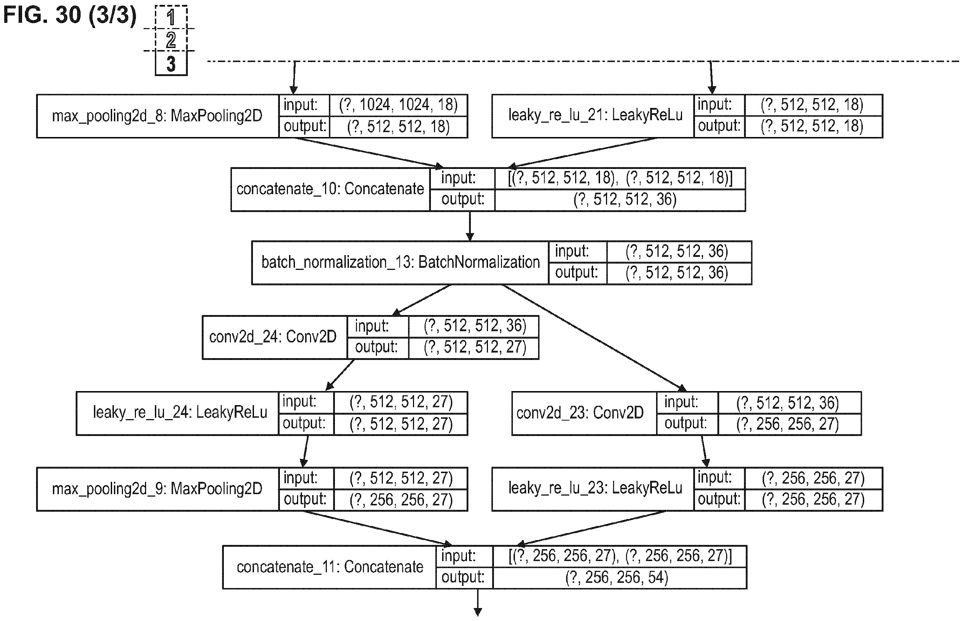

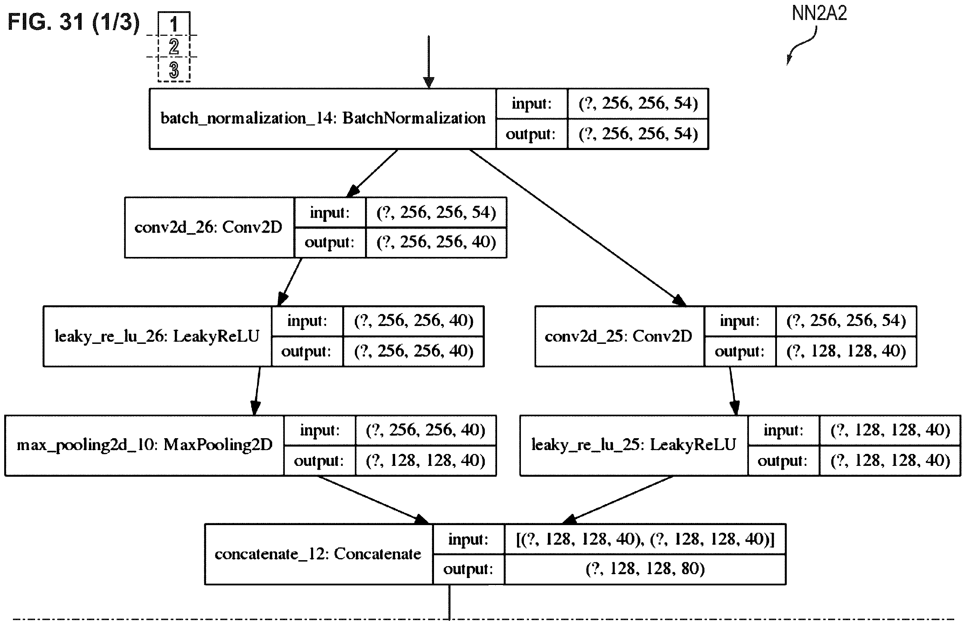

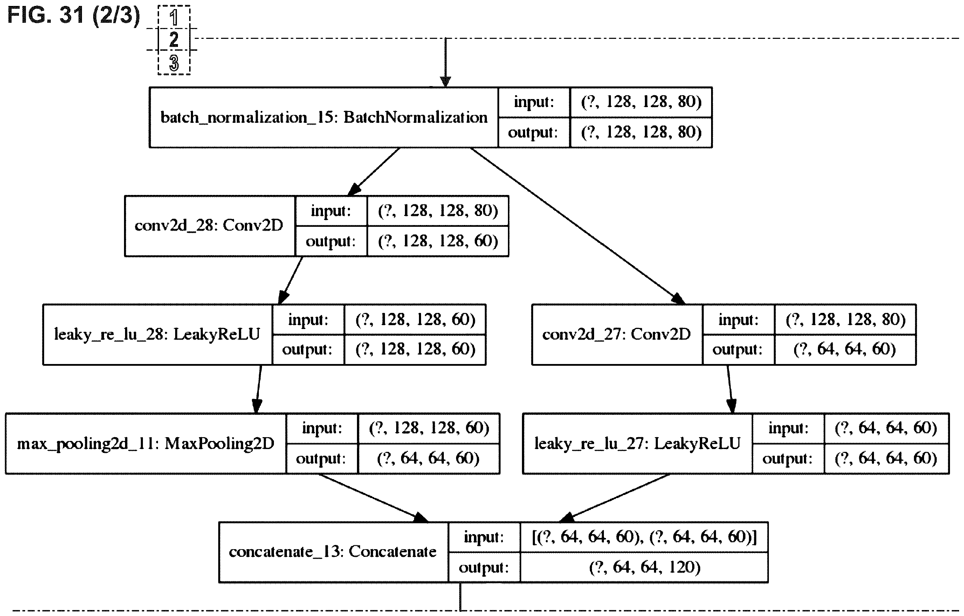

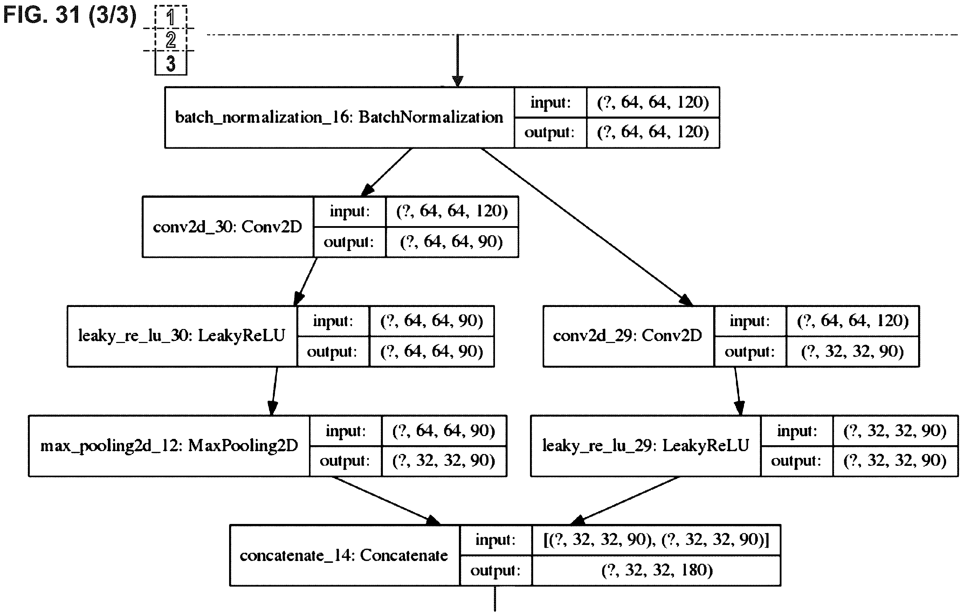

Die Teilnetze NN2A1, NN2A2, NN2A3 aus den Figuren 30, 31, 32 bilden gemeinsam eine Ausführungsform eines neuronalen Netzes NN2A, wie in der Figur 2 dargestellt. Auch hier sind wieder für die jeweiligen Prozessierungsschritte detaillierte Angaben vorhanden. Das Fluoreszenzbild FB wird durch den ersten Teil NN2A1 entgegengenommen, wobei das Fluoreszenzbild FB vorzugsweise vorher auf eine Dimensionalität von 2048×2048 skaliert wurde. Ferner wird die Segmentierungsinformation SEGA durch den ersten Teil NN2A1 entgegengenommen, wobei die Segmentierungsinformation SEGA vorzugsweise vorher auf eine Dimensionalität von 2048×2048 skaliert wurde.

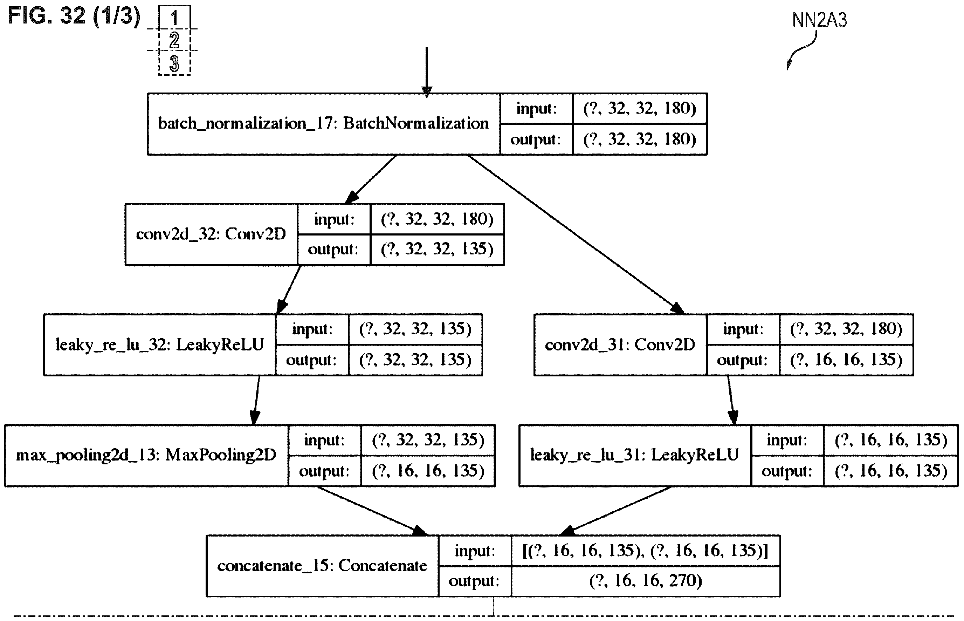

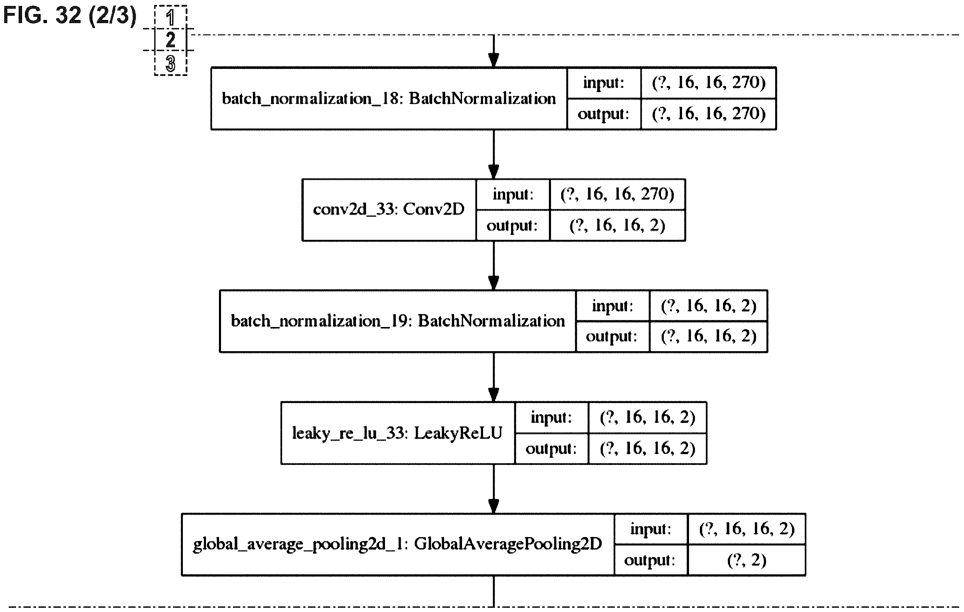

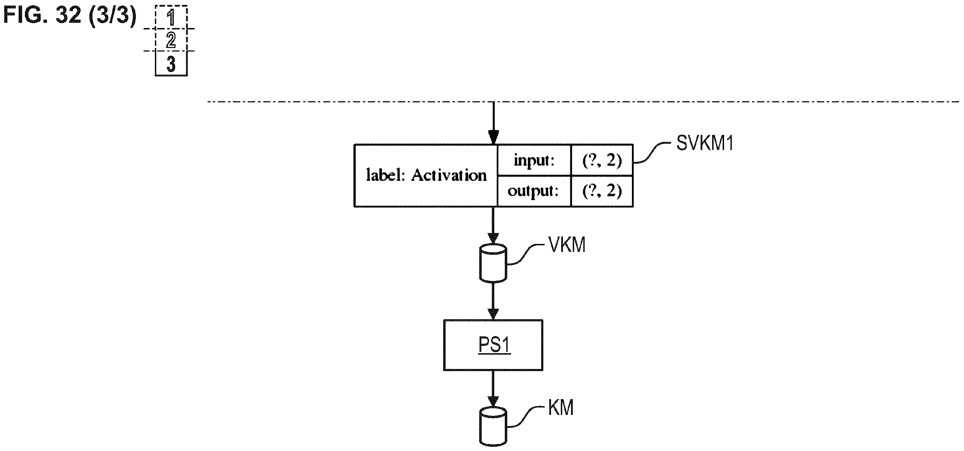

In dem dritten Teil NN2A3 aus Figur 32 wird dann in einem Schritt SVKM1 eine Ausgangsgröße VKM mit 2 Skalarwerten generiert, welche die vorläufigen Konfidenzmaße VKM_i für die 2 Klassen, Klassenindex i=1...2, mit i=1 für Endomysium-Muster und i=2 für Negativ repräsentiert. In einem bereits Überprüfungsschritt PS können dann mittels Schwellwertentscheidung Konfidenzmaße KM ermittelt werden.

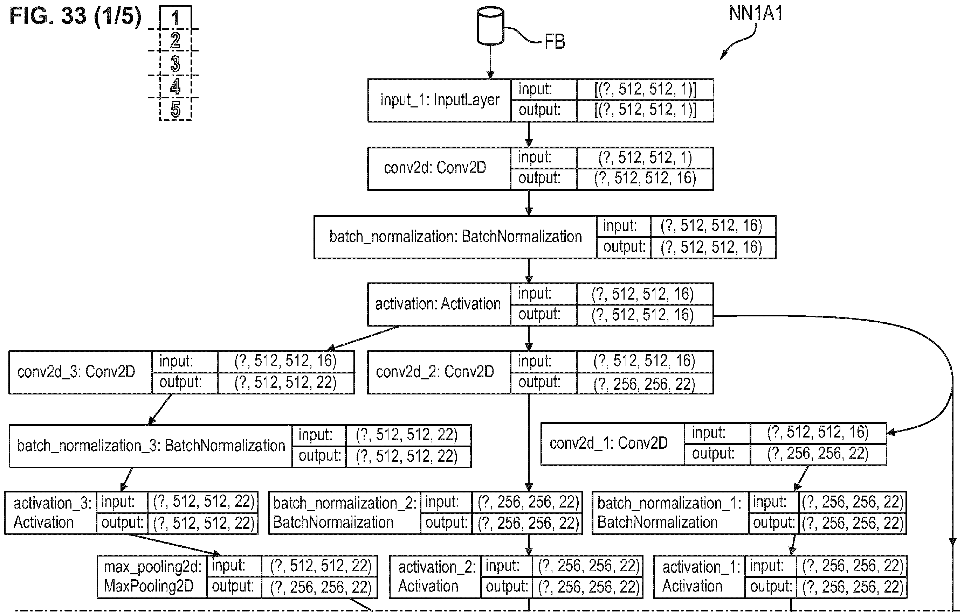

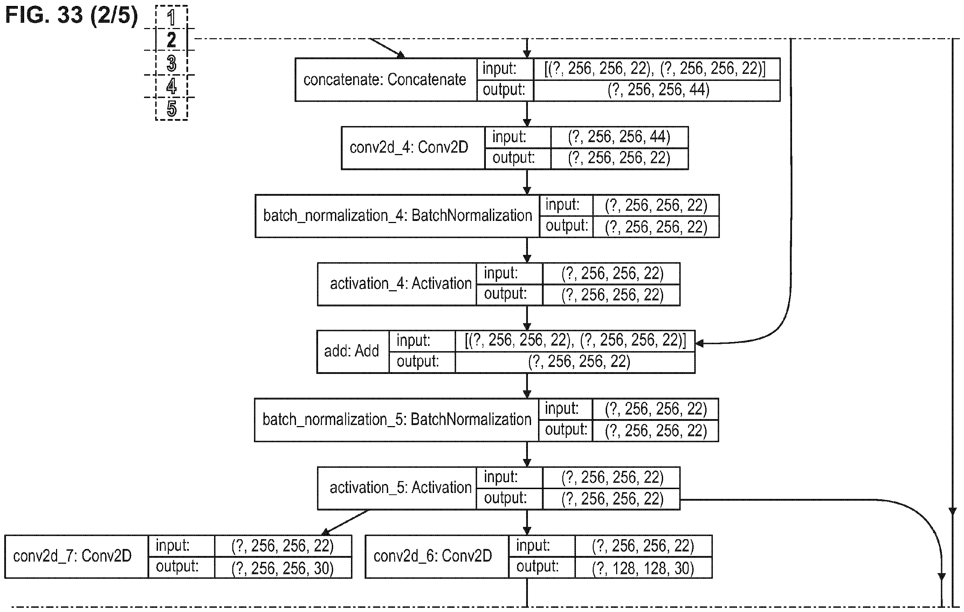

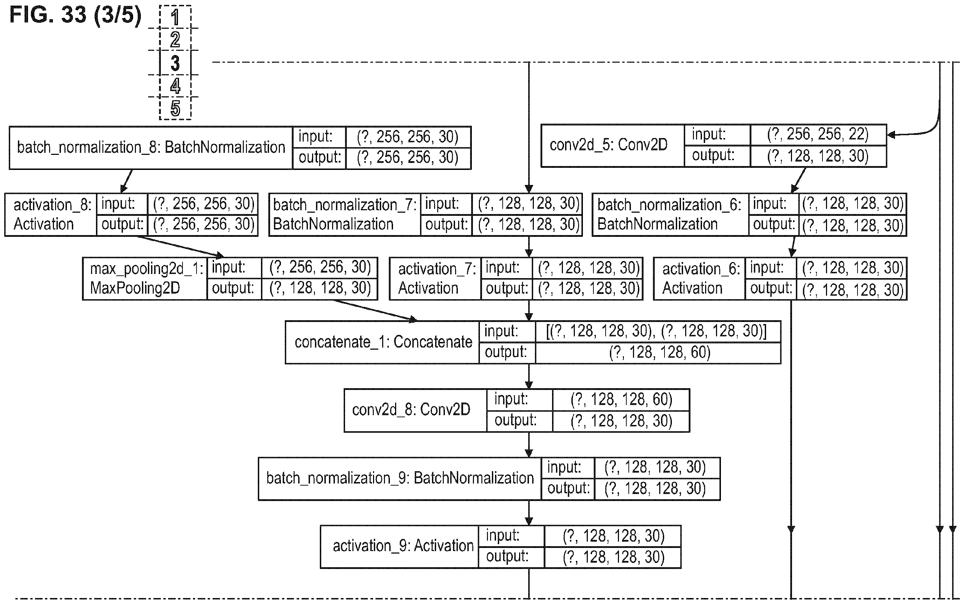

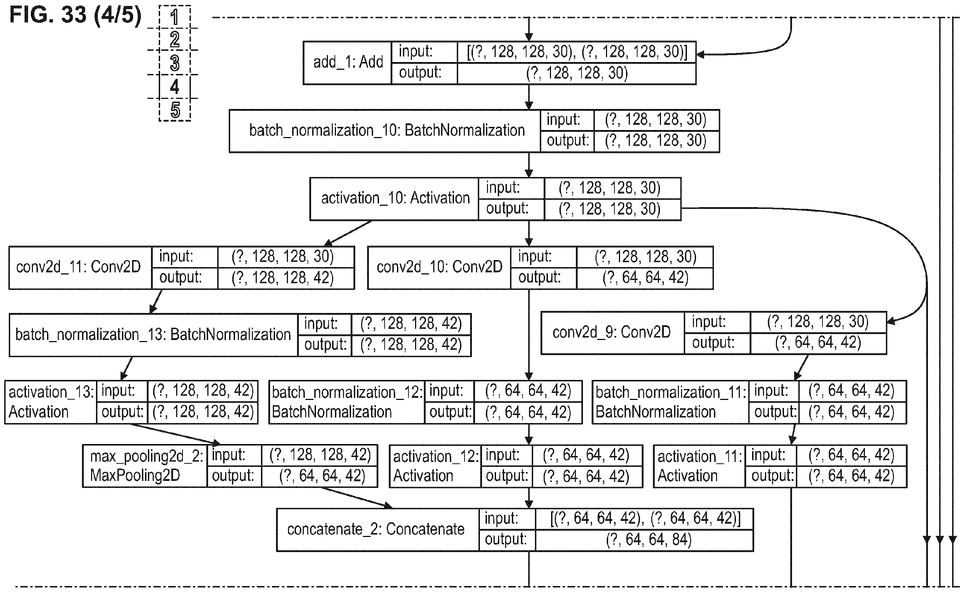

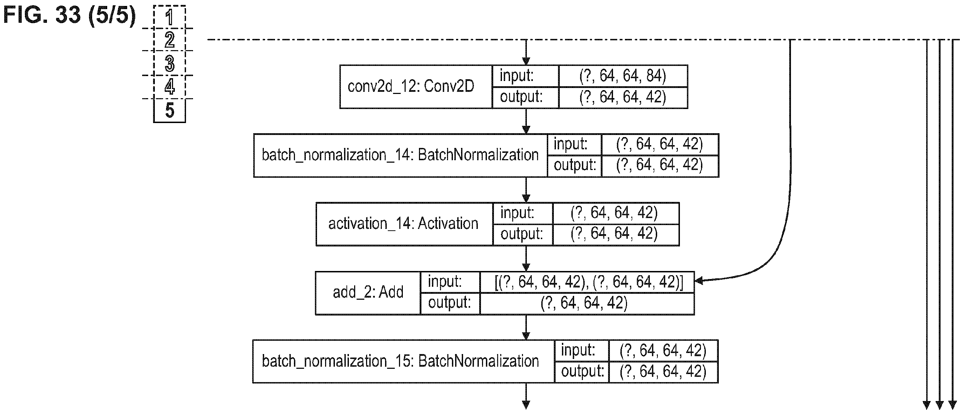

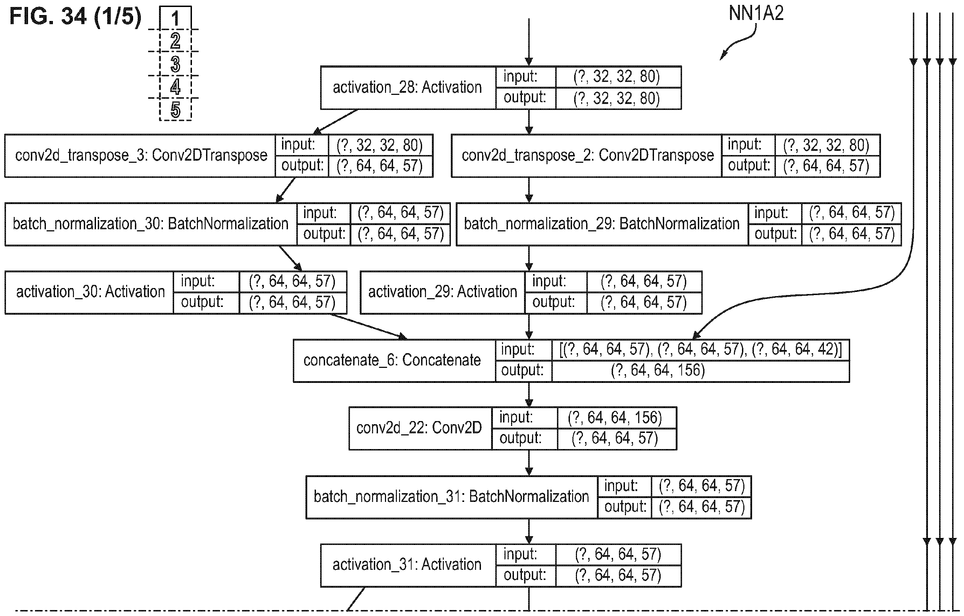

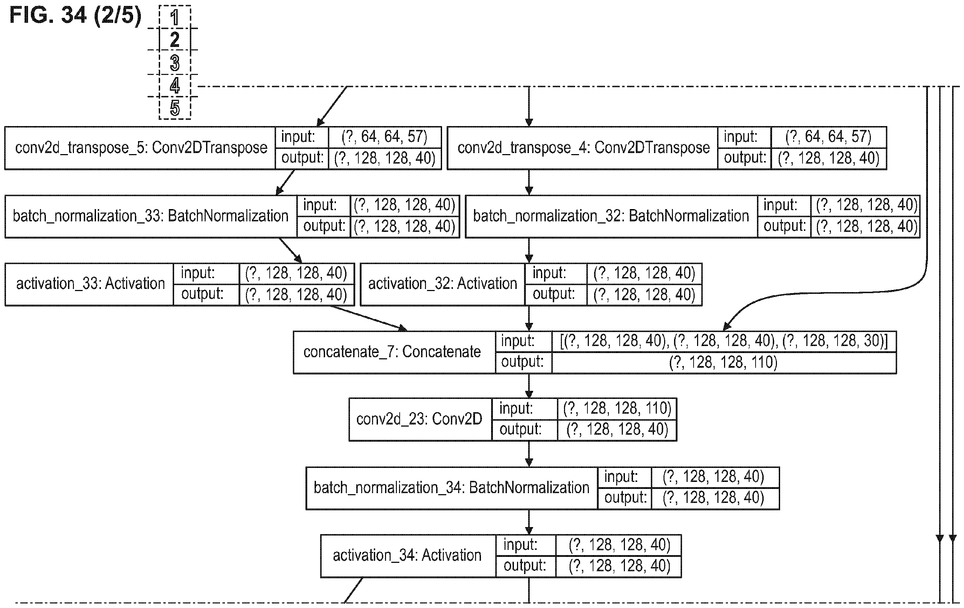

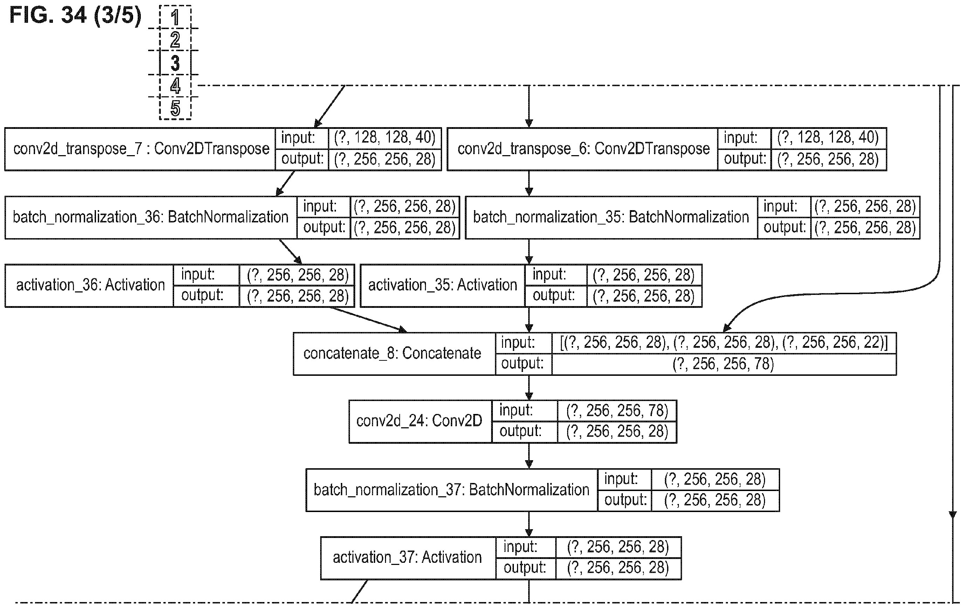

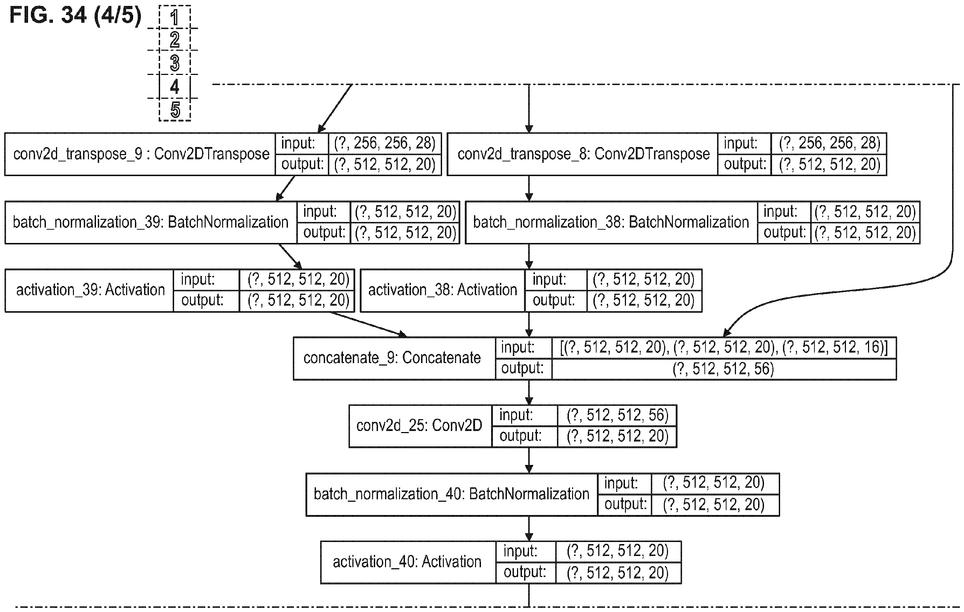

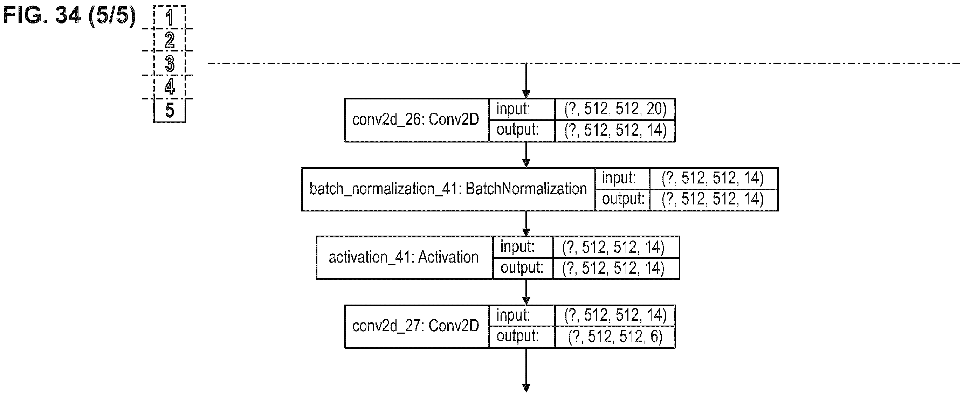

Die Figuren 33, 34, 35 zeigen Teile NN1A, NN1A2, NN1A3 eines neuronalen Netzes NN1A aus der Figur 2 für eine Segmentierung eines Fluoreszenzbildes für das Beispiel des Organschnittes als ein Ösophagus eines Affen. Auch hier sind wieder für jeden einzelnen Prozessierungsschritt detaillierte Angaben gegeben.

Das Fluoreszenzbild FB wird in der Figur 33 im ersten Schritt vorzugsweise in einer Dimensionalität von 512×512 entgegengenommen, wobei das Fluoreszenzbild FB vorzugsweise zuvor entsprechend skaliert wurde.

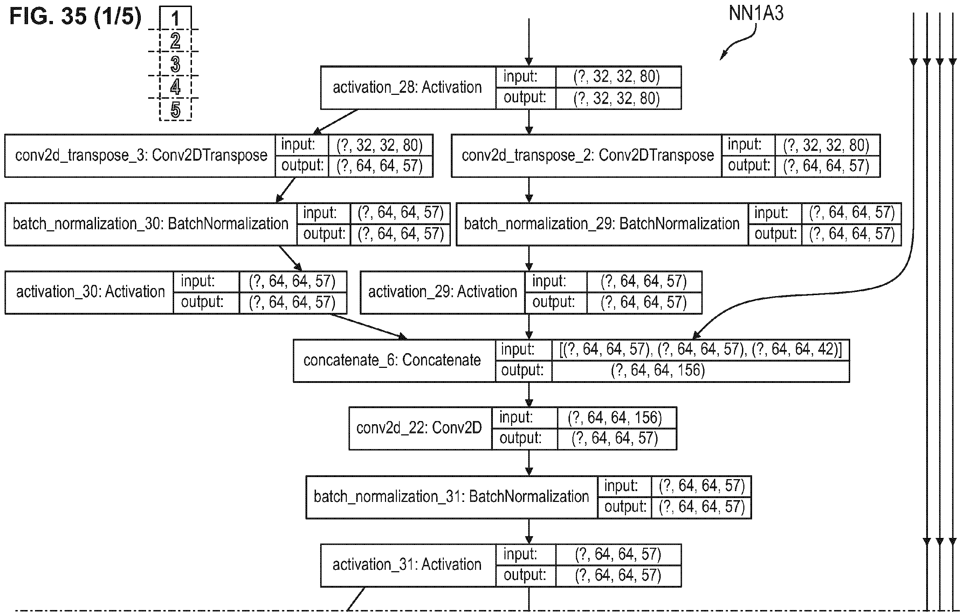

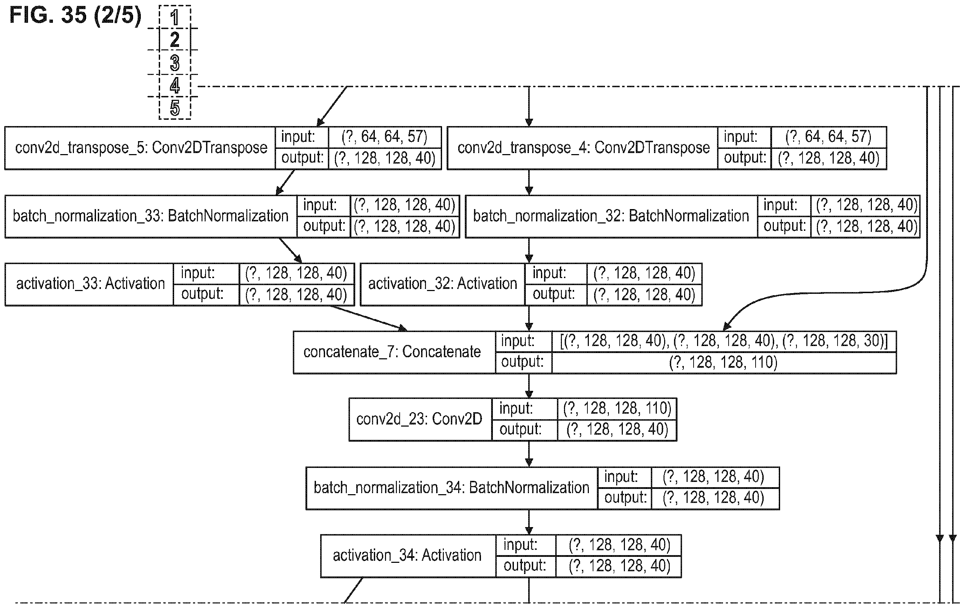

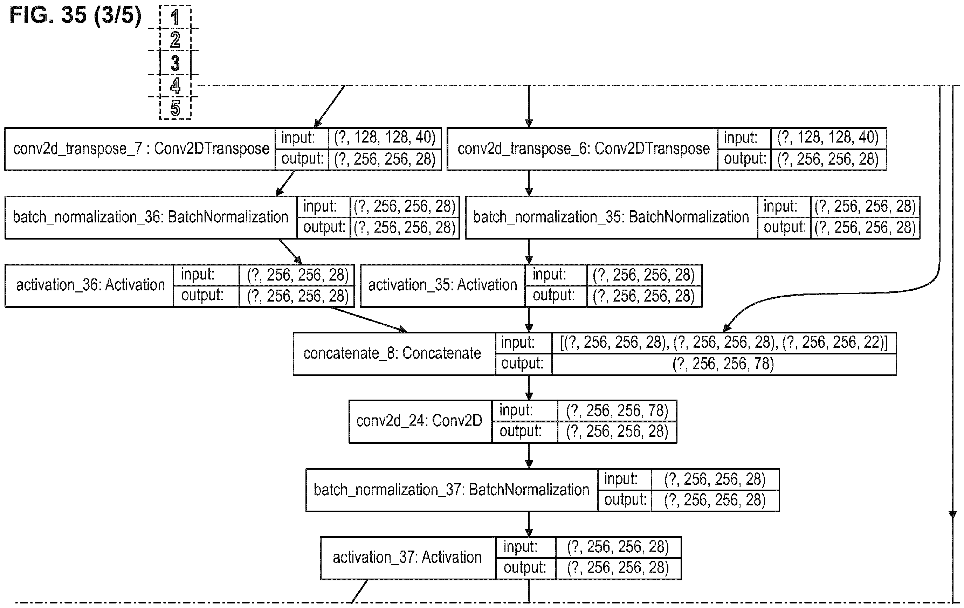

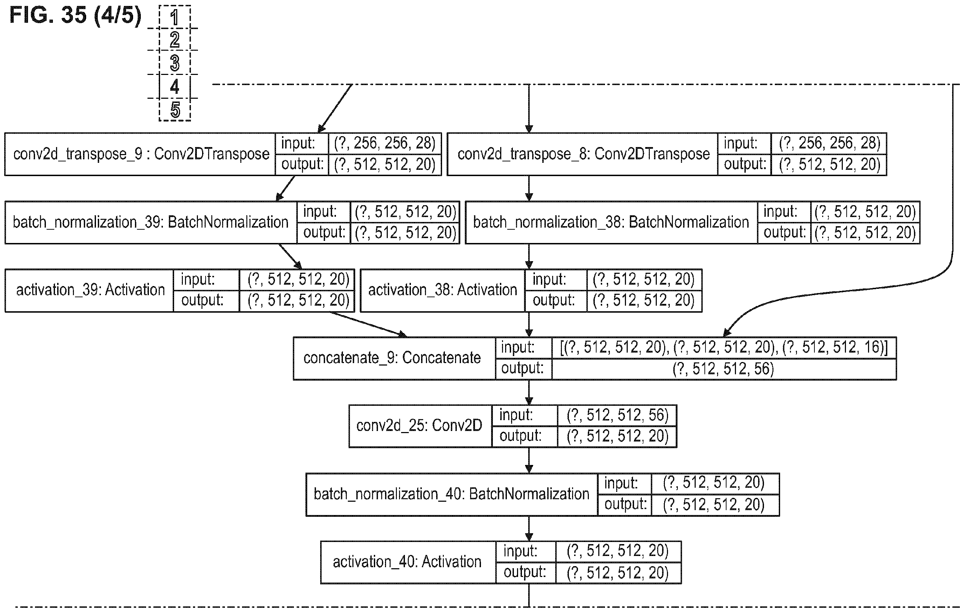

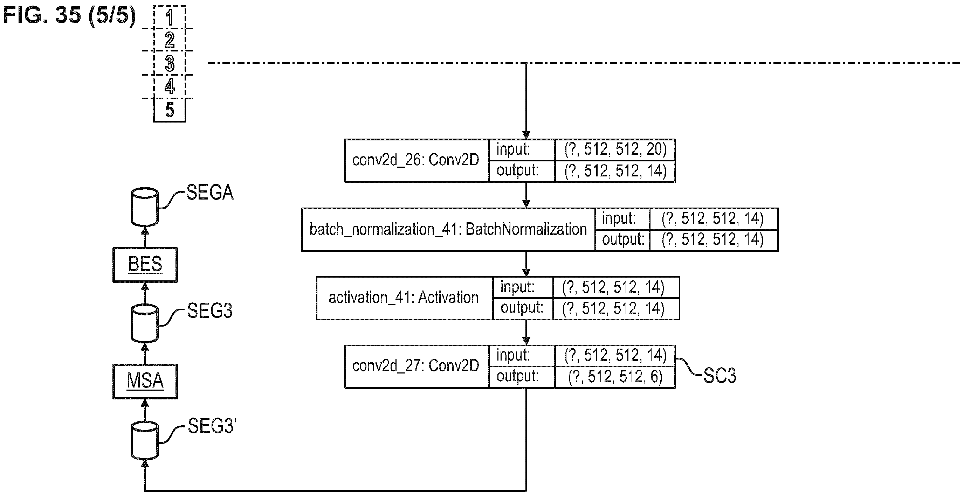

Am Ende des dritten Teils NN1A3, siehe Figur 35, des neuronalen Netzes NN1A aus Figur 2 wird dann in einem Schritt SC3 eine Segmentierungsinformation SEG3' ausgegeben, welche beispielhaft in der Figur 15 b dargestellt ist. Hierbei werden durch die Segmentierungsinformation SEG3' für die 5 Klassen beziehungsweise die 5 Teilflächen A, B, C, D, E sowie für eine weitere sechste Klasse als Hintergrundfläche HGF jeweilige Wahrscheinlichkeiten als Ausgabegrößen jeweils bezogen auf ein einzelnes von 512×512 Pixeln bereitgestellt, sodass dann für ein jedes Pixel aus einer Bildinformation der Dimension 512×512 durch eine Maximumentscheidung MSA bezogen auf die Konfidenzmaße eines bestimmten Pixels entschieden werden kann, zu welcher Klasse das Pixel gehört, um die relevante Teilfläche bzw. die Segmentierungsinformation SEG zu erhalten, welche in der Figur 15 b dargestellt ist. Hieraus lässt sich dann in einem Bestimmungsschritt BES mittels Indexauswahl die Segmentierungsinformation SEGA gewinnen, beispielhaft als SEGA in Figur 15d dargestellt.

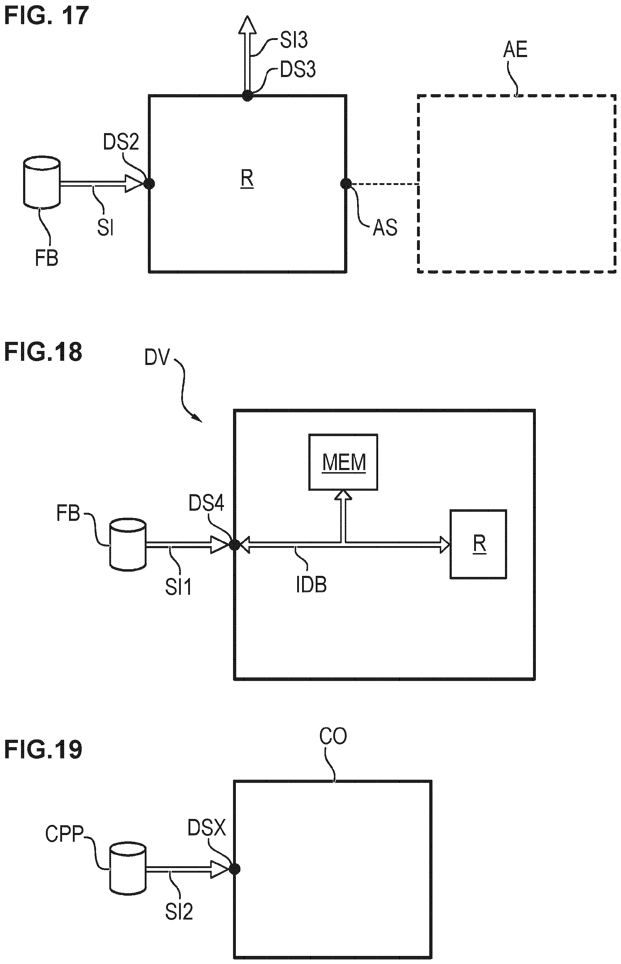

Figur 17 zeigt eine erfindungsgemäße Recheneinheit, welche vorzugsweise gemäß einer bevorzugten Ausführungsform ein Fluoreszenzbild SB als ein Datensignal SI über eine Datenschnittstelle DS2 entgegennimmt. Die Recheneinheit R kann dann die zuvor beschriebenen Informationen KM, VI ermitteln und über eine Datenschnittstelle DS3 als ein Datensignal SI3 bereitstellen. Vorzugsweise kann dies über ein leitungsgebundenes oder drahtloses Datennetzwerk erfolgen. Besonders bevorzugt weist die Recheneinheit R eine Ausgabeschnittstelle AS auf zur Ausgabe der Informationen KM, VI über eine Ausgabeeinheit AE. Die Ausgabeeinheit AE ist vorzugsweise eine Anzeigeeinheit für eine optische Anzeige der zuvor genannten Informationen.

Figur 18 zeigt eine erfindungsgemäße Datenexportvorrichtung DV gemäß einer bevorzugten Ausführungsform. Die Datennetzwerkvorrichtung DV nimmt das Fluoreszenzbild FB als ein Datensignal SI1 entweder über eine Datenschnittstelle DS4 entgegen. Die Datennetzwerkvorrichtung DV weist eine zuvor beschriebene Recheneinheit R auf sowie eine Speichereinheit MEM. Die Recheneinheit R, eine Speichereinheit MEM als auch die Datenschnittstelle DS4 werden vorzugsweise über einen internen Datenbus IDB miteinander verbunden.

Die Figur 19 zeigt eine Ausführungsform eines vorgeschlagenen Computerprogrammprodukts CPP. Das Computerprogrammprodukt CPP kann sein Datensignal SI2 über eine Datenschnittstelle DSX durch einen Computer CO entgegengenommen werden.

Obwohl manche Aspekte im Zusammenhang mit einer Vorrichtung beschrieben wurden, versteht es sich, dass diese Aspekte auch eine Beschreibung der entsprechenden Verfahren darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung die Recheneinheit R oder die Datennetzwerkvorrichtung in Hardware und/oder in Software umsetzen. Eine Umsetzung einer hier genannten Recheneinheit R kann hier als wenigstens eine Recheneinheit erfolgen oder aber durch mehrere Recheneinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Recheneinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird.

Allgemein können Ausführungsbeispiele oder Teile der Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren oder ein Teil eines Verfahrens durchzuführen, wenn das Programm auf einem Prozessor oder einer programmierbaren Hardwarekomponente abläuft.

**Ergebnisse**

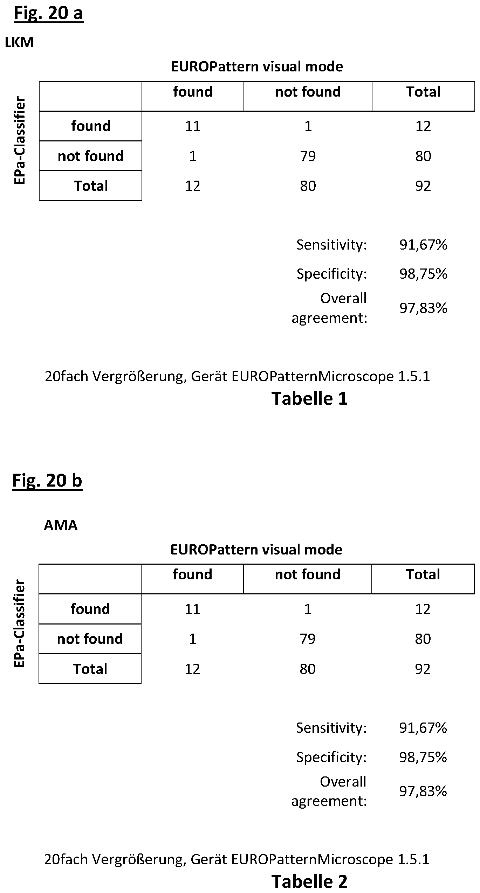

Die Figuren 20 bis 23 zeigen unterschiedliche Experimentalergebnisse für die verschiedenen Typen von Organschnitten, in diesem Fall einer Rattenniere und eines Ösophagus eines Affen.

Im Fall des Beispiels der Rattenniere wurden für das Segmentierungsnetzwerk, also das erste neuronale Netzwerk, 465 Fluoreszenzbilder im Zuge des Trainings verwendet. Hierbei wurden 75 % der 465 Bilder für eine sogenannte Backpropagation im Zuge des Trainings verwendet und 25 % der 465 Bilder als Validierungsbilder, deren Klassifikation durch das Netzwerk als Maß der Modellanpassung und der Generalisierung herangezogen wurden.

Im Fall des Beispiels der Rattenniere wurden für das Klassifikationsnetzwerk, also das zweite neuronale Netzwerk, 6300 Bilder verwendet, wobei auch hier während des Trainings eine Aufteilung der 6300 Fluoreszenzbilder in 75 % als tatsächliche Trainingsdaten für eine Backpropagation zur Anpassung der Gewichte des neuronalen Netzes verwendet wurden und 25 % der 6300 Fluoreszenzbilder für eine Validierung, also für eine Bestimmung eines Maßes der Modellanpassung Generalisierung des neuronalen Netzes.

Für den Fall des Beispiels eines Ösophagus eines Affen wurden in korrespondierender Weise 1700 Bilder für das Training des Klassifikationsnetzes, also des zweiten neuronalen Netzes, verwendet, wobei auch hier eine Aufteilung in 75 % der Bilder als Trainingsdaten für eine Backpropagation und 25 % der Bilder für eine Validierung aufgeteilt wurden.

Für den Fall des Beispiels eines Ösophagus eines Affen wurden 1200 Bilder für das Segmentierungsnetzwerk, also das erste neuronale Netz, in einer Trainingsphase verwendet, wobei auch hier eine Aufteilung von 75 % der Bilder in Trainingsdaten für eine Backprapogation und 25 % der Bilder als Validierungsbilder erfolgte.

Es wurden verschiedene positive und negative Proben verwendet. Jede Probe wurde in drei unterschiedlichen Verdünnungsstufen für unterschiedliche Inkubationen verwendet. Es wurde also für jede Probe ein Satz von drei jeweiligen Fluoreszenzbildern mit einer jeweiligen Verdünnungsstufe generiert. Wenn für eine bestimmte Probe wenigstens für das Fluoreszenzbild der geringsten Verdünnungsstufe (=höchste Probenkonzentration) durch das erfindungsgemäße Verfahren ("EPA-Classifier") ein bestimmtes Muster als präsent detektiert wurde, selbst wenn die anderen zwei Fluoreszenzbildern der stärkeren Verdünnungen (=geringere Probenkonzentrationen) als negativ bewertet wurden, dann wurde für die Probe das bestimmte Muster als generell präsent entschieden und die Probe als positiv bewertet. Wenn für eine bestimmte Probe für alle der drei Fluoreszenzbilder unterschiedlicher Verdünnungsstufen durch das erfindungsgemäße Verfahren ("EPA-Classifier") ein bestimmtes Muster als nicht präsent detektiert wurde, dann wurde das bestimmte Muster als generell nicht präsent detektiert und die Probe als generell negativ bewertet. Dieses Prinzip wurde bei allen Ergebnissen der Figuren 20 bis 23 angewendet.

Die Figur 20 a zeigt für eine Rattenniere Experimentalergebnisse, welche unter Verwendung einer 20-fachen Vergrößerung, also eine 20-fache optische Vergrößerung durch ein 20-fach-Objektiv, und unter Verwendung des Gerätes EUROPattern Microscope 1.5.1 erzielt wurden.

Es wurde eine Präsenz des LKM-Musters gemäß der Figur 20 a in 11 der 12 positiven Proben korrekt erkannt. In einem Fall wurde das LKM-Muster nicht gefunden. Bei 80 negativen Proben wurde korrekterweise das LKM-Muster bei 79 Bildern als nicht präsent entscheiden, bei einer Probe wurde fälschlicherweise das LKM-Muster detektiert. Dieses ergibt eine Sensitivität von 91,67 % und eine Spezifität von 98,75 %.

Die Figur 20 b zeigt eine Detektion des AMA-Musters im Falle einer Rattenniere ebenfalls bei 20-facher Vergrößerung und Verwendung des Gerätes EUROPattern Microscope 1.5.1. Die Ergebnisse stimmen von den Zahlenwerten her mit denen einer Detektion des LKM-Musters überein.

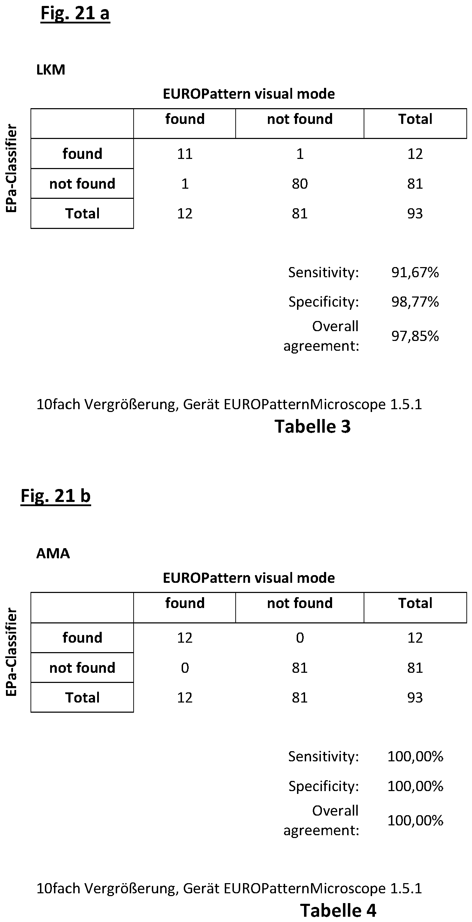

Figur 21 a zeigt Ergebnisse für eine Detektion des LKM-Musters bei einer Rattenniere unter Verwendung einer 10-fachen optischen Vergrößerung und des Gerätes EUROPattern Microscope 1.5.1. Hierbei wurden bei 12 als positiv zu befundenden Proben in 11 Fällen das LKM-Muster gefunden, jedoch in einem Fall das LKM-Muster nicht detektiert. Bei 81 als negativ zu bewertenden Proben wurde bei 80 Proben korrekt darauf entschieden, dass das LKM-Muster nicht präsent ist, während bei einer Probe fälschlicherweise das LKM-Muster als präsent detektiert wurde. Es ergeben sich dann Werte einer Sensitivität von 91,67 % sowie einer Spezifität von 98,77 %.

Die Figur 21 b zeigt Ergebnisse für eine Rattenniere ebenfalls bei 10-facher optischer Vergrößerung und Verwendung des Gerätes EUROPattern Microsope 1.5.1. Hier wurde bei 12 als positiv zu befundenden Proben in 12 Fällen korrekterweise das AMA-Muster detektiert. In 81 Fällen als negativ zu befundenden Proben wurde auch korrekterweise in 81 Proben entschieden, dass das AMA-Mustern nicht präsent ist. Die Sensitivität und die Spezifität sind in diesem Fall dann also 100 %.

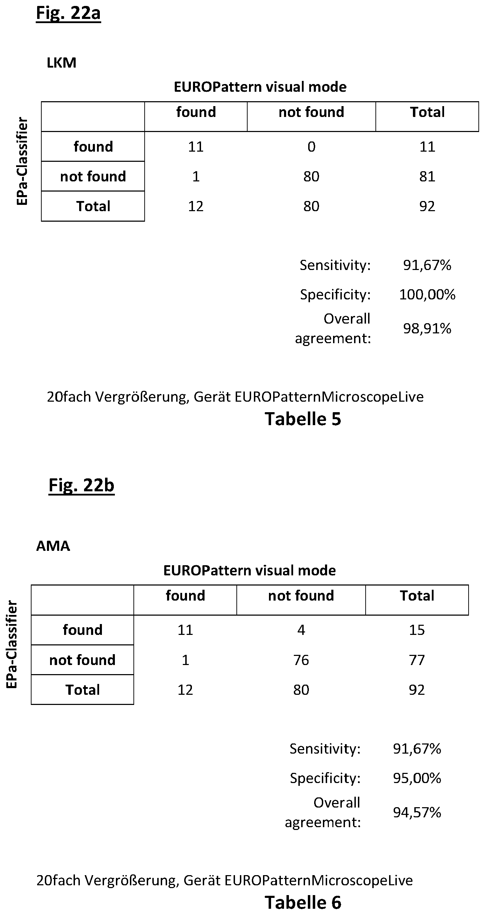

Die Figur 22 a zeigt Ergebnisse im Fall einer Rattenniere und eines LKM-Musters für eine 20-fach-Vergrößerung unter Verwendung des Gerätes EUROPattern Microscope Live. Hier wurde bei 12 als positiv zu detektierenden Proben bei 11 Proben korrekterweise das LKM-Muster als präsent detektiert, während bei einer Probe das LKM-Musters nicht detektiert wurde. In 80 Fällen als negativ zu entscheidenden Proben wurde bei allen 80 Proben das LKM-Muster als nicht präsent entschieden. Die Sensitivität sind hier 91,67 % und die Spezifität 100 %.

Die Figur 22 b zeigt Ergebnisse für eine Detektion des AMA-Mustern für eine Rattenniere bei einer 20-fachen optischen Vergrößerung und Verwendung des Gerätes EUROPattern Microscope Live. Bei 12 positiv zu entscheidenden Proben wurde bei 11 Proben korrekterweise die Präsenz des AMA-Mustern detektiert, während in einem Fall das AMA-Mustern fälschlicherweise nicht als präsent detektiert wurde. Bei 80 als negativ zu entscheidenden Proben wurde in 76 Fällen korrekterweise darauf entschieden, dass das AMA-Muster nicht präsent ist, während bei 4 Proben das AMA-Mustern als präsent entschieden wurde, was aber inkorrekt war. Die Sensitivität ergibt sich hierbei zu 91,67 % und die Spezifität zu 95 %.

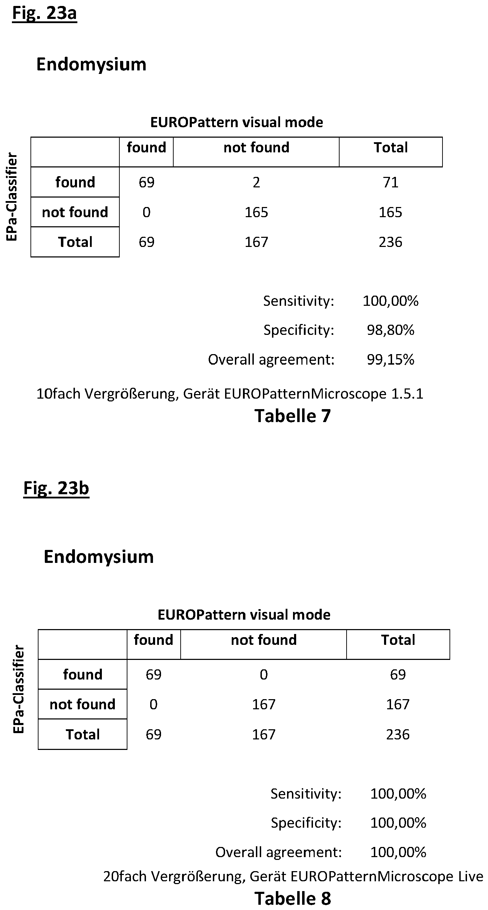

Figur 23 a zeigt für den Fall des Ösophagus eines Affen für eine Detektion des Musters Endomysium Ergebnisse unter Verwendung einer optischen 10-fach-Vergrößerung und unter Verwendung des Gerätes EUROPattern Microscope 1.5.1. Hierbei wurde bei 69 als positiv zu entscheidenden Proben bei allen 69 Proben das Muster Endomysium als präsent detektiert. Ferner wurde bei 167 als negativ zu klassifizierenden Proben in 165 Fällen darauf entschieden, dass das Muster Endomysium nicht präsent ist, was korrekt war. In 2 Fällen wurde darauf entschieden, dass das Muster Endomysium präsent sei, was jedoch inkorrekt war. Die Sensitivität ergibt sich hierbei zu 100 % und die Spezifität zu 98,8 %.

Figur 23 b zeigt Ergebnisse für den Ösophagus eines Affen für eine Detektion einer Präsenz des Endomysiums unter Verwendung einer optischen 20-fach-Vergrößerung und unter Verwendung des Gerätes EUROPattern Microscope Live. Hierbei wurde in 69 als positiv zu klassifizierenden Proben in allen 69 Fällen darauf entschieden, dass das Muster Endomysium präsent sei. In 167 als negativ zu befundenden Proben wurde in allen 167 Fällen darauf entschieden, dass das Muster Endomysium nicht präsent sei. Die Sensitivität und die Spezifität ergeben sich hiermit zu 100 %.

**Patentansprüche**

1. Verfahren zum Detektieren wenigstens einer potentiellen Präsenz wenigstens eines Fluoreszenzmustertyps auf einem Organschnitt mittels Immunfluoreszenzmikroskopie und mittels digitaler Bildverarbeitung, aufweisend

   - Bereitstellen des Organschnittes auf einem Objektträger,
   - Inkubieren des Organschnittes mit einer flüssigen Patientenprobe, welche potentiell primäre Antikörper aufweist,
   - Inkubieren des Organschnittes mit sekundären Antikörpern, welche mit einem Fluoreszenzfarbstoff markiert sind,
   - Erfassen eines Fluoreszenzbildes (FB, FB1, FB2, FB3) des Organschnittes in einem Farbkanal, welcher zu dem Fluoreszenzfarbstoff korrespondiert,
   - Bestimmen einer Teilfläche (TF1, TF2, TF3) des Fluoreszenzbildes, welche für eine Ausbildung des Fluoreszenzmustertyps relevant ist, durch Segmentieren des Fluoreszenzbildes (FB, FB1, FB2, FB3) mittels eines ersten Neuronalen Netzes (NN1, NN1A),
   - Bestimmen eines Konfidenzmaßes (KM), welches eine tatsächliche Präsenz des Fluoreszenzmustertyps indiziert, auf Basis des Fluoreszenzbildes (FB, FB1, FB2, FB3) mittels eines zweiten neuronalen Netzes (NN2, NN2A),
   - Bestimmen einer Validitätsinformation (VI), welche einen Grad einer Validität des Konfidenzmaßes (KM) indiziert, auf Basis der zuvor bestimmten Teilfläche,
   - Ausgeben des Konfidenzmaßes (KM) der tatsächlichen Präsenz des Fluoreszenzmustertypes und der Validitätsinformation (VI).

2. Verfahren nach Anspruch 1,

   ausgebildet zum Detektieren jeweiliger potentieller Präsenzen jeweiliger Fluoreszenzmustertypen auf einem Organschnitt mittels Immunfluoreszenzmikroskopie und mittels digitaler Bildverarbeitung, ferner aufweisend

   - Bestimmen einer Teilfläche (TF1, TF2, TF3) des Fluoreszenzbildes, welche für eine Ausbildung der Fluoreszenzmustertypen potentiell relevant ist, durch Segmentieren des Fluoreszenzbildes (FB, FB1, FB2, FB3) mittels eines ersten Neuronalen Netzes (NN1, NN1A),
   - Bestimmen jeweiliger Konfidenzmaße (KM), welche jeweilige tatsächliche Präsenzen der jeweiligen Fluoreszenzmustertypen indizieren, auf Basis des Fluoreszenzbildes (FB, FB1, FB2, FB3) sowie vorzugsweise auf Basis einer Information, welche die Teilfläche indiziert, mittels eines zweiten neuronalen Netzes (NN2, NN2A),
   - Bestimmen einer Validitätsinformation (VI), welche einen Grad einer Validität der Konfidenzmaße (KM) indiziert, auf Basis der zuvor bestimmten Teilfläche (TF1, TF2, TF3),
   - Ausgeben zumindest einer Teilmenge der jeweiligen Konfidenzmaße (KM) der jeweiligen tatsächlichen Präsenzen der jeweiligen Fluoreszenzmustertypen und der Validitätsinformation (VI).

3. Verfahren nach Anspruch 1, ferner aufweisend

   - Bestimmen des Konfidenzmaßes (KM) auf Basis des Fluoreszenzbildes (FB3) und auf Basis des segmentierten Fluoreszenzbildes (SEGA) mittels des zweiten neuronalen Netzes (NN2A).

4. Verfahren nach Anspruch 1, ferner aufweisend

   - Bestimmen der Validitätsinformation (VI) mittels Bestimmen eines Anteiles einer flächigen Abdeckung des Fluoreszenzbildes (FB1, FB2, FB3) durch die Teilfläche (TF1, TF2, TF3), welche für eine Ausbildung von Fluoreszenzmustern potentiell relevant ist.

**5.** Verfahren nach Anspruch 1,
ferner aufweisend,

- in dem Fall, dass ein Fluoreszenzmustertyp als tatsächlich präsent bestimmt wird, Bestimmen eines Helligkeitsgrades (HI) der Teilfläche in dem Fluoreszenzbild (FB, FB1, FB2, FB3), welche für eine Ausbildung des Fluoreszenzmustertyps potentiell relevant ist.

**6.** Verfahren nach Anspruch 5,
ferner aufweisend

- Abschätzen eines maximalen Verdünnungsgrades (VG) der Patientenprobe, bei welchem eine Inkubation des Organschnittes mit der Patientenprobe noch zu einer Präsenz eines des Fluoreszenzmustertyps führt.

**7.** Verfahren nach Anspruch 1,
ferner aufweisend

- Bestimmen des Konfidenzmaßes (KM) auf Basis des Fluoreszenzbildes (FB, FB1, FB2, FB3) und auf Basis einer Information, welche die Teilfläche indiziert, mittels des zweiten neuronalen Netzes (NN2, NN2A).

**8.** Vorrichtung (V1) zum Detektieren wenigstens einer potentiellen Präsenz wenigstens eines Fluoreszenzmustertyps auf einem Organschnitt mittels Immunfluoreszenzmikroskopie und mittels digitaler Bildverarbeitung, aufweisend

- eine Haltevorrichtung (H) für einen Objektträger mit einem Organschnitt (S), welcher mit einer Patientenprobe, aufweisend die Autoantikörper, sowie ferner mit sekundären Antikörpern, welche jeweils mit einem Fluoreszenzfarbstoff markiert sind, inkubiert wurde,
- wenigstens eine Bilderfassungseinheit (K1, K2) zum Erfassen eines Fluoreszenzbildes (SG) des Organschnittes (S) in einem Farbkanal, welcher zu dem Fluoreszenzfarbstoff korrespondiert,

sowie ferner aufweisend wenigstens eine Recheneinheit (R), welche ausgebildet ist,

- eine Teilfläche (TF1, TF2, TF3) in dem Fluoreszenzbild (FB1, FB2, FB3) zu bestimmen, welche für eine Ausbildung des Fluoreszenzmustertyps relevant ist, durch Segmentieren des Fluoreszenzbildes (FB1, FB2, FB3) mittels eines ersten Neuronalen Netzes (NN1, NN1A),
- ein Konfidenzmaß (KM) zu bestimmen, welches eine tatsächliche Präsenz des Fluoreszenzmustertyps indiziert, auf Basis des Fluoreszenzbildes (FB1, FB2, FB3) mittels eines zweiten neuronalen Netzes (NN2, NN2A),
- eine Validitätsinformation (VI) zu bestimmen, welche einen Grad einer Validität des Konfidenzmaßes (KM) indiziert, auf Basis der Teilfläche (TF1, TF2, TF3),
- sowie ferner das Konfidenzmaß (KM) der tatsächlichen Präsenzen des Fluoreszenzmustertyps und die Validitätsinformation (VI) auszugeben.

**9.** Recheneinheit (R), welche ausgebildet ist im Zuge einer digitalen Bildverarbeitung

- ein Fluoreszenzbild (FB1, FB2, FB3) entgegenzunehmen, welches eine Färbung eines Organschnittes durch einen Fluoreszenzfarbstoff repräsentiert,
- eine Teilfläche (TF1, TF2, TF3) in dem Fluoreszenzbild zu bestimmen, welche für eine Ausbildung des Fluoreszenzmustertyps relevant ist, durch Segmentieren des Fluoreszenzbildes (FB1, FB2, FB3) mittels eines ersten Neuronalen Netzes (NN1, NN1A),
- ein Konfidenzmaß (KM) zu bestimmen, welches eine tatsächliche Präsenz des Fluoreszenzmustertyps indiziert, auf Basis des Fluoreszenzbildes (FB1, FB2, FB3) mittels eines zweiten neuronalen Netzes (NN2, NN2A),
- eine Validitätsinformation (VI) zu bestimmen, welche einen Grad einer Validität des Konfidenzmaßes (KM) indiziert, auf Basis der zuvor bestimmten Teilfläche (TF1, TF2, TF3),
- sowie ferner das Konfidenzmaß (KM) der tatsächlichen Präsenz des Fluoreszenzmustertyps und die Validitätsinformation (VI) auszugeben.

**10.** Datennetzwerkvorrichtung (DV), aufweisend

- wenigstens eine Datenschnittstelle (DS4) zum Entgegennehmen eines Fluoreszenzbildes, welches eine Färbung eines Organschnittes durch einen Fluoreszenzfarbstoff repräsentiert, sowie ferner aufweisend wenigstens eine Recheneinheit (R), welche ausgebildet ist im Zuge einer digitalen Bildverarbeitung

- eine Teilfläche in dem Fluoreszenzbild zu bestimmen, welche für eine Ausbildung des Fluoreszenzmustertyps relevant ist, durch Segmentieren des Fluoreszenzbildes mittels eines ersten Neuronalen Netzes,
- ein Konfidenzmaß zu bestimmen, welches eine tatsächliche Präsenz des Fluoreszenzmustertyps indiziert, auf Basis des Fluoreszenzbildes mittels eines zweiten neuronalen Netzes,
- eine Validitätsinformation zu bestimmen, welche einen Grad einer Validität des Konfidenzmaßes indiziert, auf Basis der zuvor bestimmten Teilfläche,
- sowie ferner das Konfidenzmaß der tatsächlichen Präsenz des Fluoreszenzmustertyps und die Validitätsinformation auszugeben.

**11.** Verfahren zur digitalen Bildverarbeitung
aufweisend

- Entgegennehmen eines Fluoreszenzbildes, welches eine Färbung eines Organschnittes (S), durch einen Fluoreszenzfarbstoff repräsentiert,
- Bestimmen einer Teilfläche in dem Fluoreszenzbild, welche für eine Ausbildung des Fluoreszenzmustertyps relevant ist, durch Segmentieren des Fluoreszenzbildes mittels eines ersten Neuronalen Netzes,
- Bestimmen eines Konfidenzmaßes, welches eine tatsächliche Präsenz des Fluoreszenzmustertyps indiziert, auf Basis des Fluoreszenzbildes mittels eines zweiten neuronalen Netzes,
- Bestimmen einer Validitätsinformation, welche einen Grad einer Validität des Konfidenzmaßes indiziert, auf Basis der zuvor bestimmten Teilfläche,
- Ausgeben des Konfidenzmaßes der tatsächlichen Präsenz des Fluoreszenzmustertypes und der Validitätsinformation.

**12.** Computerprogrammprodukt (CPP), umfassend Befehle, die bei der Ausführung des Programms durch einen Computer diesen veranlassen, das Verfahren zur digitalen Bildverarbeitung nach Anspruch 11 durchzuführen.

**13.** Datenträgersignal (SI2), welches das Computerprogrammprodukt (CPP) nach Anspruch 12 überträgt.

**Claims**

**1.** Method for detecting at least one potential presence of at least one fluorescence pattern type on an organ section by means of immunofluorescence microscopy and by means of digital image processing,
comprising

- providing the organ section on a slide,
- incubating the organ section with a liquid patient sample which potentially comprises primary antibodies,
- incubating the organ section with secondary antibodies which have been labelled with a fluorescent dye,
- acquiring a fluorescence image (FB, FB1, FB2, FB3) of the organ section in a colour channel corresponding to the fluorescent dye,
- determining, by segmentation of the fluorescence image (FB, FB1, FB2, FB3) by means of a first neural network (NN1, NN1A), a sub-area (TF1, TF2, TF3) of the fluorescence image that is relevant to formation of the fluorescence pattern type,
- determining, on the basis of the fluorescence image (FB, FB1, FB2, FB3) by means of a second neural network (NN2, NN2A), a measure of confidence (KM) that indicates an actual presence of the fluorescence pattern type,
- determining, on the basis of the previously determined sub-area, validity information (VI) that indicates a degree of a validity of the measure of confidence (KM),
- outputting the measure of confidence (KM) of the actual presence of the fluorescence pattern type and the validity information (VI).

**2.** Method according to Claim 1,

designed for detection of respective potential presences of respective fluorescence pattern types on an organ section by means of immunofluorescence microscopy and by means of digital image processing,
further comprising

- determining, by segmentation of the fluorescence image (FB, FB1, FB2, FB3) by means of a first neural network (NN1, NN1A), a sub-area (TF1, TF2, TF3) of the fluorescence image that is potentially relevant to formation of the fluorescence pattern types,
- determining, on the basis of the fluorescence image (FB, FB1, FB2, FB3), and preferably on the basis of information indicating the sub-area, by means of a second neural network (NN2, NN2A), respective measures of confidence (KM) that indicate respective actual presences of the respective fluorescence pattern types,
- determining, on the basis of the previously determined sub-area (TF1, TF2, TF3), validity information (VI) that indicates a degree of a validity of the measures of confidence (KM),
- outputting at least a subset of the respective measures of confidence (KM) of the respective actual presences of the respective fluorescence pattern types and the validity information (VI).

3. Method according to Claim 1,
   further comprising

   - determining the measure of confidence (KM) on the basis of the fluorescence image (FB3) and on the basis of the segmented fluorescence image (SEGA) by means of the second neural network (NN2A).

4. Method according to Claim 1,
   further comprising

   - determining the validity information (VI) by means of determination of a proportion of a planar coverage of the fluorescence image (FB1, FB2, FB3) due to the sub-area (TF1, TF2, TF3) potentially relevant to formation of fluorescence patterns.

5. Method according to Claim 1,
   further comprising,

   - in the event of a fluorescence pattern type being determined as actually present, determining a degree of brightness (HI) of the sub-area in the fluorescence image (FB, FB1, FB2, FB3) that is potentially relevant to formation of the fluorescence pattern type.

6. Method according to Claim 5,
   further comprising

   - estimating a maximum degree of dilution (VG) of the patient sample at which incubation of the organ section with the patient sample still leads to a presence of one of the fluorescence pattern type.

7. Method according to Claim 1,
   further comprising

   - determining the measure of confidence (KM) on the basis of the fluorescence image (FB, FB1, FB2, FB3), and on the basis of information indicating the sub-area, by means of the second neural network (NN2, NN2A).

8. Apparatus (V1) for detecting at least one potential presence of at least one fluorescence pattern type on an organ section by means of immunofluorescence microscopy and by means of digital image processing,

   comprising

   - a holding device (H) for a slide containing an organ section (S) which has been incubated with a patient sample comprising autoantibodies and furthermore with secondary antibodies which have each been labelled with a fluorescent dye,
   - at least one image acquisition unit (K1, K2) for acquiring a fluorescence image (SG) of the organ section (S) in a colour channel corresponding to the fluorescent dye,

   and further comprising at least one computing unit (R) designed

   - to determine, by segmentation of the fluorescence image (FB1, FB2, FB3) by means of a first neural network (NN1, NN1A), a sub-area (TF1, TF2, TF3) in the fluorescence image (FB1, FB2, FB3) that is relevant to formation of the fluorescence pattern type,

- to determine, on the basis of the fluorescence image (FB1, FB2, FB3) by means of a second neural network (NN2, NN2A), a measure of confidence (KM) that indicates an actual presence of the fluorescence pattern type,

- to determine, on the basis of the sub-area (TF1, TF2, TF3), validity information (VI) that indicates a degree of a validity of the measure of confidence (KM),

- and furthermore to output the measure of confidence (KM) of the actual presences of the fluorescence pattern type and the validity information (VI).

9. Computing unit (R) which, in the course of digital image processing, is designed

- to receive a fluorescence image (FB1, FB2, FB3) representing staining of an organ section due to a fluorescent dye,

- to determine, by segmentation of the fluorescence image (FB1, FB2, FB3) by means of a first neural network (NN1, NN1A), a sub-area (TF1, TF2, TF3) in the fluorescence image that is relevant to formation of the fluorescence pattern type,

- to determine, on the basis of the fluorescence image (FB1, FB2, FB3) by means of a second neural network (NN2, NN2A), a measure of confidence (KM) that indicates an actual presence of the fluorescence pattern type,

- to determine, on the basis of the previously determined sub-area (TF1, TF2, TF3), validity information (VI) that indicates a degree of a validity of the measure of confidence (KM),

- and furthermore to output the measure of confidence (KM) of the actual presence of the fluorescence pattern type and the validity information (VI).

10. Data network device (DV) comprising

- at least one data interface (DS4) for receiving a fluorescence image representing staining of an organ section due to a fluorescent dye, and further comprising at least one computing unit (R) which, in the course of digital image processing, is designed

- to determine, by segmentation of the fluorescence image by means of a first neural network, a sub-area in the fluorescence image that is relevant to formation of the fluorescence pattern type,

- to determine, on the basis of the fluorescence image by means of a second neural network, a measure of confidence that indicates an actual presence of the fluorescence pattern type,

- to determine, on the basis of the previously determined sub-area, validity information that indicates a degree of a validity of the measure of confidence,

- and furthermore to output the measure of confidence of the actual presence of the fluorescence pattern type and the validity information.

11. Method for digital image processing comprising

- receiving a fluorescence image representing staining of an organ section (S) due to a fluorescent dye,

- determining, by segmentation of the fluorescence image by means of a first neural network, a sub-area in the fluorescence image that is relevant to formation of the fluorescence pattern type,

- determining, on the basis of the fluorescence image by means of a second neural network, a measure of confidence that indicates an actual presence of the fluorescence pattern type,

- determining, on the basis of the previously determined sub-area, validity information that indicates a degree of a validity of the measure of confidence,

- outputting the measure of confidence of the actual presence of the fluorescence pattern type and the validity information.

12. Computer program product (CPP) comprising commands which, upon execution of the program by a computer, prompt said computer to carry out the method for digital image processing according to Claim 11.

13. Data carrier signal (SI2) which transmits the computer program product (CPP) according to Claim 12.

**Revendications**

1. Procédé de détection d'au moins une présence potentielle d'au moins un type de motif de fluorescence sur une coupe d'organe par microscopie par immunofluorescence et traitement d'image numérique, ledit procédé comprenant les étapes suivantes

   - fournir la coupe d'organe sur un porte-objet,
   - laisser incuber la coupe d'organe avec un échantillon liquide du patient qui comporte potentiellement des anticorps primaires,
   - laisser incuber la coupe d'organe avec des anticorps secondaires marqués avec un colorant fluorescent,
   - acquérir une image de fluorescence (FB, FB1, FB2, FB3) de la coupe d'organe dans un canal de couleurs qui correspond au colorant fluorescent,
   - déterminer une partie de surface (TF1, TF2, TF3) de l'image de fluorescence, qui est pertinente pour former le type de motif de fluorescence, par segmentation de l'image de fluorescence (FB, FB1, FB2, FB3) à l'aide d'un premier réseau de neurones (NN1, NN1A),
   - déterminer un niveau de confiance (KM), qui indique une présence réelle du type de motif de fluorescence, sur la base de l'image de fluorescence (FB, FB1, FB2, FB3) au moyen d'un deuxième réseau de neurones (NN2, NN2A),
   - déterminer une information de validité (VI), qui indique un degré de validité de la mesure de confiance (KM), sur la base de la partie de surface précédemment déterminée,
   - délivrer en sortie la mesure de confiance (KM) de la présence réelle du type de motif de fluorescence et de l'information de validité (VI).

2. Procédé selon la revendication 1, conçu pour détecter des présences potentielles respectives de types de motifs de fluorescence respectifs sur une coupe d'organe par microscopie par immunofluorescence et traitement d'image numérique, le procédé comprenant en outre les étapes suivantes

   - déterminer une partie de surface (TF1, TF2, TF3) de l'image de fluorescence, qui est potentiellement pertinente pour former les types de motifs de fluorescence, par segmentation de l'image de fluorescence (FB, FB1, FB2, FB3) à l'aide d'un premier réseau de neurones (NN1, NN1A),
   - déterminer des mesures de confiance respectives (KM), qui indiquent les présences réelles respectives des types de motifs de fluorescence respectifs, sur la base de l'image de fluorescence (FB, FB1, FB2, FB3) et de préférence sur la base d'une information, qui indique la partie de surface, à l'aide d'un deuxième réseau de neurones (NN2, NN2A),
   - déterminer une information de validité (VI), qui indique un degré de validité des mesures de confiance (KM), sur la base de la partie de surface préalablement déterminée (TF1, TF2, TF3),
   - délivrer en sortie au moins un sous-ensemble des mesures de confiance respectives (KM) des présences réelles respectives des types de motifs de fluorescence respectifs et de l'information de validité (VI).

3. Procédé selon la revendication 1, comportant en outre l'étape suivante

   - déterminer la mesure de confiance (KM) sur la base de l'image de fluorescence (FB3) et sur la base de l'image de fluorescence segmentée (SEGA) à l'aide du deuxième réseau de neurones (NN2A).

4. Procédé selon la revendication 1, comportant en outre l'étape suivante

   - déterminer l'information de validité (VI) par détermination d'une proportion d'une couverture sensiblement bidimensionnelle de l'image de fluorescence (FB1, FB2, FB3) par la partie de surface (TF1, TF2, TF3), qui est potentiellement pertinente pour la formation de motifs de fluorescence.

5. Procédé selon la revendication 1, comportant en outre l'étape suivante

   - dans le cas où un type de motif de fluorescence est déterminé comme étant réellement présent, déterminer un degré de luminosité (HI) de la partie de surface dans l'image de fluorescence (FB, FB1, FB2, FB3) qui est potentiellement pertinente pour former le type de motif de fluorescence.

6. Procédé selon la revendication 5, comportant en outre l'étape suivante

- estimer un degré de dilution maximal (VG) de l'échantillon de patient pour lequel l'incubation de la coupe d'organe avec l'échantillon de patient entraîne toujours la présence d'un type de motif de fluorescence.

7. Procédé selon la revendication 1, comportant en outre l'étape suivante

- déterminer la mesure de confiance (KM) sur la base de l'image de fluorescence (FB, FB1, FB2, FB3) et sur la base d'une information, qui indique la partie de surface, à l'aide du deuxième réseau de neurones (NN2, NN2A).

8. Dispositif (V1) de détection d'au moins une présence potentielle d'au moins un type de motif de fluorescence sur une coupe d'organe par microscopie par immunofluorescence et traitement numérique d'images, ledit dispositif comportant

- un dispositif de retenue (H) destiné à un porte-objet pourvu d'une coupe d'organe (S), qui a été incubée avec un échantillon du patient contenant les auto-anticorps, et également d'anticorps secondaires, qui sont chacun marqués avec un colorant fluorescent,
- au moins une unité d'acquisition d'images (K1, K2) destinée à acquérir une image de fluorescence (SG) de la coupe d'organe (S) dans un canal de couleurs qui correspond au colorant fluorescent,

et comportant en outre au moins une unité informatique (R) qui est conçue pour

- déterminer une partie de surface (TF1, TF2, TF3) dans l'image de fluorescence (FB1, FB2, FB3), qui est pertinente pour former le type de motif de fluorescence, par segmentation de l'image de fluorescence (FB1, FB2, FB3) à l'aide un premier réseau de neurones (NN1, NN1A),
- déterminer une mesure de confiance (KM), qui indique une présence réelle du type de motif de fluorescence, sur la base de l'image de fluorescence (FB1, FB2, FB3) à l'aide d'un deuxième réseau de neurones (NN2, NN2A),
- déterminer une information de validité (VI), qui indique un degré de validité de la mesure de confiance (KM), sur la base de la partie de surface (TF1, TF2, TF3),
- et en outre délivrer en sortie la mesure de confiance (KM) des présences réelles du type de motif de fluorescence et l'information de validité (VI).

9. Unité informatique (R), qui est conçue pour

- recevoir une image de fluorescence (FB1, FB2, FB3), qui représente une coloration d'une coupe d'organe par un colorant fluorescent,
- déterminer une partie de surface (TF1, TF2, TF3) dans l'image de fluorescence, qui est pertinente pour former le type de motif de fluorescence, par segmentation de l'image de fluorescence (FB1, FB2, FB3) à l'aide d'un premier réseau de neurones (NN1, NN1A),
- déterminer une mesure de confiance (KM), qui indique une présence réelle du type de motif de fluorescence, sur la base de l'image de fluorescence (FB1, FB2, FB3) à l'aide d'un deuxième réseau de neurones (NN2, NN2A),
- déterminer une information de validité (VI), qui indique un degré de validité de la mesure de confiance (KM), sur la base de la partie de surface précédemment déterminée (TF1, TF2, TF3),
- et en outre délivrer en sortie la mesure de confiance (KM) de la présence réelle du type de motif de fluorescence et de l'information de validité (VI), au cours d'un traitement d'image numérique.

10. Dispositif de réseau de données (DV), comportant

- au moins une interface de données (DS4) destinée à recevoir une image de fluorescence, qui représente une coloration d'une coupe d'organe par un colorant fluorescent, et comportant également au moins une unité informatique (R) qui est conçue pour
- déterminer une partie de surface dans l'image de fluorescence, qui est pertinente pour former le type motif de fluorescence, par segmentation de l'image de fluorescence à l'aide d'un premier réseau de neurones,
- déterminer une mesure de confiance qui indique une présence réelle du type motif de fluorescence, sur la base de l'image de fluorescence à l'aide d'un deuxième réseau de neurones,
- déterminer une information de validité, qui indique un degré de validité de la mesure de confiance, sur la base de la partie de surface précédemment déterminée,
- et en outre délivrer en sortie la mesure de confiance de la présence réelle du type de motif de fluorescence et de l'information de validité, au cours du traitement d'image numérique.

**11.** Procédé de traitement d'image numérique comportant les étapes suivantes

- recevoir une image de fluorescence, qui représente une coloration d'une coupe d'organe (S) par un colorant fluorescent,
- déterminer une partie de surface dans l'image de fluorescence, qui est pertinente pour former le type de motif de fluorescence, par segmentation de l'image de fluorescence à l'aide d'un premier réseau de neurones,
- déterminer une mesure de confiance, qui indique une présence réelle du type de motif de fluorescence, sur la base de l'image de fluorescence à l'aide d'un deuxième réseau de neurones,
- déterminer une information de validité, qui indique un degré de validité de la mesure de confiance, sur la base de la partie de surface précédemment déterminée,
- délivrer en sortie la mesure de confiance de la présence réelle du type de motif de fluorescence et de l'information de validité.

**12.** Progiciel (CPP) comprenant des instructions qui, lorsque le programme est exécuté par un ordinateur, ordonnent à celui-ci de mettre en oeuvre le procédé de traitement d'images numérique selon la revendication 11.

**13.** Signal porteur de données (SI2) qui transmet le progiciel (CPP) selon la revendication 12.

**FIG. 1**

**FIG. 2**

## FIG. 3

FB3

SEGA

TF3

S7

VI

## FIG. 4

S6

FB

SEG,SEGA

NN2, NN2A

VKM

SWL

PS

KM

**FIG. 5**

Fig.6

# Fig.7

## Fig.8

HG      TB21     FB, FB2

AF

Fig.9

# Fig. 10

# Fig.11

Fig.12

Epithelium E

Lamina Propia D

Muscularis mucosae C

Circular Muscle B

Longitudinal Muscle A

Fig.13

FB, FB3

E    D    C  B  A

HGF

Fig.14a                    FB4

Fig.14b

FB5

Fig.15

a

FB3

b

A

B

C

D

E

SEG3

HGF

c

FB33

d

TF3

SEGA

Fig.16

**FIG. 17**

SI3

DS3

AE

DS2

SI

FB

R

AS

**FIG.18**

DV

FB

DS4

SI1

MEM

IDB

R

**FIG.19**

CO

CPP

DSX

SI2

## Fig. 20 a

**LKM**

**EUROPattern visual mode**

|  | found | not found | Total |
|---|---|---|---|
| **found** | 11 | 1 | 12 |
| **not found** | 1 | 79 | 80 |
| **Total** | 12 | 80 | 92 |

*EPa-Classifier*

| | |
|---|---|
| Sensitivity: | 91,67% |
| Specificity: | 98,75% |
| Overall agreement: | 97,83% |

20fach Vergrößerung, Gerät EUROPatternMicroscope 1.5.1

**Tabelle 1**

## Fig. 20 b

**AMA**

**EUROPattern visual mode**

|  | found | not found | Total |
|---|---|---|---|
| **found** | 11 | 1 | 12 |
| **not found** | 1 | 79 | 80 |
| **Total** | 12 | 80 | 92 |

*EPa-Classifier*

| | |
|---|---|
| Sensitivity: | 91,67% |
| Specificity: | 98,75% |
| Overall agreement: | 97,83% |

20fach Vergrößerung, Gerät EUROPatternMicroscope 1.5.1

**Tabelle 2**

## Fig. 21 a

**LKM**

**EUROPattern visual mode**

| EPa-Classifier | | found | not found | Total |
|---|---|---|---|---|
| | **found** | 11 | 1 | 12 |
| | **not found** | 1 | 80 | 81 |
| | **Total** | 12 | 81 | 93 |

| | |
|---|---|
| Sensitivity: | 91,67% |
| Specificity: | 98,77% |
| Overall agreement: | 97,85% |

10fach Vergrößerung, Gerät EUROPatternMicroscope 1.5.1

**Tabelle 3**

## Fig. 21 b

**AMA**

**EUROPattern visual mode**

| EPa-Classifier | | found | not found | Total |
|---|---|---|---|---|
| | **found** | 12 | 0 | 12 |
| | **not found** | 0 | 81 | 81 |
| | **Total** | 12 | 81 | 93 |

| | |
|---|---|
| Sensitivity: | 100,00% |
| Specificity: | 100,00% |
| Overall agreement: | 100,00% |

10fach Vergrößerung, Gerät EUROPatternMicroscope 1.5.1

**Tabelle 4**

## Fig. 22a

**LKM**

**EUROPattern visual mode**

| EPa-Classifier | | found | not found | Total |
|---|---|---|---|---|
| | **found** | 11 | 0 | 11 |
| | **not found** | 1 | 80 | 81 |
| | **Total** | 12 | 80 | 92 |

| | |
|---|---|
| Sensitivity: | 91,67% |
| Specificity: | 100,00% |
| Overall agreement: | 98,91% |

20fach Vergrößerung, Gerät EUROPatternMicroscopeLive

**Tabelle 5**

## Fig. 22b

**AMA**

**EUROPattern visual mode**

| EPa-Classifier | | found | not found | Total |
|---|---|---|---|---|
| | **found** | 11 | 4 | 15 |
| | **not found** | 1 | 76 | 77 |
| | **Total** | 12 | 80 | 92 |

| | |
|---|---|
| Sensitivity: | 91,67% |
| Specificity: | 95,00% |
| Overall agreement: | 94,57% |

20fach Vergrößerung, Gerät EUROPatternMicroscopeLive

**Tabelle 6**

## Fig. 23a

### Endomysium

**EUROPattern visual mode**

<div style="text-align:center">EPa-Classifier</div>

|  | found | not found | Total |
|---|---|---|---|
| **found** | 69 | 2 | 71 |
| **not found** | 0 | 165 | 165 |
| **Total** | 69 | 167 | 236 |

Sensitivity: 100,00%

Specificity: 98,80%

Overall agreement: 99,15%

10fach Vergrößerung, Gerät EUROPatternMicroscope 1.5.1

**Tabelle 7**

## Fig. 23b

### Endomysium

**EUROPattern visual mode**

|  | found | not found | Total |
|---|---|---|---|
| **found** | 69 | 0 | 69 |
| **not found** | 0 | 167 | 167 |
| **Total** | 69 | 167 | 236 |

Sensitivity: 100,00%

Specificity: 100,00%

Overall agreement: 100,00%

20fach Vergrößerung, Gerät EUROPatternMicroscope Live

**Tabelle 8**

FIG. 24 (1/4)

| 1 |
|---|
| 2 |
| 3 |
| 4 |

FB

NN21

| input_3: InputLayer | input: | [(?, 2048, 2048, 1)] |
|---|---|---|
| | output: | [(?, 2048, 2048, 1)] |

| lambda_2: Lambda | input: | (?, 2048, 2048, 1) |
|---|---|---|
| | output: | (?, 2048, 2048, 1) |

| conv2d_32: Conv2D | input: | (?, 2048, 2048, 1) |
|---|---|---|
| | output: | (?, 2048, 2048, 8) |

| batch_normalization_18: BatchNormalization | input: | (?, 2048, 2048, 8) |
|---|---|---|
| | output: | (?, 2048, 2048, 8) |

| leaky_re_lu_32: LeakyReLU | input: | (?, 2048, 2048, 8) |
|---|---|---|
| | output: | (?, 2048, 2048, 8) |

EP 4 016 081 B1

FIG. 24 (2/4)

| | 1 |
|---|---|
| | 2 |
| | 3 |
| | 4 |

| conv2d_34: Conv2D | input: | (?, 2048, 2048, 8) |
|---|---|---|
| | output: | (?, 2048, 2048, 12) |

BSP

| leaky_re_lu_34: LeakyReLU | input: | (?, 2048, 2048, 12) |
|---|---|---|
| | output: | (?, 2048, 2048, 12) |

| conv2d_33: Conv2D | input: | (?, 2048, 2048, 8) |
|---|---|---|
| | output: | (?, 1024, 1024, 12) |

| max_pooling2d_14: MaxPooling2D | input: | (?, 2048, 2048, 12) |
|---|---|---|
| | output: | (?, 1024, 1024, 12) |

| leaky_re_lu_33: LeakyReLU | input: | (?, 1024, 1024, 12) |
|---|---|---|
| | output: | (?, 1024, 1024, 12) |

| concatenate_14: Concatenate | input: | [(?, 1024, 1024, 12), (?, 1024, 1024, 12)] |
|---|---|---|
| | output: | (?, 1024, 1024, 24) |

| batch_normalization_19: BatchNormalization | input: | (?, 1024, 1024, 24) |
|---|---|---|
| | output: | (?, 1024, 1024, 24) |

EP 4 016 081 B1

FIG. 24 (3/4)

| 1 |
| 2 |
| 3 |
| 4 |

| conv2d_36: Conv2D | input: | (?, 1024, 1024, 24) |
|---|---|---|
| | output: | (?, 1024, 1024, 19) |

| leaky_re_lu_36: LeakyReLU | input: | (?, 1024, 1024, 19) |
|---|---|---|
| | output: | (?, 1024, 1024, 19) |

| conv2d_35: Conv2D | input: | (?, 1024, 1024, 24) |
|---|---|---|
| | output: | (?, 512, 512, 19) |

| max_pooling2d_15: MaxPooling2D | input: | (?, 1024, 1024, 19) |
|---|---|---|
| | output: | (?, 512, 512, 19) |

| leaky_re_lu_35: LeakyReLU | input: | (?, 512, 512, 19) |
|---|---|---|
| | output: | (?, 512, 512, 19) |

| concatenate_15: Concatenate | input: | [(?, 512, 512, 19), (?, 512, 512, 19)] |
|---|---|---|
| | output: | (?, 512, 512, 38) |

| batch_normalization_20: BatchNormalization | input: | (?, 512, 512, 38) |
|---|---|---|
| | output: | (?, 512, 512, 38) |

EP 4 016 081 B1

FIG. 24 (4/4)

| conv2d_38: Conv2D | input: | (?, 512, 512, 38) |
|---|---|---|
| | output: | (?, 512, 512, 30) |

| leaky_re_lu_38: LeakyReLU | input: | (?, 512, 512, 30) |
|---|---|---|
| | output: | (?, 512, 512, 30) |

| conv2d_37: Conv2D | input: | (?, 512, 512, 38) |
|---|---|---|
| | output: | (?, 256, 256, 30) |

| max_pooling2d_16: MaxPooling2D | input: | (?, 512, 512, 30) |
|---|---|---|
| | output: | (?, 256, 256, 30) |

| leaky_re_lu_37: LeakyReLU | input: | (?, 256, 256, 30) |
|---|---|---|
| | output: | (?, 256, 256, 30) |

| concatenate_16: Concatenate | input: | [(?, 256, 256, 30), (?, 256, 256, 30)] |
|---|---|---|
| | output: | (?, 256, 256, 60) |

EP 4 016 081 B1

FIG. 25 (1/4)

| 1 |
| 2 |
| 3 |
| 4 |

NN22

| batch_normalization_20: BatchNormalization | input: | (?, 512, 512, 38) |
|---|---|---|
| | output: | (?, 512, 512, 38) |

| conv2d_38: Conv2D | input: | (?, 512, 512, 38) |
|---|---|---|
| | output: | (?, 512, 512, 30) |

| leaky_re_lu_38: LeakyReLU | input: | (?, 512, 512, 30) |
|---|---|---|
| | output: | (?, 512, 512, 30) |

| conv2d_37: Conv2D | input: | (?, 512, 512, 38) |
|---|---|---|
| | output: | (?, 256, 256, 30) |

| max_pooling2d_16: MaxPooling2D | input: | (?, 512, 512, 30) |
|---|---|---|
| | output: | (?, 256, 256, 30) |

| leaky_re_lu_37: LeakyReLU | input: | (?, 256, 256, 30) |
|---|---|---|
| | output: | (?, 256, 256, 30) |

| concatenate_16: Concatenate | input: | [(?, 256, 256, 30), (?, 256, 256, 30)] |
|---|---|---|
| | output: | (?, 256, 256, 60) |

EP 4 016 081 B1

# FIG. 25 (2/4)

EP 4 016 081 B1

FIG. 25 (3/4)

FIG. 25 (4/4)

| | 1 |
|---|---|
| | 2 |
| | 3 |
| | 4 |

| batch_normalization_23: BatchNormalization | input: | (?, 64, 64, 152) |
|---|---|---|
| | output: | (?, 64, 64, 152) |

| conv2d_44: Conv2D | input: | (?, 64, 64, 152) |
|---|---|---|
| | output: | (?, 64, 64, 121) |

| leaky_re_lu_44: LeakyReLU | input: | (?, 64, 64, 121) |
|---|---|---|
| | output: | (?, 64, 64, 121) |

| conv2d_43: Conv2D | input: | (?, 64, 64, 152) |
|---|---|---|
| | output: | (?, 32, 32, 121) |

| max_pooling2d_19: MaxPooling2D | input: | (?, 64, 64, 121) |
|---|---|---|
| | output: | (?, 32, 32, 121) |

| leaky_re_lu_43: LeakyReLU | input: | (?, 32, 32, 121) |
|---|---|---|
| | output: | (?, 32, 32, 121) |

| concatenate_19: Concatenate | input: | [(?, 32, 32, 121), (?, 32, 32, 121)] |
|---|---|---|
| | output: | (?, 32, 32, 242) |

EP 4 016 081 B1

FIG. 26 (1/3)

EP 4 016 081 B1

NN23

| batch_normalization_24: BatchNormalization | input: | (?, 32, 32, 242) |
| --- | --- | --- |
| | output: | (?, 32, 32, 242) |

| conv2d_46: Conv2D | input: | (?, 32, 32, 242) |
| --- | --- | --- |
| | output: | (?, 32, 32, 193) |

| leaky_re_lu_46: LeakyReLU | input: | (?, 32, 32, 193) |
| --- | --- | --- |
| | output: | (?, 32, 32, 193) |

| conv2d_45: Conv2D | input: | (?, 32, 32, 242) |
| --- | --- | --- |
| | output: | (?, 16, 16, 193) |

| max_pooling2d_20: MaxPooling2D | input: | (?, 32, 32, 193) |
| --- | --- | --- |
| | output: | (?, 16, 16, 193) |

| leaky_re_lu_45: LeakyReLU | input: | (?, 16, 16, 193) |
| --- | --- | --- |
| | output: | (?, 16, 16, 193) |

FIG. 26 (2/3)

| 1 |
|---|
| 2 |
| 3 |

EP 4 016 081 B1

| concatenate_20: Concatenate | input: | [(?, 16, 16, 193), (?, 16, 16, 193)] |
|---|---|---|
| | output: | (?, 16, 16, 386) |

| batch_normalization_25: BatchNormalization | input: | (?, 16, 16, 386) |
|---|---|---|
| | output: | (?, 16, 16, 386) |

| conv2d_47: Conv2D | input: | (?, 16, 16, 386) |
|---|---|---|
| | output: | (?, 16, 16, 3) |

| batch_normalization_26: BatchNormalization | input: | (?, 16, 16, 3) |
|---|---|---|
| | output: | (?, 16, 16, 3) |

FIG. 26 (3/3)

EP 4 016 081 B1

| leaky_re_lu_47: LeakyReLU | input: | (?, 16, 16, 3) |
| | output: | (?, 16, 16, 3) |

| global_average_pooling2d_2: GlobalAveragePooling2D | input: | (?, 16, 16, 3) |
| | output: | (?, 3) |

| label: Activation | input: | (?, 3) |
| | output: | (?, 3) |

— SVKM2

— VKM

PS

— KM

EP 4 016 081 B1

**FIG. 27 (1/4)**

NN11

FB

| input_1: InputLayer | input: | (None, 512, 512, 1) |
| | output: | (None, 512, 512, 1) |

| lambda_1: Lambda | input: | (None, 512, 512, 1) |
| | output: | (None, 512, 512, 1) |

| conv2d_1: Conv2D | input: | (None, 512, 512, 1) |
| | output: | (None, 256, 256, 8) |

| conv2d_2: Conv2D | input: | (None, 256, 256, 8) |
| | output: | (None, 128, 128, 8) |

| conv2d_3: Conv2D | input: | (None, 256, 256, 8) |
| | output: | (None, 128, 128, 8) |

| batch_normalization_2: BatchNormalization | input: | (None, 128, 128, 8) |
| | output: | (None, 128, 128, 8) |

53

FIG. 27 (2/4)

FIG. 27 (3/4)

| 1 |
|---|
| 2 |
| 3 |
| 4 |

| batch_normalization_5: BatchNormalization | input: | (None, 64, 64,11) |
|---|---|---|
| | output: | (None, 64, 64,11) |

| conv2d_5: Conv2D | input: | (None, 128, 128, 8) |
|---|---|---|
| | output: | (None, 64, 64,11) |

| activation_3: Activation | input: | (None, 64, 64,11) |
|---|---|---|
| | output: | (None, 64, 64,11) |

| batch_normalization_4: BatchNormalization | input: | (None, 64, 64,11) |
|---|---|---|
| | output: | (None, 64, 64,11) |

| conv2d_7: Conv2D | input: | (None, 64, 64,11) |
|---|---|---|
| | output: | (None, 64, 64,11) |

| batch_normalization_6: BatchNormalization | input: | (None, 64, 64,11) |
|---|---|---|
| | output: | (None, 64, 64,11) |

| add_2: Add | input: | [(None, 64, 64,11), (None, 64, 64,11)] |
|---|---|---|
| | output: | (None, 64, 64,11) |

| activation_4: Activation | input: | (None, 64, 64,11) |
|---|---|---|
| | output: | (None, 64, 64,11) |

EP 4 016 081 B1

FIG. 27 (4/4)

| 1 |
| 2 |
| 3 |
| 4 |

| conv2d_9: Conv2D | input: | (None, 64, 64,11) |
| | output: | (None, 32, 32,15) |

| batch_normalization_8: BatchNormalization | input: | (None, 32, 32,15) |
| | output: | (None, 32, 32,15) |

| conv2d_8: Conv2D | input: | (None, 64, 64,11) |
| | output: | (None, 32, 32,15) |

| activation_5: Activation | input: | (None, 32, 32,15) |
| | output: | (None, 32, 32,15) |

| batch_normalization_7: BatchNormalization | input: | (None, 32, 32,15) |
| | output: | (None, 32, 32,15) |

| conv2d_10: Conv2D | input: | (None, 32, 32,15) |
| | output: | (None, 32, 32,15) |

| batch_normalization_9: BatchNormalization | input: | (None, 32, 32,15) |
| | output: | (None, 32, 32,15) |

| add_3: Add | input: | [(None, 32, 32,15), (None, 32, 32,15)] |
| | output: | (None, 32, 32,15) |

EP 4 016 081 B1

FIG. 28 (1/3)

FIG. 28 (1/3)

NN12

| activation_6: Activation | input: | (None, 32, 32,15) |
| | output: | (None, 32, 32,15) |

| conv2d_12: Conv2D | input: | (None, 32, 32, 15) |
| | output: | (None, 16, 16, 21) |

| batch_normalization_11: BatchNormalization | input: | (None, 16,16, 21) |
| | output: | (None, 16,16, 21) |

| conv2d_11: Conv2D | input: | (None, 32, 32, 15) |
| | output: | (None, 16, 16, 21) |

| activation_7: Activation | input: | (None, 16,16, 21) |
| | output: | (None, 16,16, 21) |

| batch_normalization_10: BatchNormalization | input: | (None, 16, 16, 21) |
| | output: | (None, 16, 16, 21) |

| conv2d_13: Conv2D | input: | (None, 16, 16, 21) |
| | output: | (None, 16, 16, 21) |

| batch_normalization_12: BatchNormalization | input: | (None, 16, 16, 21) |
| | output: | (None, 16, 16, 21) |

EP 4 016 081 B1

FIG. 28 (2/3)

| | 1 |
|---|---|
| | 2 |
| | 3 |

| add_4: Add | input: | [(None, 16, 16, 21), (None, 16, 16, 21)] |
|---|---|---|
| | output: | (None, 16, 16, 21) |

| activation_8: Activation | input: | (None, 16, 16, 21) |
|---|---|---|
| | output: | (None, 16, 16, 21) |

| conv2d_transpose_1: Conv2DTranspose | input: | (None, 16, 16, 21) |
|---|---|---|
| | output: | (None, 32, 32, 10) |

| batch_normalization_13: BatchNormalization | input: | (None, 32, 32, 10) |
|---|---|---|
| | output: | (None, 32, 32, 10) |

| activation_9: Activation | input: | (None, 32, 32, 10) |
|---|---|---|
| | output: | (None, 32, 32, 10) |

| concatenate_1: Concatenate | input: | [(None, 32, 32, 10), (None, 32, 32, 15)] |
|---|---|---|
| | output: | (None, 32, 32, 25) |

EP 4 016 081 B1

FIG. 28 (3/3)

**FIG. 29 (1/3)**

EP 4 016 081 B1

NN13

| activation_12: Activation | input: | (None, 64, 64, 7) |
|---|---|---|
| | output: | (None, 64, 64, 7) |

| conv2d_transpose_3: Conv2DTranspose | input: | (None, 64, 64, 7) |
|---|---|---|
| | output: | (None, 128, 128, 5) |

| batch_normalization_17: BatchNormalization | input: | (None, 128, 128, 5) |
|---|---|---|
| | output: | (None, 128, 128, 5) |

| activation_13: Activation | input: | (None, 128, 128, 5) |
|---|---|---|
| | output: | (None, 128, 128, 5) |

| concatenate_3: Concatenate | input: | [(None, 128, 128, 5), (None, 128, 128, 8)] |
|---|---|---|
| | output: | (None, 128, 128, 13) |

| conv2d_16: Conv2D | input: | (None, 128, 128, 13) |
|---|---|---|
| | output: | (None, 128, 128, 5) |

FIG. 29 (2/3)

| batch_normalization_18: BatchNormalization | input: | (None, 128, 128, 5) |
| | output: | (None, 128, 128, 5) |

| activation_14: Activation | input: | (None, 128, 128, 5) |
| | output: | (None, 128, 128, 5) |

| conv2d_transpose_4: Conv2DTranspose | input: | (None, 128, 128, 5) |
| | output: | (None, 256, 256, 3) |

| batch_normalization_19: BatchNormalization | input: | (None, 256, 256, 3) |
| | output: | (None, 256, 256, 3) |

| activation_15: Activation | input: | (None, 256, 256, 3) |
| | output: | (None, 256, 256, 3) |

| concatenate_4: Concatenate | input: | [(None, 256, 256, 3), (None, 256, 256, 8)] |
| | output: | (None, 256, 256, 11) |

FIG. 29 (3/3)

| 1 |
|---|
| 2 |
| 3 |

SEG

MS

SEG'

SC

| conv2d_17: Conv2D | input: | (None, 256, 256, 11) |
|---|---|---|
| | output: | (None, 256, 256, 3) |

| batch_normalization_20: BatchNormalization | input: | (None, 256, 256, 3) |
|---|---|---|
| | output: | (None, 256, 256, 3) |

| activation_16: Activation | input: | (None, 256, 256, 3) |
|---|---|---|
| | output: | (None, 256, 256, 3) |

| conv2d_transpose_5: Conv2DTranspose | input: | (None, 256, 256, 3) |
|---|---|---|
| | output: | (None, 512, 512, 8) |

| batch_normalization_21: BatchNormalization | input: | (None, 512, 512, 8) |
|---|---|---|
| | output: | (None, 512, 512, 8) |

| activation_17: Activation | input: | (None, 512, 512, 8) |
|---|---|---|
| | output: | (None, 512, 512, 8) |

| conv2d_18: Conv2D | input: | (None, 512, 512, 8) |
|---|---|---|
| | output: | (None, 512, 512, 2) |

EP 4 016 081 B1

FIG. 30 (1/3)

```
┌───┐
│ 1 │
├───┤
│ 2 │
├───┤
│ 3 │
└───┘
```

NN2A1

FB

SEGA

| input_3: InputLayer | input: | [(?, 2048, 2048, 1)] |
|---|---|---|
| | output: | [(?, 2048, 2048, 1)] |

| input_4: InputLayer | input: | [(?, 2048, 2048, 1)] |
|---|---|---|
| | output: | [(?, 2048, 2048, 1)] |

| conv2d_17: Conv2D | input: | (?, 2048, 2048, 1) |
|---|---|---|
| | output: | (?, 2048, 2048, 8) |

| conv2d_18: Conv2D | input: | (?, 2048, 2048, 1) |
|---|---|---|
| | output: | (?, 2048, 2048, 8) |

| batch_normalization_10: BatchNormalization | input: | (?, 2048, 2048, 8) |
|---|---|---|
| | output: | (?, 2048, 2048, 8) |

| batch_normalization_11: BatchNormalization | input: | (?, 2048, 2048, 8) |
|---|---|---|
| | output: | (?, 2048, 2048, 8) |

| leaky_re_lu_17: LeakyReLu | input: | (?, 2048, 2048, 8) |
|---|---|---|
| | output: | (?, 2048, 2048, 8) |

| leaky_re_lu_18: LeakyReLu | input: | (?, 2048, 2048, 8) |
|---|---|---|
| | output: | (?, 2048, 2048, 8) |

| concatenate_8: Concatenate | input: | [(?, 2048, 2048, 8), (?, 2048, 2048, 8)] |
|---|---|---|
| | output: | (?, 2048, 2048, 16) |

EP 4 016 081 B1

FIG. 30 (2/3)

| conv2d_20: Conv2D | input: | (?, 2048, 2048, 16) |
|---|---|---|
| | output: | (?, 2048, 2048, 12) |

| leaky_re_lu_20: LeakyReLu | input: | (?, 2048, 2048, 12) |
|---|---|---|
| | output: | (?, 2048, 2048, 12) |

| conv2d_19: Conv2D | input: | (?, 2048, 2048, 16) |
|---|---|---|
| | output: | (?, 1024, 1024, 12) |

| max_pooling2d_7: MaxPooling2D | input: | (?, 2048, 2048, 12) |
|---|---|---|
| | output: | (?, 1024, 1024, 12) |

| leaky_re_lu_19: LeakyReLu | input: | (?, 1024, 1024, 12) |
|---|---|---|
| | output: | (?, 1024, 1024, 12) |

| concatenate_9: Concatenate | input: | [(?, 1024, 1024, 12), (?, 1024, 1024, 12)] |
|---|---|---|
| | output: | (?, 1024, 1024, 24) |

| batch_normalization_12: BatchNormalization | input: | (?, 1024, 1024, 24) |
|---|---|---|
| | output: | (?, 1024, 1024, 24) |

| conv2d_22: Conv2D | input: | (?, 1024, 1024, 24) |
|---|---|---|
| | output: | (?, 1024, 1024, 18) |

| leaky_re_lu_22: LeakyReLu | input: | (?, 1024, 1024, 18) |
|---|---|---|
| | output: | (?, 1024, 1024, 18) |

| conv2d_21: Conv2D | input: | (?, 1024, 1024, 24) |
|---|---|---|
| | output: | (?, 512, 512, 18) |

EP 4 016 081 B1

FIG. 30 (3/3)

EP 4 016 081 B1

FIG. 31 (1/3)

NN2A2

| batch_normalization_14: BatchNormalization | input: | (?, 256, 256, 54) |
|---|---|---|
| | output: | (?, 256, 256, 54) |

| conv2d_26: Conv2D | input: | (?, 256, 256, 54) |
|---|---|---|
| | output: | (?, 256, 256, 40) |

| leaky_re_lu_26: LeakyReLU | input: | (?, 256, 256, 40) |
|---|---|---|
| | output: | (?, 256, 256, 40) |

| conv2d_25: Conv2D | input: | (?, 256, 256, 54) |
|---|---|---|
| | output: | (?, 128, 128, 40) |

| max_pooling2d_10: MaxPooling2D | input: | (?, 256, 256, 40) |
|---|---|---|
| | output: | (?, 128, 128, 40) |

| leaky_re_lu_25: LeakyReLU | input: | (?, 128, 128, 40) |
|---|---|---|
| | output: | (?, 128, 128, 40) |

| concatenate_12: Concatenate | input: | [(?, 128, 128, 40), (?, 128, 128, 40)] |
|---|---|---|
| | output: | (?, 128, 128, 80) |

EP 4 016 081 B1

FIG. 31 (2/3)

| batch_normalization_15: BatchNormalization | input: | (?, 128, 128, 80) |
|---|---|---|
| | output: | (?, 128, 128, 80) |

| conv2d_28: Conv2D | input: | (?, 128, 128, 80) |
|---|---|---|
| | output: | (?, 128, 128, 60) |

| leaky_re_lu_28: LeakyReLU | input: | (?, 128, 128, 60) |
|---|---|---|
| | output: | (?, 128, 128, 60) |

| conv2d_27: Conv2D | input: | (?, 128, 128, 80) |
|---|---|---|
| | output: | (?, 64, 64, 60) |

| max_pooling2d_11: MaxPooling2D | input: | (?, 128, 128, 60) |
|---|---|---|
| | output: | (?, 64, 64, 60) |

| leaky_re_lu_27: LeakyReLU | input: | (?, 64, 64, 60) |
|---|---|---|
| | output: | (?, 64, 64, 60) |

| concatenate_13: Concatenate | input: | [(?, 64, 64, 60), (?, 64, 64, 60)] |
|---|---|---|
| | output: | (?, 64, 64, 120) |

EP 4 016 081 B1

FIG. 31 (3/3)

| 1 |
|---|
| 2 |
| 3 |

| batch_normalization_16: BatchNormalization | input: | (?, 64, 64, 120) |
|---|---|---|
| | output: | (?, 64, 64, 120) |

| conv2d_30: Conv2D | input: | (?, 64, 64, 120) |
|---|---|---|
| | output: | (?, 64, 64, 90) |

| leaky_re_lu_30: LeakyReLU | input: | (?, 64, 64, 90) |
|---|---|---|
| | output: | (?, 64, 64, 90) |

| conv2d_29: Conv2D | input: | (?, 64, 64, 120) |
|---|---|---|
| | output: | (?, 32, 32, 90) |

| max_pooling2d_12: MaxPooling2D | input: | (?, 64, 64, 90) |
|---|---|---|
| | output: | (?, 32, 32, 90) |

| leaky_re_lu_29: LeakyReLU | input: | (?, 32, 32, 90) |
|---|---|---|
| | output: | (?, 32, 32, 90) |

| concatenate_14: Concatenate | input: | [(?, 32, 32, 90), (?, 32, 32, 90)] |
|---|---|---|
| | output: | (?, 32, 32, 180) |

EP 4 016 081 B1

**FIG. 32 (1/3)**

```
┌─┐
│1│
├─┤
┊2┊
├─┤
┊3┊
└─┘
```

NN2A3

| batch_normalization_17: BatchNormalization | input: | (?, 32, 32, 180) |
|---|---|---|
| | output: | (?, 32, 32, 180) |

| conv2d_32: Conv2D | input: | (?, 32, 32, 180) |
|---|---|---|
| | output: | (?, 32, 32, 135) |

| leaky_re_lu_32: LeakyReLU | input: | (?, 32, 32, 135) |
|---|---|---|
| | output: | (?, 32, 32, 135) |

| conv2d_31: Conv2D | input: | (?, 32, 32, 180) |
|---|---|---|
| | output: | (?, 16, 16, 135) |

| max_pooling2d_13: MaxPooling2D | input: | (?, 32, 32, 135) |
|---|---|---|
| | output: | (?, 16, 16, 135) |

| leaky_re_lu_31: LeakyReLU | input: | (?, 16, 16, 135) |
|---|---|---|
| | output: | (?, 16, 16, 135) |

| concatenate_15: Concatenate | input: | [(?, 16, 16, 135), (?, 16, 16, 135)] |
|---|---|---|
| | output: | (?, 16, 16, 270) |

EP 4 016 081 B1

FIG. 32 (2/3)

| batch_normalization_18: BatchNormalization | input: | (?, 16, 16, 270) |
|---|---|---|
| | output: | (?, 16, 16, 270) |

| conv2d_33: Conv2D | input: | (?, 16, 16, 270) |
|---|---|---|
| | output: | (?, 16, 16, 2) |

| batch_normalization_19: BatchNormalization | input: | (?, 16, 16, 2) |
|---|---|---|
| | output: | (?, 16, 16, 2) |

| leaky_re_lu_33: LeakyReLU | input: | (?, 16, 16, 2) |
|---|---|---|
| | output: | (?, 16, 16, 2) |

| global_average_pooling2d_1: GlobalAveragePooling2D | input: | (?, 16, 16, 2) |
|---|---|---|
| | output: | (?, 2) |

EP 4 016 081 B1

FIG. 32 (3/3)

**FIG. 33 (1/5)**

NN1A1

| input_1: InputLayer | input: | [(?, 512, 512, 1)] |
|---|---|---|
| | output: | [(?, 512, 512, 1)] |

| conv2d: Conv2D | input: | (?, 512, 512, 1) |
|---|---|---|
| | output: | (?, 512, 512, 16) |

| batch_normalization: BatchNormalization | input: | (?, 512, 512, 16) |
|---|---|---|
| | output: | (?, 512, 512, 16) |

| activation: Activation | input: | (?, 512, 512, 16) |
|---|---|---|
| | output: | (?, 512, 512, 16) |

| conv2d_3: Conv2D | input: | (?, 512, 512, 16) |
|---|---|---|
| | output: | (?, 512, 512, 22) |

| conv2d_2: Conv2D | input: | (?, 512, 512, 16) |
|---|---|---|
| | output: | (?, 256, 256, 22) |

| batch_normalization_3: BatchNormalization | input: | (?, 512, 512, 22) |
|---|---|---|
| | output: | (?, 512, 512, 22) |

| conv2d_1: Conv2D | input: | (?, 512, 512, 16) |
|---|---|---|
| | output: | (?, 256, 256, 22) |

| activation_3: Activation | input: | (?, 512, 512, 22) |
|---|---|---|
| | output: | (?, 512, 512, 22) |

| batch_normalization_2: BatchNormalization | input: | (?, 256, 256, 22) |
|---|---|---|
| | output: | (?, 256, 256, 22) |

| batch_normalization_1: BatchNormalization | input: | (?, 256, 256, 22) |
|---|---|---|
| | output: | (?, 256, 256, 22) |

| max_pooling2d: MaxPooling2D | input: | (?, 512, 512, 22) |
|---|---|---|
| | output: | (?, 256, 256, 22) |

| activation_2: Activation | input: | (?, 256, 256, 22) |
|---|---|---|
| | output: | (?, 256, 256, 22) |

| activation_1: Activation | input: | (?, 256, 256, 22) |
|---|---|---|
| | output: | (?, 256, 256, 22) |

FB

EP 4 016 081 B1

FIG. 33 (2/5)

| 1 |
| 2 |
| 3 |
| 4 |
| 5 |

| concatenate: Concatenate | input: | [(?, 256, 256, 22), (?, 256, 256, 22)] |
| | output: | (?, 256, 256, 44) |

| conv2d_4: Conv2D | input: | (?, 256, 256, 44) |
| | output: | (?, 256, 256, 22) |

| batch_normalization_4: BatchNormalization | input: | (?, 256, 256, 22) |
| | output: | (?, 256, 256, 22) |

| activation_4: Activation | input: | (?, 256, 256, 22) |
| | output: | (?, 256, 256, 22) |

| add: Add | input: | [(?, 256, 256, 22), (?, 256, 256, 22)] |
| | output: | (?, 256, 256, 22) |

| batch_normalization_5: BatchNormalization | input: | (?, 256, 256, 22) |
| | output: | (?, 256, 256, 22) |

| activation_5: Activation | input: | (?, 256, 256, 22) |
| | output: | (?, 256, 256, 22) |

| conv2d_7: Conv2D | input: | (?, 256, 256, 22) |
| | output: | (?, 256, 256, 30) |

| conv2d_6: Conv2D | input: | (?, 256, 256, 22) |
| | output: | (?, 128, 128, 30) |

EP 4 016 081 B1

FIG. 33 (3/5)

EP 4 016 081 B1

FIG. 33 (4/5)

1
2
3
4
5

| add_1: Add | input: | [(?, 128, 128, 30), (?, 128, 128, 30)] |
|---|---|---|
| | output: | (?, 128, 128, 30) |

| batch_normalization_10: BatchNormalization | input: | (?, 128, 128, 30) |
|---|---|---|
| | output: | (?, 128, 128, 30) |

| activation_10: Activation | input: | (?, 128, 128, 30) |
|---|---|---|
| | output: | (?, 128, 128, 30) |

| conv2d_11: Conv2D | input: | (?, 128, 128, 30) |
|---|---|---|
| | output: | (?, 128, 128, 42) |

| conv2d_10: Conv2D | input: | (?, 128, 128, 30) |
|---|---|---|
| | output: | (?, 64, 64, 42) |

| batch_normalization_13: BatchNormalization | input: | (?, 128, 128, 42) |
|---|---|---|
| | output: | (?, 128, 128, 42) |

| conv2d_9: Conv2D | input: | (?, 128, 128, 30) |
|---|---|---|
| | output: | (?, 64, 64, 42) |

| activation_13: Activation | input: | (?, 128, 128, 42) |
|---|---|---|
| | output: | (?, 128, 128, 42) |

| batch_normalization_12: BatchNormalization | input: | (?, 64, 64, 42) |
|---|---|---|
| | output: | (?, 64, 64, 42) |

| batch_normalization_11: BatchNormalization | input: | (?, 64, 64, 42) |
|---|---|---|
| | output: | (?, 64, 64, 42) |

| max_pooling2d_2: MaxPooling2D | input: | (?, 128, 128, 42) |
|---|---|---|
| | output: | (?, 64, 64, 42) |

| activation_12: Activation | input: | (?, 64, 64, 42) |
|---|---|---|
| | output: | (?, 64, 64, 42) |

| activation_11: Activation | input: | (?, 64, 64, 42) |
|---|---|---|
| | output: | (?, 64, 64, 42) |

| concatenate_2: Concatenate | input: | [(?, 64, 64, 42), (?, 64, 64, 42)] |
|---|---|---|
| | output: | (?, 64, 64, 84) |

EP 4 016 081 B1

# FIG. 33 (5/5)

| conv2d_12: Conv2D | input: | (?, 64, 64, 84) |
| | output: | (?, 64, 64, 42) |

| batch_normalization_14: BatchNormalization | input: | (?, 64, 64, 42) |
| | output: | (?, 64, 64, 42) |

| activation_14: Activation | input: | (?, 64, 64, 42) |
| | output: | (?, 64, 64, 42) |

| add_2: Add | input: | [(?, 64, 64, 42), (?, 64, 64, 42)] |
| | output: | (?, 64, 64, 42) |

| batch_normalization_15: BatchNormalization | input: | (?, 64, 64, 42) |
| | output: | (?, 64, 64, 42) |

FIG. 34 (1/5)

NN1A2

| activation_28: Activation | input: | (?, 32, 32, 80) |
|---|---|---|
| | output: | (?, 32, 32, 80) |

| conv2d_transpose_3: Conv2DTranspose | input: | (?, 32, 32, 80) |
|---|---|---|
| | output: | (?, 64, 64, 57) |

| conv2d_transpose_2: Conv2DTranspose | input: | (?, 32, 32, 80) |
|---|---|---|
| | output: | (?, 64, 64, 57) |

| batch_normalization_30: BatchNormalization | input: | (?, 64, 64, 57) |
|---|---|---|
| | output: | (?, 64, 64, 57) |

| batch_normalization_29: BatchNormalization | input: | (?, 64, 64, 57) |
|---|---|---|
| | output: | (?, 64, 64, 57) |

| activation_30: Activation | input: | (?, 64, 64, 57) |
|---|---|---|
| | output: | (?, 64, 64, 57) |

| activation_29: Activation | input: | (?, 64, 64, 57) |
|---|---|---|
| | output: | (?, 64, 64, 57) |

| concatenate_6: Concatenate | input: | [(?, 64, 64, 57), (?, 64, 64, 57), (?, 64, 64, 42)] |
|---|---|---|
| | output: | (?, 64, 64, 156) |

| conv2d_22: Conv2D | input: | (?, 64, 64, 156) |
|---|---|---|
| | output: | (?, 64, 64, 57) |

| batch_normalization_31: BatchNormalization | input: | (?, 64, 64, 57) |
|---|---|---|
| | output: | (?, 64, 64, 57) |

| activation_31: Activation | input: | (?, 64, 64, 57) |
|---|---|---|
| | output: | (?, 64, 64, 57) |

EP 4 016 081 B1

FIG. 34 (2/5)

```
┌─────────────────────────────────┐
│ 1 │
│ 2 │
│ 3 │
│ 4 │
│ 5 │
└─────────────────────────────────┘
```

| conv2d_transpose_5: Conv2DTranspose | input: | (?, 64, 64, 57) |
|---|---|---|
| | output: | (?, 128, 128, 40) |

| conv2d_transpose_4: Conv2DTranspose | input: | (?, 64, 64, 57) |
|---|---|---|
| | output: | (?, 128, 128, 40) |

| batch_normalization_33: BatchNormalization | input: | (?, 128, 128, 40) |
|---|---|---|
| | output: | (?, 128, 128, 40) |

| batch_normalization_32: BatchNormalization | input: | (?, 128, 128, 40) |
|---|---|---|
| | output: | (?, 128, 128, 40) |

| activation_33: Activation | input: | (?, 128, 128, 40) |
|---|---|---|
| | output: | (?, 128, 128, 40) |

| activation_32: Activation | input: | (?, 128, 128, 40) |
|---|---|---|
| | output: | (?, 128, 128, 40) |

| concatenate_7: Concatenate | input: | [(?, 128, 128, 40), (?, 128, 128, 40), (?, 128, 128, 30)] |
|---|---|---|
| | output: | (?, 128, 128, 110) |

| conv2d_23: Conv2D | input: | (?, 128, 128, 110) |
|---|---|---|
| | output: | (?, 128, 128, 40) |

| batch_normalization_34: BatchNormalization | input: | (?, 128, 128, 40) |
|---|---|---|
| | output: | (?, 128, 128, 40) |

| activation_34: Activation | input: | (?, 128, 128, 40) |
|---|---|---|
| | output: | (?, 128, 128, 40) |

EP 4 016 081 B1

FIG. 34 (3/5)

| 1 |
|---|
| 2 |
| 3 |
| 4 |
| 5 |

| conv2d_transpose_7 : Conv2DTranspose | input: | (?, 128, 128, 40) |
|---|---|---|
| | output: | (?, 256, 256, 28) |

| conv2d_transpose_6: Conv2DTranspose | input: | (?, 128, 128, 40) |
|---|---|---|
| | output: | (?, 256, 256, 28) |

| batch_normalization_36: BatchNormalization | input: | (?, 256, 256, 28) |
|---|---|---|
| | output: | (?, 256, 256, 28) |

| batch_normalization_35: BatchNormalization | input: | (?, 256, 256, 28) |
|---|---|---|
| | output: | (?, 256, 256, 28) |

| activation_36: Activation | input: | (?, 256, 256, 28) |
|---|---|---|
| | output: | (?, 256, 256, 28) |

| activation_35: Activation | input: | (?, 256, 256, 28) |
|---|---|---|
| | output: | (?, 256, 256, 28) |

| concatenate_8: Concatenate | input: | [(?, 256, 256, 28), (?, 256, 256, 28), (?, 256, 256, 22)] |
|---|---|---|
| | output: | (?, 256, 256, 78) |

| conv2d_24: Conv2D | input: | (?, 256, 256, 78) |
|---|---|---|
| | output: | (?, 256, 256, 28) |

| batch_normalization_37: BatchNormalization | input: | (?, 256, 256, 28) |
|---|---|---|
| | output: | (?, 256, 256, 28) |

| activation_37: Activation | input: | (?, 256, 256, 28) |
|---|---|---|
| | output: | (?, 256, 256, 28) |

EP 4 016 081 B1

79

# FIG. 34 (4/5)

EP 4 016 081 B1

FIG. 34 (5/5)

EP 4 016 081 B1

# FIG. 35 (1/5)

NN1A3

| activation_28: Activation | input: | (?, 32, 32, 80) |
|---|---|---|
| | output: | (?, 32, 32, 80) |

| conv2d_transpose_3: Conv2DTranspose | input: | (?, 32, 32, 80) |
|---|---|---|
| | output: | (?, 64, 64, 57) |

| conv2d_transpose_2: Conv2DTranspose | input: | (?, 32, 32, 80) |
|---|---|---|
| | output: | (?, 64, 64, 57) |

| batch_normalization_30: BatchNormalization | input: | (?, 64, 64, 57) |
|---|---|---|
| | output: | (?, 64, 64, 57) |

| batch_normalization_29: BatchNormalization | input: | (?, 64, 64, 57) |
|---|---|---|
| | output: | (?, 64, 64, 57) |

| activation_30: Activation | input: | (?, 64, 64, 57) |
|---|---|---|
| | output: | (?, 64, 64, 57) |

| activation_29: Activation | input: | (?, 64, 64, 57) |
|---|---|---|
| | output: | (?, 64, 64, 57) |

| concatenate_6: Concatenate | input: | [(?, 64, 64, 57), (?, 64, 64, 57), (?, 64, 64, 42)] |
|---|---|---|
| | output: | (?, 64, 64, 156) |

| conv2d_22: Conv2D | input: | (?, 64, 64, 156) |
|---|---|---|
| | output: | (?, 64, 64, 57) |

| batch_normalization_31: BatchNormalization | input: | (?, 64, 64, 57) |
|---|---|---|
| | output: | (?, 64, 64, 57) |

| activation_31: Activation | input: | (?, 64, 64, 57) |
|---|---|---|
| | output: | (?, 64, 64, 57) |

FIG. 35 (2/5)

```
| 1 |
| 2 |
| 3 |
| 4 |
| 5 |
```

| conv2d_transpose_5: Conv2DTranspose | input: | (?, 64, 64, 57) |
|---|---|---|
| | output: | (?, 128, 128, 40) |

| conv2d_transpose_4: Conv2DTranspose | input: | (?, 64, 64, 57) |
|---|---|---|
| | output: | (?, 128, 128, 40) |

| batch_normalization_33: BatchNormalization | input: | (?, 128, 128, 40) |
|---|---|---|
| | output: | (?, 128, 128, 40) |

| batch_normalization_32: BatchNormalization | input: | (?, 128, 128, 40) |
|---|---|---|
| | output: | (?, 128, 128, 40) |

| activation_33: Activation | input: | (?, 128, 128, 40) |
|---|---|---|
| | output: | (?, 128, 128, 40) |

| activation_32: Activation | input: | (?, 128, 128, 40) |
|---|---|---|
| | output: | (?, 128, 128, 40) |

| concatenate_7: Concatenate | input: | [(?, 128, 128, 40), (?, 128, 128, 40), (?, 128, 128, 30)] |
|---|---|---|
| | output: | (?, 128, 128, 110) |

| conv2d_23: Conv2D | input: | (?, 128, 128, 110) |
|---|---|---|
| | output: | (?, 128, 128, 40) |

| batch_normalization_34: BatchNormalization | input: | (?, 128, 128, 40) |
|---|---|---|
| | output: | (?, 128, 128, 40) |

| activation_34: Activation | input: | (?, 128, 128, 40) |
|---|---|---|
| | output: | (?, 128, 128, 40) |

EP 4 016 081 B1

FIG. 35 (3/5)

```
| 1 |
| 2 |
| 3 |
| 4 |
| 5 |
```

| conv2d_transpose_7 : Conv2DTranspose | input: | (?, 128, 128, 40) |
| | output: | (?, 256, 256, 28) |

| conv2d_transpose_6: Conv2DTranspose | input: | (?, 128, 128, 40) |
| | output: | (?, 256, 256, 28) |

| batch_normalization_36: BatchNormalization | input: | (?, 256, 256, 28) |
| | output: | (?, 256, 256, 28) |

| batch_normalization_35: BatchNormalization | input: | (?, 256, 256, 28) |
| | output: | (?, 256, 256, 28) |

| activation_36: Activation | input: | (?, 256, 256, 28) |
| | output: | (?, 256, 256, 28) |

| activation_35: Activation | input: | (?, 256, 256, 28) |
| | output: | (?, 256, 256, 28) |

| concatenate_8: Concatenate | input: | [(?, 256, 256, 28), (?, 256, 256, 28), (?, 256, 256, 22)] |
| | output: | (?, 256, 256, 78) |

| conv2d_24: Conv2D | input: | (?, 256, 256, 78) |
| | output: | (?, 256, 256, 28) |

| batch_normalization_37: BatchNormalization | input: | (?, 256, 256, 28) |
| | output: | (?, 256, 256, 28) |

| activation_37: Activation | input: | (?, 256, 256, 28) |
| | output: | (?, 256, 256, 28) |

EP 4 016 081 B1

FIG. 35 (4/5)

| 1 |
| 2 |
| 3 |
| 4 |
| 5 |

| conv2d_transpose_9 : Conv2DTranspose | input: | (?, 256, 256, 28) |
| | output: | (?, 512, 512, 20) |

| conv2d_transpose_8: Conv2DTranspose | input: | (?, 256, 256, 28) |
| | output: | (?, 512, 512, 20) |

| batch_normalization_39: BatchNormalization | input: | (?, 512, 512, 20) |
| | output: | (?, 512, 512, 20) |

| batch_normalization_38: BatchNormalization | input: | (?, 512, 512, 20) |
| | output: | (?, 512, 512, 20) |

| activation_39: Activation | input: | (?, 512, 512, 20) |
| | output: | (?, 512, 512, 20) |

| activation_38: Activation | input: | (?, 512, 512, 20) |
| | output: | (?, 512, 512, 20) |

| concatenate_9: Concatenate | input: | [(?, 512, 512, 20), (?, 512, 512, 20), (?, 512, 512, 16)] |
| | output: | (?, 512, 512, 56) |

| conv2d_25: Conv2D | input: | (?, 512, 512, 56) |
| | output: | (?, 512, 512, 20) |

| batch_normalization_40: BatchNormalization | input: | (?, 512, 512, 20) |
| | output: | (?, 512, 512, 20) |

| activation_40: Activation | input: | (?, 512, 512, 20) |
| | output: | (?, 512, 512, 20) |

EP 4 016 081 B1

FIG. 35 (5/5)

```
┌─ ─┐
┆ 1 ┆
├─ ─┤
┆ 2 ┆
├─ ─┤
┆ 3 ┆
├─ ─┤
┆ 4 ┆
├───┤
│ 5 │
└───┘
```

SEGA

BES

SEG3

MSA

SEG3'

| conv2d_26: Conv2D | input: | (?, 512, 512, 20) |
| | output: | (?, 512, 512, 14) |

| batch_normalization_41: BatchNormalization | input: | (?, 512, 512, 14) |
| | output: | (?, 512, 512, 14) |

| activation_41: Activation | input: | (?, 512, 512, 14) |
| | output: | (?, 512, 512, 14) |

| conv2d_27: Conv2D | input: | (?, 512, 512, 14) |
| | output: | (?, 512, 512, 6) |

SC3

EP 4 016 081 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3258247 A1
- EP 3712618 A1

- EP 3767587 A1